# EUROPEAN PATENT APPLICATION

(11) **EP 4 506 366 A1**
(43) Date of publication of application: **12.02.2025**
(21) Application number: 23778477.2
(22) Date of filing: 31.03.2023
(51) Int. Cl.: C07K 16/46, C07K 16/28, C07K 19/00, C12N 15/13, C12N 15/867, C12N 5/10, A61P 35/00, A61K 39/395

(54) **ANTIGEN-BINDING PROTEIN TARGETING PD-L1 AND CD40, PREPARATION THEREFOR, AND USE THEREOF**

(30) Priority: 02.04.2022 CN 202210351186
(71) Applicant: Harbour Biomed (Shanghai) Co., Ltd, Shanghai 201203 (CN)
(72) Inventor: LUO, Haishan, Shanghai 201203 (CN); MENG, Zihong, Shanghai 201203 (CN); RONG, Jun, Shanghai 201203 (CN); CHEN, Hongwei, Shanghai 201203 (CN); WANG, Yuandong, Shanghai 201203 (CN); YANG, Yunxing, Shanghai 201203 (CN); CHEN, Fei, Shanghai 201203 (CN); WANG, Youhong, Shanghai 201203 (CN); LU, Jia, Shanghai 201203 (CN); LI, Shutao, Shanghai 201203 (CN); DENG, Gang, Shanghai 201203 (CN); GAN, Xin, Shanghai 201203 (CN); RONG, Yiping, Shanghai 201203 (CN)
(74) Representative: Murgitroyd & Company
(86) International application number: PCT/CN2023/085540
(87) International publication number: WO 2023/186113

(57) **Abstract**

Disclosed are an antigen-binding protein targeting PD-L1 and CD40, preparation therefor, and use thereof. The antigen-binding protein targeting PD-L1 and CD40, by means of blocking a PD-1/PD-L1 inhibitory signaling pathway, acting on an activated CD40 receptor, and simultaneously activating an antigen-presenting cell and a lymphocyte, achieves a significant synergistic anti-tumor effect; meanwhile, the activation of the antigen-binding protein targeting PD-L1 and CD40 on an immune cell only occurs at a tumor microenvironment site, so that problems of systemic drug toxicity and side effects are significantly solved, which enables the antigen-binding protein targeting PD-L1 and CD40 to exert an excellent anti-tumor efficacy under very safe conditions.

## Description

The present application claims the right of priority for the Chinese patent application 202210351186X with the filing date of 2 April 2022. This Chinese patent application is incorporated herein by reference in its entirety.

### Technical Field

The present invention relates to the field of biopharmaceuticals, and in particular to a bispecific antigen-binding protein targeting PD-L1 and CD40, preparation therefor, and use thereof.

### Background

CD40 is a glycosylated type I transmembrane protein and a member of the tumor necrosis factor receptor superfamily (TNFRSF), and also known as tumor necrosis factor receptor superfamily member 5 (TNFRSF5). CD40 is expressed on the surface of a series of antigen-presenting cells (APCs), including a monocyte, a dendritic cell (DC), a B cell, and a macrophage. CD40L, a ligand of CD40, is mainly expressed on the surface of lymphocytes including a T cell, a B cell, and an NK cell, and usually exists in the form of a trimer and a polymer. CD40 and CD40L are a pair of co-stimulatory molecules. Activation of CD40 downstream signaling pathway requires the crosslinking of CD40 to CD40L in the trimer and polymer form. CD40 and CD40L interact on the cell surface, causing CD40 to redistribute to membrane lipid rafts and undergo conformational changes. CD40 recruits TNFR-associated factor (TRAF) in the cytoplasm by the intracellular terminal domain to promote intracellular signal transduction, thereby activating different signaling pathways, such as classical and non-classical nuclear factor κB pathways, p38 mitogen-activated protein kinase, phosphatidylinositol-3 kinase (PI3K) and phospholipase Cγ pathways, and further regulating apoptosis, cell cycle progression, cytokine production and the expression of cell surface immune regulator via the genes targeted by these signaling pathways. Therefore, activation of CD40 can increase antigen presentation, promote cytokine secretion, activate lymphocyte, and simultaneously stimulate and activate the human innate immune system and acquired immune system, thereby producing a synergistic effect to resist the occurrence and development of cancers.

CD40 is also widely expressed in tumor cells and is expressed in almost all B-cell malignancies and a wide range of solid tumors, including melanoma, lung cancer, breast cancer, colon cancer, prostate cancer, pancreatic cancer, kidney cancer, ovarian cancer, and head and neck cancer. CD40 expressed on the surface of tumor cells can mediate tumor cell death. In the absence of immune helper cells, CD40 expressed on the surface of a variety of tumor cells will mediate direct cytotoxic effects by crosslinking to CD40L. In vitro, crosslinking of CD40 to CD40L has been shown to induce tumor cell apoptosis and inhibit the growth of solid tumor cells and B malignant tumor cells. In vivo, activation of CD40 also mediates inhibitory effects on tumors. Evidence indicated that in immunodeficient mice, intervention with CD40 on the surface of tumor cells by CD40L could inhibit the growth of breast cancer cell transplant tumor or B lymphocyte transplant tumor without lymphocyte activation.

Therefore, the mechanism of CD40-mediated tumor cell death can be dual, namely, stimulation of the immune system to kill tumor cells and direct tumor cytotoxicity, which can be synergistic in anti-tumor effects. An agonistic anti-CD40 antibody, similar to CD40L, can crosslink and activate CD40 on the surface of immune cells and tumor cells, exerting a significant anti-tumor effect. This anti-tumor effect has been demonstrated in trials in preclinical animal models and clinical tumor patients. The anti-CD40 antibody can be combined with chemotherapeutic drugs, such as gemcitabine and paclitaxel, or with immunomodulatory drugs, such as a PD-1 antibody and a CTLA-4 antibody, to produce a synergistic anti-tumor effect.

At present, in the field of anti-tumor, there are nearly 20 CD40 antibody drugs in the clinical trial stage, but the earliest product is only in Phase II clinical trials, and there is no commercially available product yet. The main problems of the CD40 antibody in clinical practice include low objective response rate, significant toxic side effects and low tolerated dose. These problems may be due to the weak activity of agonists, such as Celldex product CDX-1140. In Phase I clinical trial of CDX-1140, no complete or partial responses were observed in 42 patients treated with monotherapy. In the combined treatment with rhFLT3L, only 1 of the 20 patients experienced partial response. This product has a weak activation effect on DC cells in vitro, and the agonist activity cannot be enhanced in the presence of crosslinking. Another reason is that CD40 antibody does not have selective activation property, resulting in systemic toxic side effects. For example, in clinical trials, Roche CD40 antibody, Selicrelumab, caused significant toxic side effects such as cytokine release syndrome and liver toxicity in some patients at a dosage exceeded 0.2 mg/kg. In clinical trials, Apexigen CD40 antibody, APX005M, caused significant neutropenia, as well as further sepsis and septic shock in some patients at a dosage exceeded 0.3 mg/kg. In summary, existing clinical CD40 antibodies face the problem and challenge of a too-small therapeutic window between effectiveness and safety. Therefore, the development of other CD40 antibodies will provide the possibility for the treatment of various tumors and has high scientific and market value.

PD-L1, programmed death ligand 1, also known as CD274 and B7H1, is a glycosylated type I transmembrane protein and one of the ligands of PD-1, programmed death protein 1. Under normal circumstances, PD-L1 is widely expressed on antigen-presenting cells, such as a dendritic cell, a macrophage and a B cell, as well as other immune cells. When PD-L1 binds to the PD-1 receptor expressed on the surface of T cells, it inhibits the activation of T cells and the secretion of cytokines, and plays a key role in inducing the body's immune contraction and maintaining immune tolerance. On the other hand, PD-L1 is abundantly expressed on the surface of many cancer cells, including renal cell carcinoma, breast cancer, colorectal cancer, gastric cancer, non-small cell lung cancer, papillary thyroid carcinoma and testicular cancer. Binding of PD-L1 on tumor cells to PD-1 on tumor-infiltrating T cells (TILs) activates Src homology region 2 domain-containing phosphatases (SHPs), leading to inhibition of the T cell receptor (TCR) pathway and T cell activity. In addition, tumor cells interrupt immune surveillance and promote cancer cell survival by using the PD-L1/PD-1 signaling pathway.

Therefore, blocking the PD-L1/PD-1 signaling axis by an antibody can reactivate suppressed and exhausted immune cells such as T cells in the tumor microenvironment, thereby eliminating cancer cells by the immune cells and reestablishing immune balance. Based on the above-mentioned findings, therapeutic PD-L1 antibodies (e.g., atezolizumab, avelumab and durvalumab) and PD-1 antibodies (e.g., nivolumab, pembrolizumab and cemiplimab) have been developed and marketed, and have achieved clinical response of 10%-40% in clinical practice for various cancers, including melanoma, small cell lung cancer, non-small cell lung cancer, renal cell carcinoma, head and neck squamous cell carcinoma, classical Hodgkin's lymphoma, and Merkel cell carcinoma.

However, although anti-PD-L1/PD-1 therapy has shown impressive effects in tumor treatment, especially in solid tumor treatment, durable response occurs only in a small number of patients, and some patients who initially respond to treatment eventually develop acquired resistance. At the same time, there are still a large number of patients who do not respond to the anti-PD-L1/PD-1 therapy, that is, primary resistance. Therefore, it is urgent to develop more effective immunotherapies that can produce a synergistic anti-tumor effect with the anti-PD-L1/PD-1 therapy to overcome primary and acquired resistance. This direction has become the focus of tumor immunotherapy at the present stage.

Currently, there are no antigen-binding protein targeting PD-L1 and CD40 on the market, and there is a great demand for the antigen-binding protein targeting both PD-L1 and CD40 in the market.

### Summary

In view of the technical defects of an antigen-binding protein targeting PD-L1 and CD40 in the prior art, such as limited efficacy and poor safety, the present invention provides an antigen-binding protein targeting PD-L1 and CD40, preparation therefor, and use thereof. The antigen-binding protein targeting PD-L1 and CD40, by means of blocking a PD-1/PD-L1 inhibitory signaling pathway, acting on an activated CD40 receptor, and simultaneously activating an antigen-presenting cell and a lymphocyte, achieves a significant synergistic anti-tumor effect; meanwhile, the activation of the antigen-binding protein targeting PD-L1 and CD40 on an immune cell only occurs at a tumor microenvironment site, so that problems of systemic drug toxicity and side effects are significantly solved, which enables the antigen-binding protein targeting PD-L1 and CD40 to exert an excellent anti-tumor efficacy under very safe conditions.

In order to solve the above-mentioned technical problems, a first aspect of the present invention provides an antigen-binding protein targeting PD-L1 and CD40, which comprises a first protein functional region and a second protein functional region, wherein the first protein functional region comprises an antigen-binding protein targeting CD40, and the second protein functional region comprises an antigen-binding protein targeting PD-L1, wherein
the antigen-binding protein targeting CD40 comprises a light chain variable region (VL) comprising LCDR1, LCDR2, and LCDR3 and a heavy chain variable region (VH) comprising HCDR1, HCDR2, and HCDR3, the LCDR1 comprises the amino acid sequence set forth in SEQ ID NO: 38 or a variant 1 with 3, 2 or 1 amino acid mutation in SEQ ID NO: 38, the LCDR2 comprises the amino acid sequence set forth in SEQ ID NO: 43 or a variant 2 with 3, 2 or 1 amino acid mutation in SEQ ID NO: 43, the LCDR3 comprises the amino acid sequence set forth in SEQ ID NO: 48 or a variant 3 with 3, 2 or 1 amino acid mutation in SEQ ID NO: 48, the HCDR1 comprises the amino acid sequence set forth in SEQ ID NO: 8 or a variant 4 with 3, 2 or 1 amino acid mutation in SEQ ID NO: 8, the HCDR2 comprises the amino acid sequence set forth in SEQ ID NO: 18 or a variant 5 with 3, 2 or 1 amino acid mutation in SEQ ID NO: 18, and the HCDR3 comprises the amino acid sequence set forth in SEQ ID NO: 27 or a variant 6 with 3, 2 or 1 amino acid mutation in SEQ ID NO: 27.

In some technical solutions of the present invention, the antigen-binding protein targeting CD40 comprises a light chain variable region (VL) comprising LCDR1, LCDR2, and LCDR3 and a heavy chain variable region (VH) comprising HCDR1, HCDR2, and HCDR3, the LCDR1 comprises the amino acid sequence set forth in SEQ ID NO: 38, the LCDR2 comprises the amino acid sequence set forth in SEQ ID NO: 43, the LCDR3 comprises the amino acid sequence set forth in SEQ ID NO: 48 or a variant 3 with 3, 2 or 1 amino acid mutation in SEQ ID NO: 48, the HCDR1 comprises the amino acid sequence set forth in SEQ ID NO: 8, the HCDR2 comprises the amino acid sequence set forth in SEQ ID NO: 18, and the HCDR3 comprises the amino acid sequence set forth in SEQ ID NO: 27.

In some technical solutions of the present invention, the variant 3 comprises an amino acid sequence having a PTM site mutation in the amino acid sequence set forth in SEQ ID NO: 48, preferably comprises an amino acid sequence having an amino acid mutation at position 4 and/or position 5 of the amino acid sequence set forth in SEQ ID NO: 48, preferably the amino acid mutation is an amino acid substitution, more preferably a conservative amino acid substitution. Preferably, the variant 3 is an amino acid sequence having an N4A/F/Y/V/N and/or S5N mutation in the amino acid sequence set forth in SEQ ID NO: 48. More preferably, the variant 3 is an amino acid sequence set forth in any one of SEQ ID NOs: 49-53.

In some technical solutions of the present invention, in the antigen-binding protein targeting CD40, the LCDR1 comprises the amino acid sequence set forth in SEQ ID NO: 38, the LCDR2 comprises the amino acid sequence set forth in SEQ ID NO: 43, and the LCDR3 comprises amino acid sequence 3 set forth in any one of SEQ ID NOs: 48-53, the HCDR1 comprises the amino acid sequence set forth in SEQ ID NO: 8, the HCDR2 comprises the amino acid sequence set forth in SEQ ID NO: 18, and the HCDR3 comprises the amino acid sequence set forth in SEQ ID NO: 27. See Table 1-1 for specific CDR combinations.

**Table 1-1. Specific CDR combinations of the antigen-binding protein targeting CD40**

| | **SEQ ID NO:** | | | | | |
|---|---|---|---|---|---|---|
| Molecular number | LCDR1 | LCDR2 | LCDR3 | HCDR1 | HCDR2 | HCDR3 |
| PR003379 | 38 | 43 | 48 | 8 | 18 | 27 |
| PR006495 | 38 | 43 | 49 | 8 | 18 | 27 |
| PR006496 | 38 | 43 | 50 | 8 | 18 | 27 |
| PR006497 | 38 | 43 | 51 | 8 | 18 | 27 |
| PR006504 | 38 | 43 | 52 | 8 | 18 | 27 |
| PR006511 | 38 | 43 | 53 | 8 | 18 | 27 |

In some technical solutions of the present invention, in the antigen-binding protein targeting CD40, the VL comprises the amino acid sequence set forth in SEQ ID NO: 68 or an amino acid sequence having at least 80%, 85%, 90%, 92%, 94%, 95%, 96%, 97%, 98% or 99% identity to SEQ ID NO: 68, and the VH comprises the amino acid sequence set forth in SEQ ID NO: 60 or an amino acid sequence having at least 80%, 85%, 90%, 92%, 94%, 95%, 96%, 97%, 98% or 99% identity to SEQ ID NO: 60. More preferably, the VL comprises the amino acid sequence set forth in any one of SEQ ID NOs: 68-73, and the VH comprises the amino acid sequence set forth in SEQ ID NO: 60. See Table 1-2 for specific VL and VH combinations.

**Table 1-2. Specific VL and VH combinations for the antigen-binding protein targeting CD40**

| | **SEQ ID NO:** | |
|---|---|---|
| Molecular number | VL | VH |
| PR003379 | 68 | 60 |
| PR006495 | 69 | 60 |
| PR006496 | 70 | 60 |
| PR006497 | 71 | 60 |
| PR006504 | 72 | 60 |
| PR006511 | 73 | 60 |

In some technical solutions of the present invention, the antigen-binding protein targeting CD40 is a full-length antibody comprising a light chain and a heavy chain, the light chain comprises a light chain constant region (CL), preferably the light chain constant region is a light chain constant region of human antibody, more preferably the light chain constant region of the human antibody is a light chain constant region of κ subtype, the heavy chain comprises a heavy chain constant region (CH), and the heavy chain constant region is preferably a heavy chain constant region of human antibody, more preferably a heavy chain constant region of hIgG1, hIgG2, hIgG3 or hIgG4 subtype, and further preferably a heavy chain constant region of hIgG1 subtype.

In some technical solutions of the present invention, the antigen-binding protein targeting CD40 is a full-length antibody comprising a light chain and a heavy chain, the light chain comprises the amino acid sequence set forth in any one of SEQ ID NOs: 87-92, and the heavy chain comprises the amino acid sequence set forth in SEQ ID NO: 78 or 84. Preferably, the light chain comprises the amino acid sequence set forth in SEQ ID NO: 87, and the heavy chain comprises the amino acid sequence set forth in SEQ ID NO: 78, or, the light chain comprises the amino acid sequence set forth in any one of SEQ ID NOs: 88-92, and the heavy chain comprises the amino acid sequence set forth in SEQ ID NO: 84. See Table 1-3 for specific combinations of light chain and heavy chain.

**Table 1-3. Specific combinations of light chain and heavy chain of the antigen-binding protein targeting CD40**

| | **SEQ ID NO:** | |
|---|---|---|
| Molecular number | Light chain | Heavy chain |
| PR003379 | 87 | 78 |
| PR006495 | 88 | 84 |
| PR006496 | 89 | 84 |
| PR006497 | 90 | 84 |
| PR006504 | 91 | 84 |
| PR006511 | 92 | 84 |

In some technical solutions of the present invention, the antigen-binding protein targeting PD-L1 comprises a heavy chain variable region (VH) comprising HCDR1, HCDR2, and HCDR3, the HCDR1 comprises the amino acid sequence set forth in SEQ ID NO: 5 or a variant 7 with 3, 2 or 1 amino acid mutation in SEQ ID NO: 5, the HCDR2 comprises the amino acid sequence set forth in SEQ ID NO: 16 or a variant 8 with 3, 2 or 1 amino acid mutation in SEQ ID NO: 16, and the HCDR3 comprises the amino acid sequence set forth in SEQ ID NO: 25 or a variant 9 with 3, 2 or 1 amino acid mutation in SEQ ID NO: 25.

In some technical solutions of the present invention, the variant 7 is an amino acid sequence having an amino acid mutation at position 3 and/or 6 of SEQ ID NO: 5, the variant 8 is an amino acid sequence having an amino acid mutation at position 1, 3 and/or 6 of SEQ ID NO: 16, and the variant 9 is an amino acid sequence having an amino acid mutation at position 4, 8 and/or 11 of SEQ ID NO: 25, the amino acid mutation is preferably an amino acid substitution, more preferably a conservative amino acid substitution. More preferably, the variant 7 is an amino acid sequence having N3T/D and/or N5S mutation in SEQ ID NO: 5, the variant 8 is an amino acid sequence having W1R, D3T and/or K5E mutation in SEQ ID NO: 16, and the variant 9 is an amino acid sequence having I4L, V8I and/or A11D mutation in SEQ ID NO: 25. Preferably, the variant 7 is an amino acid sequence set forth in SEQ ID NO: 7 or 9, the variant 8 is an amino acid sequence set forth in SEQ ID NO: 19, and the variant 9 is an amino acid sequence set forth in SEQ ID NO: 28, 29 or 30.

In some technical solutions of the present invention, in the antigen-binding protein targeting PD-L1, the HCDR1 comprises the amino acid sequence set forth in SEQ ID NO: 5, 7 or 9, the HCDR2 comprises the amino acid sequence set forth in SEQ ID NO: 16 or 19, and the HCDR3 comprises the amino acid sequence set forth in SEQ ID NO: 25, 28, 29 or 30.

In some technical solutions of the present invention, in the antigen-binding protein targeting PD-L1, the HCDR1 comprises the amino acid sequence set forth in SEQ ID NO: 5, the HCDR2 comprises the amino acid sequence set forth in SEQ ID NO: 16, and the HCDR3 comprises the amino acid sequence set forth in SEQ ID NO: 25; or, the HCDR1 comprises the amino acid sequence set forth in SEQ ID NO: 7, the HCDR2 comprises the amino acid sequence set forth in SEQ ID NO: 16, and the HCDR3 comprises the amino acid sequence set forth in SEQ ID NO: 25; or, the HCDR1 comprises the amino acid sequence set forth in SEQ ID NO: 7, the HCDR2 comprises the amino acid sequence set forth in SEQ ID NO: 19, and the HCDR3 comprises the amino acid sequence set forth in SEQ ID NO: 28; or, the HCDR1 comprises the amino acid sequence set forth in SEQ ID NO: 9, the HCDR2 comprises the amino acid sequence set forth in SEQ ID NO: 19, and the HCDR3 comprises the amino acid sequence set forth in SEQ ID NO: 25; or, the HCDR1 comprises the amino acid sequence set forth in SEQ ID NO: 9, the HCDR2 comprises the amino acid sequence set forth in SEQ ID NO: 19, and the HCDR3 comprises the amino acid sequence set forth in SEQ ID NO: 29; or, the HCDR1 comprises the amino acid sequence set forth in SEQ ID NO: 9, the HCDR2 comprises the amino acid sequence set forth in SEQ ID NO: 19, and the HCDR3 comprises the amino acid sequence set forth in SEQ ID NO: 30; or, the HCDR1 comprises the amino acid sequence set forth in SEQ ID NO: 9, the HCDR2 comprises the amino acid sequence set forth in SEQ ID NO: 19, and the HCDR3 comprises the amino acid sequence set forth in SEQ ID NO: 28.

In some technical solutions of the present invention, in the antigen-binding protein targeting PD-L1, the VH comprises the amino acid sequence set forth in SEQ ID NO: 57 or an amino acid sequence having at least 85%, 90%, 92%, 94%, 95%, 96%, 97%, 98% or 99% identity to SEQ ID NO: 57. More preferably, the VH comprises the amino acid sequence set forth in any one of SEQ ID NOs: 57-65.

In some technical solutions of the present invention, the first protein functional region is an immunoglobulin comprising the antigen-binding protein targeting CD40, and the second protein functional region comprises one, two or more VHs of the antigen-binding protein targeting PD-L1.

In some technical solutions of the present invention, the VHs of the antigen-binding protein targeting PD-L1 are directly linked to the immunoglobulin; or, the VHs are linked to the immunoglobulin via a linker; or, when the number of the VHs is greater than 1, each of the VHs can be linked to the immunoglobulin directly or via a linker, respectively.

In some technical solutions of the present invention, when the VHs are linked to the immunoglobulin via a linker, the linker is selected from any one of the amino acid sequences set forth in GS, GGS and SEQ ID NOs: 100-106.

In some technical solutions of the present invention, the second protein functional region comprises two VHs of the antigen-binding protein targeting PD-L1. Preferably, the two VHs comprised in the second protein functional region are identical; and/or, the C-termini of the two VHs are linked to the N-termini of the two heavy chains in the immunoglobulin via a linker set forth in GGS or SEQ ID NO: 5, respectively.

In some technical solutions of the present invention, the first protein functional region comprises two light chains comprising an amino acid sequence set forth in any one of SEQ ID NOs: 88-92, and two heavy chains comprising an amino acid sequence set forth in SEQ ID NO: 84, the second protein functional region comprises two VHs comprising an amino acid sequence set forth in any one of SEQ ID NOs: 79-83, and the C-termini of the two VHs are linked to the N-termini of the two heavy chains in the first protein functional region via a linker set forth in GGS or SEQ ID NO: 5, respectively.

Preferably, the antigen-binding protein targeting PD-L1 and CD40 comprises a first polypeptide chain and a second polypeptide chain as shown below:
a second polypeptide chain consisting of the amino acid sequence set forth in SEQ ID NO: 89, and a first polypeptide chain consisting of the amino acid sequence set forth in SEQ ID NO: 93; or, a second polypeptide chain consisting of the amino acid sequence set forth in SEQ ID NO: 88, and a first polypeptide chain consisting of the amino acid sequence set forth in SEQ ID NO: 94; or, a second polypeptide chain consisting of the amino acid sequence set forth in SEQ ID NO: 91, and a first polypeptide chain consisting of the amino acid sequence set forth in SEQ ID NO: 94; or, a second polypeptide chain consisting of the amino acid sequence set forth in SEQ ID NO: 90, and a first polypeptide chain consisting of the amino acid sequence set forth in SEQ ID NO: 93; or, a second polypeptide chain consisting of the amino acid sequence set forth in SEQ ID NO: 92, and a first polypeptide chain consisting of the amino acid sequence set forth in SEQ ID NO: 93; or, a second polypeptide chain consisting of the amino acid sequence set forth in SEQ ID NO: 88, and a first polypeptide chain consisting of the amino acid sequence set forth in SEQ ID NO: 95; or, a second polypeptide chain consisting of the amino acid sequence set forth in SEQ ID NO: 88, and a first polypeptide chain consisting of the amino acid sequence set forth in SEQ ID NO: 96; or, a second polypeptide chain consisting of the amino acid sequence set forth in SEQ ID NO: 88, and a first polypeptide chain consisting of the amino acid sequence set forth in SEQ ID NO: 97; or, a second polypeptide chain consisting of the amino acid sequence set forth in SEQ ID NO: 91, and a first polypeptide chain consisting of the amino acid sequence set forth in SEQ ID NO: 95; or, a second polypeptide chain consisting of the amino acid sequence set forth in SEQ ID NO: 91, and a first polypeptide chain consisting of the amino acid sequence set forth in SEQ ID NO: 93; or, a second polypeptide chain consisting of the amino acid sequence set forth in SEQ ID NO: 91, and a first polypeptide chain consisting of the amino acid sequence set forth in SEQ ID NO: 96; or, a second polypeptide chain consisting of the amino acid sequence set forth in SEQ ID NO: 89, and a first polypeptide chain consisting of the amino acid sequence set forth in SEQ ID NO: 97; or, a second polypeptide chain consisting of the amino acid sequence set forth in SEQ ID NO: 90, and a first polypeptide chain consisting of the amino acid sequence set forth in SEQ ID NO: 97; or, a second polypeptide chain consisting of the amino acid sequence set forth in SEQ ID NO: 88, and a first polypeptide chain consisting of the amino acid sequence set forth in SEQ ID NO: 98; or, a second polypeptide chain consisting of the amino acid sequence set forth in SEQ ID NO: 88, and a first polypeptide chain consisting of the amino acid sequence set forth in SEQ ID NO: 99; or, a second polypeptide chain consisting of the amino acid sequence set forth in SEQ ID NO: 91, and a first polypeptide chain consisting of the amino acid sequence set forth in SEQ ID NO: 98; or, a second polypeptide chain consisting of the amino acid sequence set forth in SEQ ID NO: 91, and a first polypeptide chain consisting of the amino acid sequence set forth in SEQ ID NO: 99.

In order to solve the above-mentioned technical problems, a second aspect of the present invention provides an antigen-binding protein targeting PD-L1 and CD40, which comprises a first protein functional region and a second protein functional region, wherein the first protein functional region comprises an antigen-binding protein targeting CD40, and the second protein functional region comprises an antigen-binding protein targeting PD-L1, wherein
the antigen-binding protein targeting PD-L1 comprises a heavy chain variable region (VH) comprising HCDR1, HCDR2, and HCDR3, the HCDR1 comprises the amino acid sequence set forth in SEQ ID NO: 5 or a variant 7 with 3, 2 or 1 amino acid mutation in SEQ ID NO: 5, the HCDR2 comprises the amino acid sequence set forth in SEQ ID NO: 16 or a variant 8 with 3, 2 or 1 amino acid mutation in SEQ ID NO: 16, and the HCDR3 comprises the amino acid sequence set forth in SEQ ID NO: 25 or a variant 9 with 3, 2 or 1 amino acid mutation in SEQ ID NO: 25.

In some technical solutions of the present invention, the variant 7 is an amino acid sequence having an amino acid mutation at position 3 and/or 6 of SEQ ID NO: 5, the variant 8 is an amino acid sequence having an amino acid mutation at position 1, 3 and/or 6 of SEQ ID NO: 16, and the variant 9 is an amino acid sequence having an amino acid mutation at position 4, 8 and/or 11 of SEQ ID NO: 25, the amino acid mutation is preferably an amino acid substitution, more preferably a conservative amino acid substitution. More preferably, the variant 7 is an amino acid sequence having N3T/D and/or N5S mutation in SEQ ID NO: 5, the variant 8 is an amino acid sequence having W1R, D3T and/or K5E mutation in SEQ ID NO: 16, and the variant 9 is an amino acid sequence having I4L, V8I and/or A11D mutation in SEQ ID NO: 25. Preferably, the variant 7 is an amino acid sequence set forth in SEQ ID NO: 7 or 9, the variant 8 is an amino acid sequence set forth in SEQ ID NO: 19, and the variant 9 is an amino acid sequence set forth in SEQ ID NO: 28, 29 or 30.

In some technical solutions of the present invention, in the antigen-binding protein targeting PD-L1, the HCDR1 comprises the amino acid sequence set forth in SEQ ID NO: 5, 7 or 9, the HCDR2 comprises the amino acid sequence set forth in SEQ ID NO: 16 or 19, and the HCDR3 comprises the amino acid sequence set forth in SEQ ID NO: 25, 28, 29 or 30.

In some technical solutions of the present invention, in the antigen-binding protein targeting PD-L1, the HCDR1 comprises the amino acid sequence set forth in SEQ ID NO: 5, the HCDR2 comprises the amino acid sequence set forth in SEQ ID NO: 16, and the HCDR3 comprises the amino acid sequence set forth in SEQ ID NO: 25; or, the HCDR1 comprises the amino acid sequence set forth in SEQ ID NO: 7, the HCDR2 comprises the amino acid sequence set forth in SEQ ID NO: 16, and the HCDR3 comprises the amino acid sequence set forth in SEQ ID NO: 25; or, the HCDR1 comprises the amino acid sequence set forth in SEQ ID NO: 7, the HCDR2 comprises the amino acid sequence set forth in SEQ ID NO: 19, and the HCDR3 comprises the amino acid sequence set forth in SEQ ID NO: 28; or, the HCDR1 comprises the amino acid sequence set forth in SEQ ID NO: 9, the HCDR2 comprises the amino acid sequence set forth in SEQ ID NO: 19, and the HCDR3 comprises the amino acid sequence set forth in SEQ ID NO: 25; or, the HCDR1 comprises the amino acid sequence set forth in SEQ ID NO: 9, the HCDR2 comprises the amino acid sequence set forth in SEQ ID NO: 19, and the HCDR3 comprises the amino acid sequence set forth in SEQ ID NO: 29; or, the HCDR1 comprises the amino acid sequence set forth in SEQ ID NO: 9, the HCDR2 comprises the amino acid sequence set forth in SEQ ID NO: 19, and the HCDR3 comprises the amino acid sequence set forth in SEQ ID NO: 30; or, the HCDR1 comprises the amino acid sequence set forth in SEQ ID NO: 9, the HCDR2 comprises the amino acid sequence set forth in SEQ ID NO: 19, and the HCDR3 comprises the amino acid sequence set forth in SEQ ID NO: 28.

In some technical solutions of the present invention, in the antigen-binding protein targeting PD-L1, the VH comprises the amino acid sequence set forth in SEQ ID NO: 57 or an amino acid sequence having at least 85%, 90%, 92%, 94%, 95%, 96%, 97%, 98% or 99% identity to SEQ ID NO: 57. More preferably, the VH comprises the amino acid sequence set forth in any one of SEQ ID NOs: 57-65.

In some technical solutions of the present invention, the antigen-binding protein targeting CD40 comprises a light chain variable region (VL) comprising LCDR1, LCDR2, and LCDR3 and a heavy chain variable region (VH) comprising HCDR1, HCDR2, and HCDR3, the LCDR1 comprises the amino acid sequence set forth in SEQ ID NO: 38 or a variant 1 with 3, 2 or 1 amino acid mutation in SEQ ID NO: 38, the LCDR2 comprises the amino acid sequence set forth in SEQ ID NO: 43 or a variant 2 with 3, 2 or 1 amino acid mutation in SEQ ID NO: 43, the LCDR3 comprises the amino acid sequence set forth in SEQ ID NO: 48 or a variant 3 with 3, 2 or 1 amino acid mutation in SEQ ID NO: 48; the HCDR1 comprises the amino acid sequence set forth in SEQ ID NO: 8 or a variant 4 with 3, 2 or 1 amino acid mutation in SEQ ID NO: 8, the HCDR2 comprises the amino acid sequence set forth in SEQ ID NO: 18 or a variant 5 with 3, 2 or 1 amino acid mutation in SEQ ID NO: 18, and the HCDR3 comprises the amino acid sequence set forth in SEQ ID NO: 27 or a variant 6 with 3, 2 or 1 amino acid mutation in SEQ ID NO: 27.

In some technical solutions of the present invention, the antigen-binding protein targeting CD40 comprises a light chain variable region (VL) comprising LCDR1, LCDR2, and LCDR3 and a heavy chain variable region (VH) comprising HCDR1, HCDR2, and HCDR3, the LCDR1 comprises the amino acid sequence set forth in SEQ ID NO: 38, the LCDR2 comprises the amino acid sequence set forth in SEQ ID NO: 43, the LCDR3 comprises the amino acid sequence set forth in SEQ ID NO: 48 or a variant 3 with 3, 2 or 1 amino acid mutation in SEQ ID NO: 48; the HCDR1 comprises the amino acid sequence set forth in SEQ ID NO: 8, the HCDR2 comprises the amino acid sequence set forth in SEQ ID NO: 18, and the HCDR3 comprises the amino acid sequence set forth in SEQ ID NO: 27.

In some technical solutions of the present invention, the variant 3 comprises an amino acid sequence having a PTM site mutation in the amino acid sequence set forth in SEQ ID NO: 48, preferably comprises an amino acid sequence having an amino acid mutation at position 4 and/or position 5 of the amino acid sequence set forth in SEQ ID NO: 48, preferably the amino acid mutation is an amino acid substitution, more preferably a conservative amino acid substitution. Preferably, the variant 3 is an amino acid sequence having an N4A/F/Y/V/N and/or S5N mutation in the amino acid sequence set forth in SEQ ID NO: 48. More preferably, the variant 3 is an amino acid sequence set forth in any one of SEQ ID NOs: 49-53.

In some technical solutions of the present invention, in the antigen-binding protein targeting CD40, the LCDR1 comprises the amino acid sequence set forth in SEQ ID NO: 38, the LCDR2 comprises the amino acid sequence set forth in SEQ ID NO: 43, and the LCDR3 comprises amino acid sequence 3 set forth in any one of SEQ ID NOs: 48-53, the HCDR1 comprises the amino acid sequence set forth in SEQ ID NO: 8, the HCDR2 comprises the amino acid sequence set forth in SEQ ID NO: 18, and the HCDR3 comprises the amino acid sequence set forth in SEQ ID NO: 27. See Table 1-1 for specific CDR combinations.

In some technical solutions of the present invention, in the antigen-binding protein targeting CD40, the VL comprises the amino acid sequence set forth in SEQ ID NO: 68 or an amino acid sequence having at least 80%, 85%, 90%, 92%, 94%, 95%, 96%, 97%, 98% or 99% identity to SEQ ID NO: 68, and the VH comprises the amino acid sequence set forth in SEQ ID NO: 60 or an amino acid sequence having at least 80%, 85%, 90%, 92%, 94%, 95%, 96%, 97%, 98% or 99% identity to SEQ ID NO: 60; more preferably, the VL comprises the amino acid sequence set forth in any one of SEQ ID NOs: 68-73, and the VH comprises the amino acid sequence set forth in SEQ ID NO: 60. See Table 1-2 for specific VL and VH combinations.

In some technical solutions of the present invention, the antigen-binding protein targeting CD40 is a full-length antibody comprising a light chain and a heavy chain, the light chain comprises a light chain constant region (CL), preferably the light chain constant region is a light chain constant region of human antibody, more preferably the light chain constant region of the human antibody is a light chain constant region of κ subtype, the heavy chain comprises a heavy chain constant region (CH), and the heavy chain constant region is preferably a heavy chain constant region of human antibody, more preferably a heavy chain constant region of hIgG1, hIgG2, hIgG3 or hIgG4 subtype, and further preferably a heavy chain constant region of hIgG1 subtype.

In some technical solutions of the present invention, the antigen-binding protein targeting CD40 is a full-length antibody comprising a light chain and a heavy chain, the light chain comprises the amino acid sequence set forth in any one of SEQ ID NOs: 87-92, and the heavy chain comprises the amino acid sequence set forth in SEQ ID NO: 78 or 84. Preferably, the light chain comprises the amino acid sequence set forth in SEQ ID NO: 87, and the heavy chain comprises the amino acid sequence set forth in SEQ ID NO: 78, or, the light chain comprises the amino acid sequence set forth in any one of SEQ ID NOs: 88-92, and the heavy chain comprises the amino acid sequence set forth in SEQ ID NO: 84. See Table 1-3 for specific combinations of light chain and heavy chain.

In some technical solutions of the present invention, the first protein functional region is an immunoglobulin comprising the antigen-binding protein targeting CD40, and the second protein functional region comprises one, two or more VHs of the antigen-binding protein targeting PD-L1.

In some technical solutions of the present invention, the VHs of the antigen-binding protein targeting PD-L1 are directly linked to the immunoglobulin; or, the VHs are linked to the immunoglobulin via a linker; or, when the number of the VHs is greater than 1, each of the VHs can be linked to the immunoglobulin directly or via a linker, respectively.

In some technical solutions of the present invention, when the VHs are linked to the immunoglobulin via a linker, the linker is selected from any one of the amino acid sequences set forth in GS, GGS and SEQ ID NOs: 100-106.

In some technical solutions of the present invention, the second protein functional region comprises two VHs of the antigen-binding protein targeting PD-L1. Preferably, the two VHs comprised in the second protein functional region are identical; and/or, the C-termini of the two VHs are linked to the N-termini of the two heavy chains in the immunoglobulin via a linker set forth in GGS or SEQ ID NO: 5, respectively.

In some technical solutions of the present invention, the first protein functional region comprises two light chains comprising the amino acid sequence set forth in any one of SEQ ID NOs: 88-92, and two heavy chains comprising the amino acid sequence set forth in SEQ ID NO: 84, the second protein functional region comprises two VHs comprising the amino acid sequence set forth in any one of SEQ ID NOs: 79-83, and the C-termini of the two VHs are linked to the N-termini of the two heavy chains in the first protein functional region via a linker set forth in GGS or SEQ ID NO: 5, respectively.

Preferably, the antigen-binding protein targeting PD-L1 and CD40 comprises a first polypeptide chain and a second polypeptide chain as shown below:
a second polypeptide chain consisting of the amino acid sequence set forth in SEQ ID NO: 89, and a first polypeptide chain consisting of the amino acid sequence set forth in SEQ ID NO: 93; or, a second polypeptide chain consisting of the amino acid sequence set forth in SEQ ID NO: 88, and a first polypeptide chain consisting of the amino acid sequence set forth in SEQ ID NO: 94; or, a second polypeptide chain consisting of the amino acid sequence set forth in SEQ ID NO: 91, and a first polypeptide chain consisting of the amino acid sequence set forth in SEQ ID NO: 94; or, a second polypeptide chain consisting of the amino acid sequence set forth in SEQ ID NO: 90, and a first polypeptide chain consisting of the amino acid sequence set forth in SEQ ID NO: 93; or, a second polypeptide chain consisting of the amino acid sequence set forth in SEQ ID NO: 92, and a first polypeptide chain consisting of the amino acid sequence set forth in SEQ ID NO: 93; or, a second polypeptide chain consisting of the amino acid sequence set forth in SEQ ID NO: 88, and a first polypeptide chain consisting of the amino acid sequence set forth in SEQ ID NO: 95; or, a second polypeptide chain consisting of the amino acid sequence set forth in SEQ ID NO: 88, and a first polypeptide chain consisting of the amino acid sequence set forth in SEQ ID NO: 96; or, a second polypeptide chain consisting of the amino acid sequence set forth in SEQ ID NO: 88, and a first polypeptide chain consisting of the amino acid sequence set forth in SEQ ID NO: 97; or, a second polypeptide chain consisting of the amino acid sequence set forth in SEQ ID NO: 91, and a first polypeptide chain consisting of the amino acid sequence set forth in SEQ ID NO: 95; or, a second polypeptide chain consisting of the amino acid sequence set forth in SEQ ID NO: 91, and a first polypeptide chain consisting of the amino acid sequence set forth in SEQ ID NO: 93; or, a second polypeptide chain consisting of the amino acid sequence set forth in SEQ ID NO: 91, and a first polypeptide chain consisting of the amino acid sequence set forth in SEQ ID NO: 96; or, a second polypeptide chain consisting of the amino acid sequence set forth in SEQ ID NO: 89, and a first polypeptide chain consisting of the amino acid sequence set forth in SEQ ID NO: 97; or, a second polypeptide chain consisting of the amino acid sequence set forth in SEQ ID NO: 90, and a first polypeptide chain consisting of the amino acid sequence set forth in SEQ ID NO: 97; or, a second polypeptide chain consisting of the amino acid sequence set forth in SEQ ID NO: 88, and a first polypeptide chain consisting of the amino acid sequence set forth in SEQ ID NO: 98; or, a second polypeptide chain consisting of the amino acid sequence set forth in SEQ ID NO: 88, and a first polypeptide chain consisting of the amino acid sequence set forth in SEQ ID NO: 99; or, a second polypeptide chain consisting of the amino acid sequence set forth in SEQ ID NO: 91, and a first polypeptide chain consisting of the amino acid sequence set forth in SEQ ID NO: 98; or, a second polypeptide chain consisting of the amino acid sequence set forth in SEQ ID NO: 91, and a first polypeptide chain consisting of the amino acid sequence set forth in SEQ ID NO: 99.

In the present invention, the variant 1, variant 2, variant 3, variant 4, variant 5, variant 6, variant 7, variant 8 and variant 9 are only used to distinguish different variants, the first protein functional region and the second protein functional region are only used to distinguish different protein functions, the first polypeptide chain and the second polypeptide chain are only used to distinguish different polypeptide chains, and the numbers and serial numbers do not indicate the actual order they represent.

The amino acid sequence of the CDR in the present invention is determined according to rules of Chothia definition.

In order to solve the above-mentioned technical problems, another aspect of the present invention provides a chimeric antigen receptor (CAR) comprising the antigen-binding protein targeting PD-L1 and CD40 of the present invention.

In order to solve the above-mentioned technical problems, another aspect of the present invention provides an isolated nucleic acid encoding the antigen-binding protein targeting PD-L1 and CD40 of the present invention.

In order to solve the above-mentioned technical problems, another aspect of the present invention provides a recombinant expression vector comprising the isolated nucleic acid of the present invention. Preferably, the recombinant expression vector comprises a eukaryotic cell expression vector and/or a prokaryotic cell expression vector.

In order to solve the above-mentioned technical problems, another aspect of the present invention provides a transformant comprising the isolated nucleic acid of the present invention or the recombinant expression vector of the present invention. Preferably, the host cell of the transformant is a prokaryotic cell and/or a eukaryotic cell, the prokaryotic cell is preferably an *E. coli* cell such as a TG1 or a BL21, and the eukaryotic cell is preferably an HEK293 cell or a CHO cell.

In order to solve the above-mentioned technical problems, another aspect of the present invention provides a method for preparing the antigen-binding protein targeting PD-L1 and CD40 of the present invention, which comprises the following steps: culturing the transformant of the present invention, and obtaining the antigen-binding protein targeting PD-L1 and CD40 from the culture.

In order to solve the above-mentioned technical problems, another aspect of the present invention provides an antibody-drug conjugate (ADC) comprising the antigen-binding protein targeting PD-L1 and CD40 of the present invention, and a cytotoxic agent or a label. Preferably, the cytotoxic agent is MMAF or MMAE, and the label is a fluorescent agent.

In order to solve the above-mentioned technical problems, another aspect of the present invention provides a genetically modified cell expressing the chimeric antigen receptor of the present invention. Preferably, the genetically modified cell is a eukaryotic cell, preferably an isolated human cell, more preferably an immune cell such as T cell or NK cell.

In order to solve the above-mentioned technical problems, another aspect of the present invention provides a pharmaceutical composition comprising one or more of the antigen-binding protein targeting PD-L1 and CD40 of the present invention, the chimeric antigen receptor of the present invention, the isolated nucleic acid of the present invention, the recombinant expression vector of the present invention, the genetically modified cell of the present invention and the antibody-drug conjugate of the present invention, and/or a pharmaceutically acceptable carrier. Preferably, the pharmaceutical composition further comprises other anti-tumor drugs as active ingredients. The anti-tumor drugs include, but are not limited to: a chemotherapeutic drug, a nucleotide drug, a small molecule targeted drug, a monoclonal antibody drug, a bi/multi-specific antibody drug, a recombinant protein drug, an immunomodulatory drug, a cell therapy drug and a gene therapy drug.

In order to solve the above-mentioned technical problems, another aspect of the present invention provides a detection reagent comprising the antigen-binding protein targeting PD-L1 and CD40 of the present invention and/or the antibody-drug conjugate of the present invention. Preferably, the detection reagent is in a liquid dosage form, a gaseous dosage form, a solid dosage form and a semi-solid dosage form. More preferably, the detection reagent further comprises a secondary antibody, CD40 or a derivative thereof, the secondary antibody is, for example, an anti-human IgG antibody conjugated to horseradish peroxidase and an anti-human IgG antibody conjugated to biotin.

In order to solve the above-mentioned technical problems, another aspect of the present invention provides a kit of parts comprising kit A containing one or more of the antigen-binding protein targeting PD-L1 and CD40 of the present invention, the chimeric antigen receptor of the present invention, the pharmaceutical composition of the present invention, the detection reagent of the present invention, the genetically modified cell of the present invention and the antibody-drug conjugate of the present invention.

Preferably, the kit of parts further comprises kit B containing other anti-tumor antibodies or a pharmaceutical composition comprising the other anti-tumor antibodies, and/or one or more selected from the group consisting of a hormone preparation, a targeting small molecule preparation, a proteasome inhibitor, an imaging agent, a diagnostic agent, a chemotherapeutic agent, an oncolytic drug, a cytotoxic agent, a cytokine, an activator of a costimulatory molecule, an inhibitor of an inhibitory molecule, and a vaccine.

In order to solve the above-mentioned technical problems, another aspect of the present invention provides the use of one or more of the antigen-binding protein targeting PD-L1 and CD40 of the present invention, the chimeric antigen receptor of the present invention, the isolated nucleic acid of the present invention, the recombinant expression vector of the present invention, the pharmaceutical composition of the present invention, the detection reagent of the present invention, the kit of parts of the present invention, the genetically modified cell of the present invention and the antibody-drug conjugate of the present invention in the preparation of a drug for diagnosing, preventing and/or treating PD-L1 and/or CD40 related diseases. Preferably, the PD-L1 and/or CD40 related diseases are PD-L1 related tumors, CD40 related tumors or PD-L1×CD40 related tumors. Preferably, the CD40 related tumors include solid tumors and hematologic malignancies, and the PD-L1 related tumors include solid tumors and hematologic malignancies. More preferably, the CD40 related tumors include B-cell NHL, chronic lymphocytic leukemia (CLL), hairy cell leukemia (HCL), Hodgkin's disease, multiple myeloma, bladder cancer, kidney cancer, ovarian cancer, cervical cancer, breast cancer, lung cancer, nasopharyngeal cancer, malignant melanoma, pancreatic cancer and colon cancer; the PD-L1 related tumors include squamous cell carcinoma, myeloma, small cell lung cancer, non-small cell lung cancer (NSCLC), head and neck squamous cell carcinoma (HNSCC), glioma, Hodgkin's lymphoma, non-Hodgkin's lymphoma, diffuse large B-cell lymphoma (DLBCL), follicular lymphoma, acute lymphoblastic leukemia (ALL), acute myeloid leukemia (AML), chronic lymphocytic leukemia (CLL), chronic myeloid leukemia (CML), primary mediastinal large B-cell lymphoma, mantle cell lymphoma (MCL), small lymphocytic lymphoma (SLL), T-cell/histiocyte-rich large B-cell lymphoma, multiple myeloma, myeloid cell leukemia-1 protein (Mcl-1), myelodysplastic syndrome (MDS), gastrointestinal cancer, kidney cancer, ovarian cancer, liver cancer, lymphoblastic leukemia, lymphocytic leukemia, colorectal cancer, endometrial cancer, kidney cancer, prostate cancer, thyroid cancer, melanoma, chondrosarcoma, neuroblastoma, pancreatic cancer, glioblastoma multiforme, gastric cancer, bone cancer, Ewing's sarcoma, cervical cancer, brain cancer, stomach cancer, bladder cancer, hepatoma, breast cancer, colon cancer, hepatocellular carcinoma (HCC), clear cell renal cell carcinoma (RCC), head and neck cancer, throat cancer, hepatobiliary cancer, central nervous system cancer, esophageal cancer, malignant pleural mesothelioma, systemic light chain amyloidosis, lymphoplasmacytic lymphoma, myelodysplastic syndrome, myeloproliferative neoplasm, neuroendocrine tumor, Merkel cell carcinoma, testicular cancer and skin cancer, preferably colon cancer with high expression of PD-L1.

In order to solve the above-mentioned technical problems, another aspect of the present invention provides a method for detecting PD-L1 and/or CD40 in a sample, which comprises the step of contacting the sample with one or more of the antigen-binding protein targeting PD-L1 and CD40 of the present invention, the detection reagent of the present invention, and the antibody-drug conjugate of the present invention. The sample comprises, for example, a blood sample (e.g., a whole blood sample and a serum sample) and a reagent comprising PD-L1 and/or CD40. Preferably, the method is for a non-therapeutic/diagnostic purpose, such as in scientific research, detecting the concentration of PD-L1 and/or CD40 standards, whether other reagents are contaminated by PD-L1 and/or CD40, etc.

In order to solve the above-mentioned technical problems, another aspect of the present invention provides a method for diagnosing, treating and/or preventing PD-L1 and/or CD40 related diseases, the method comprising administering to a patient in need thereof a therapeutically effective amount of one or more of the antigen-binding protein targeting PD-L1 and CD40 of the present invention, the chimeric antigen receptor of the present invention, the isolated nucleic acid of the present invention, the recombinant expression vector of the present invention, the pharmaceutical composition of the present invention, the detection reagent of the present invention, the genetically modified cell of the present invention, and the antibody-drug conjugate of the present invention, or using the kit of parts of the present invention to diagnose or treat the patient in need thereof. Preferably, the PD-L1 and/or CD40 related diseases are PD-L1 related tumors, CD40 related tumors or PD-L1×CD40 related tumors. Preferably, the CD40 related tumors include solid tumors and hematologic malignancies, and the PD-L1 related tumors include solid tumors and hematologic malignancies. More preferably, the CD40 related tumors include B-cell NHL, chronic lymphocytic leukemia (CLL), hairy cell leukemia (HCL), Hodgkin's disease, multiple myeloma, bladder cancer, kidney cancer, ovarian cancer, cervical cancer, breast cancer, lung cancer, nasopharyngeal cancer, malignant melanoma, pancreatic cancer and colon cancer; the PD-L1 related tumors include squamous cell carcinoma, myeloma, small cell lung cancer, non-small cell lung cancer (NSCLC), head and neck squamous cell carcinoma (HNSCC), glioma, Hodgkin's lymphoma, non-Hodgkin's lymphoma, diffuse large B-cell lymphoma (DLBCL), follicular lymphoma, acute lymphoblastic leukemia (ALL), acute myeloid leukemia (AML), chronic lymphocytic leukemia (CLL), chronic myeloid leukemia (CML), primary mediastinal large B-cell lymphoma, mantle cell lymphoma (MCL), small lymphocytic lymphoma (SLL), T-cell/histiocyte-rich large B-cell lymphoma, multiple myeloma, myeloid cell leukemia-1 protein (Mcl-1), myelodysplastic syndrome (MDS), gastrointestinal cancer, kidney cancer, ovarian cancer, liver cancer, lymphoblastic leukemia, lymphocytic leukemia, colorectal cancer, endometrial cancer, kidney cancer, prostate cancer, thyroid cancer, melanoma, chondrosarcoma, neuroblastoma, pancreatic cancer, glioblastoma multiforme, gastric cancer, bone cancer, Ewing's sarcoma, cervical cancer, brain cancer, stomach cancer, bladder cancer, hepatoma, breast cancer, colon cancer, hepatocellular carcinoma (HCC), clear cell renal cell carcinoma (RCC), head and neck cancer, throat cancer, hepatobiliary cancer, central nervous system cancer, esophageal cancer, malignant pleural mesothelioma, systemic light chain amyloidosis, lymphoplasmacytic lymphoma, myelodysplastic syndrome, myeloproliferative neoplasm, neuroendocrine tumor, Merkel cell carcinoma, testicular cancer and skin cancer, preferably colon cancer with high expression of PD-L1.

In order to solve the above-mentioned technical problems, another aspect of the present invention provides the antigen-binding protein targeting PD-L1 and CD40 of the present invention, the chimeric antigen receptor of the present invention, the isolated nucleic acid of the present invention, the recombinant expression vector of the present invention, the pharmaceutical composition of the present invention, the detection reagent of the present invention, the kit of parts of the present invention, the genetically modified cell of the present invention and the antibody-drug conjugate of the present invention, for use in diagnosing, preventing and/or treating PD-L1 and/or CD40 related diseases. Preferably, the PD-L1 and/or CD40 related diseases are PD-L1 related tumors, CD40 related tumors or PD-L1×CD40 related tumors. Preferably, the CD40 related tumors include solid tumors and hematologic malignancies, and the PD-L1 related tumors include solid tumors and hematologic malignancies. More preferably, the CD40 related tumors include B-cell NHL, chronic lymphocytic leukemia (CLL), hairy cell leukemia (HCL), Hodgkin's disease, multiple myeloma, bladder cancer, kidney cancer, ovarian cancer, cervical cancer, breast cancer, lung cancer, nasopharyngeal cancer, malignant melanoma, pancreatic cancer and colon cancer; the PD-L1 related tumors include squamous cell carcinoma, myeloma, small cell lung cancer, non-small cell lung cancer (NSCLC), head and neck squamous cell carcinoma (HNSCC), glioma, Hodgkin's lymphoma, non-Hodgkin's lymphoma, diffuse large B-cell lymphoma (DLBCL), follicular lymphoma, acute lymphoblastic leukemia (ALL), acute myeloid leukemia (AML), chronic lymphocytic leukemia (CLL), chronic myeloid leukemia (CML), primary mediastinal large B-cell lymphoma, mantle cell lymphoma (MCL), small lymphocytic lymphoma (SLL), T-cell/histiocyte-rich large B-cell lymphoma, multiple myeloma, myeloid cell leukemia-1 protein (Mcl-1), myelodysplastic syndrome (MDS), gastrointestinal cancer, kidney cancer, ovarian cancer, liver cancer, lymphoblastic leukemia, lymphocytic leukemia, colorectal cancer, endometrial cancer, kidney cancer, prostate cancer, thyroid cancer, melanoma, chondrosarcoma, neuroblastoma, pancreatic cancer, glioblastoma multiforme, gastric cancer, bone cancer, Ewing's sarcoma, cervical cancer, brain cancer, stomach cancer, bladder cancer, hepatoma, breast cancer, colon cancer, hepatocellular carcinoma (HCC), clear cell renal cell carcinoma (RCC), head and neck cancer, throat cancer, hepatobiliary cancer, central nervous system cancer, esophageal cancer, malignant pleural mesothelioma, systemic light chain amyloidosis, lymphoplasmacytic lymphoma, myelodysplastic syndrome, myeloproliferative neoplasm, neuroendocrine tumor, Merkel cell carcinoma, testicular cancer and skin cancer, preferably colon cancer with high expression of PD-L1.

In the present invention, the term "with 3, 2 or 1 amino acid mutation" refers to including amino acid insertion, deletion or substitution occurring on the basis of the original amino acid sequence. An exemplary explanation is that the mutation of CDRs can comprise 3, 2 or 1 amino acid mutation, and the same or a different number of amino acid residues can optionally be selected for mutation between these CDRs, for example, it can be 1 amino acid mutation for CDR1, but no amino acid mutation for CDR2 and CDR3.

In the present invention, the term "PTM site mutation" refers to an amino acid mutation at the PTM site in the sequence of a variant comparing to the original amino acid sequence. Depending on different antibody sequences and different PTM sequence patterns, there are different mutation design methods. One method is to replace the "hot spot" amino acid (such as N or S in the NS pattern) with an amino acid having similar physicochemical properties (e.g., mutation of N to Q). If the PTM sequence pattern is derived from somatic hypermutation and is not present in the germline gene sequences, another method may be to replace the sequence pattern with the corresponding germline gene sequence.

In the present invention, the VH, VL or the full-length antibody can comprise mutations on the basis of the defined sequence. The mutation is a deletion, substitution or addition of one or more amino acid residues occurring in the defined amino acid sequence, and the mutated amino acid sequence has at least 80% sequence identity to the defined amino acid sequence, and maintains or improves the binding activity of the antigen-binding protein comprising the mutated amino acid sequence; The at least 80% sequence identity is preferably at least 85% sequence identity or at least 90% sequence identity; more preferably at least 95% sequence identity; most preferably at least 99% sequence identity.

In the present invention, "include", "comprise" and "is/are" have the same meaning in certain specific embodiments.

On the basis of meeting common knowledge in the art, the above-mentioned various preferred conditions can be combined in any form, such that various preferred examples of the present invention are obtained.

Reagents and raw materials used in the present invention are all commercially available.

The present invention has the positive improvement effects as follows.

The antigen-binding protein targeting CD40 of the present invention have properties of high affinity for CD40 and strong agonistic activity on signaling pathway, especially the enhanced agonistic activity after crosslinking, such that the antigen-binding protein targeting CD40 of the present invention has a greater treatment window and provides a good basis for significantly improving patient response rate under safe and tolerable dosage conditions in clinical trials. The antigen-binding protein targeting PD-L1 of the present invention has a high affinity for PD-L1 and can effectively inhibit the biological activity of PD-L1. On this basis, the antigen-binding protein targeting PD-L1 and CD40 of the present invention has excellent anti-tumor efficacy, good safety, and outstanding druggability. The antigen-binding protein targeting PD-L1 and CD40 of the present invention achieves a significant synergistic anti-tumor effect by means of blocking a PD-1/PD-L1 inhibitory signaling pathway, acting on an activated CD40 receptor, simultaneously activating an antigen-presenting cell and a lymphocyte, enhancing the presentation of tumor antigen by the antigen-presenting cell to the lymphocyte, and promoting the response of lymphocyte; meanwhile, the activation of an immune cell by the antigen-binding protein targeting PD-L1 and CD40 only occurs at a tumor microenvironment site, so that problems of systemic drug toxicity and side effects are significantly solved, which enables the antigen-binding protein to exert an excellent anti-tumor efficacy under very safe conditions. At present, there is no new antibody drug for the target combination on the market at home or abroad. The present invention is expected to bring a new opportunity for the treatment of various tumors.

### Brief Description of the Drawings

FIG. 1A-FIG. 1D respectively show the levels of binding of some CD40 antibodies of the present invention to Raji cells highly expressing human CD40.

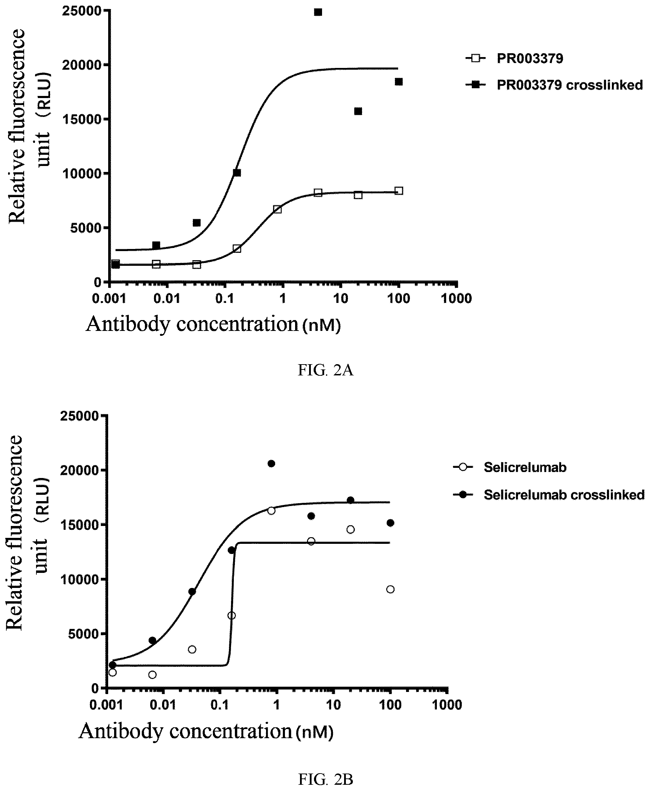

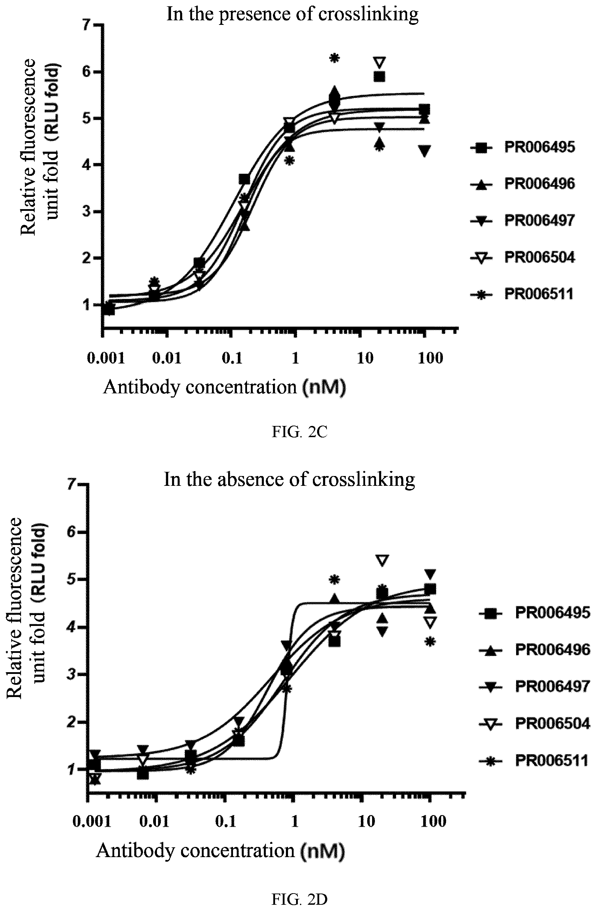

FIG. 2A shows the activation effect of PR003379 in the presence of or in the absence of crosslinking mediated by the cell expressing human CD32B on HEK293-hCD40-NFκB fluorescent reporter gene cells, FIG. 2B shows the activation effect of control Selicrelumab in the presence of or in the absence of crosslinking mediated by the cell expressing CD32B on HEK293-hCD40-NFκB fluorescent reporter gene cells, FIG. 2C shows the activation effect of other CD40 antibodies in the presence of crosslinking mediated by the cell expressing CD32B on HEK293-hCD40-NFxB fluorescent reporter gene cells, and FIG. 2D shows the activation effect of other CD40 antibodies in the absence of crosslinking mediated by the cell expressing human CD32B on HEK293-hCD40-NFκB fluorescent reporter gene cells.
FIG. 3A and FIG. 3B respectively show the levels of binding of some PD-L1 antibodies of the present invention to the CHO-K1 cell highly expressing human PD-L1, CHO-K1/hPD-L1.
FIG. 4A and FIG. 4B respectively show the inhibitory effects of some PD-L1 antibodies of the present invention on the PD-1 signaling pathway in reporter gene cell experiments.
FIG. 5 shows a schematic diagram of the molecular structure of the PD-L1×CD40 bispecific binding protein.
FIG. 6A and FIG. 6B respectively show the levels of binding of some PD-L1×CD40 bispecific binding proteins of the present invention to human PD-L1 cells, CHO-K1/hPD-L1.
FIG. 7A and FIG. 7B respectively show the levels of binding of some PD-L1×CD40 bispecific binding proteins of the present invention to cynomolgus monkey PD-L1 cells, CHO-K1/cyPD-L.
FIG. 8A and FIG. 8B respectively show the levels of binding of some PD-L1×CD40 bispecific binding proteins of the present invention to human CD40 cells, CHO-K1/hCD40.
FIG. 9A and FIG. 9B respectively show the levels of binding of some PD-L1×CD40 bispecific binding proteins of the present invention to cynomolgus monkey CD40 cells, CHO-K1/cyCD40.
FIG. 10A and FIG. 10B respectively show that some PD-L1×CD40 bispecific binding proteins of the present invention do not bind to CHO-K1 cells.
FIG. 11A and FIG. 11B respectively show the levels of binding of some PD-L1×CD40 bispecific binding proteins of the present invention to NCI-H226 cells highly expressing human PD-L1.
FIG. 12A and FIG. 12B respectively show the levels of binding of some PD-L1×CD40 bispecific binding proteins of the present invention to Raji cells highly expressing human CD40.
FIG. 13A and FIG. 13B respectively show the levels of binding of some PD-L1×CD40 bispecific binding proteins of the present invention to MDA-MB-231 cells expressing human PD-L1 and human CD40.
Figs. 14A and 14B show the first batch of experiment results of the inhibitory effects of the PD-L1×CD40 bispecific binding proteins on the PD-1 signaling pathway in reporter gene cell experiments, and Figs. 14C and 14D show the second batch of experiment results of the inhibitory effects of the PD-L1×CD40 bispecific binding proteins on the PD-1 signaling pathway in reporter gene cell experiments.
FIG. 15A-FIG. 15C show the first batch of experiment results of the activation effect of PD-L1×CD40 bispecific binding proteins on the CD40 signaling pathway in reporter gene cell experiments, FIG. 15D and FIG. 15E show the second batch of experiment results of the activation effect of PD-L1×CD40 bispecific binding proteins on the CD40 signaling pathway in reporter gene cell experiments, and FIG. 15F-FIG. 15K show the third batch of experiments results of the activation effect of PD-L1 ×CD40 bispecific binding proteins on the CD40 signaling pathway in reporter gene cell experiments.
FIG. 16A and FIG. 16B respectively show the secretion levels of IL12p40 promoted by activation of human DC cells (donor #1) by some PD-L1×CD40 bispecific binding proteins of the present invention.
FIG. 17A and FIG. 17B respectively show the secretion levels of IL12p40 promoted by activation of human DC cells (donor #2) by some PD-L1×CD40 bispecific binding proteins of the present invention.
FIG. 18A and FIG. 18B respectively show the secretion levels of IL12p40 promoted by activation of human DC cells (donor #3) by some PD-L1×CD40 bispecific binding proteins of the present invention.
FIGs. 19A-19B show the secretion levels of IL12p40 in iDC cells promoted by activation of human DC cells (donor #4) by PD-L1×CD40 bispecific binding proteins, and Figs. 19C-19D show the secretion levels of IL12p40 in mDC cells promoted by activation of human DC cells (donor #4) by PD-L1×CD40 bispecific binding proteins.
FIG. 20 shows the proliferation levels of human B cells (donor #1) promoted by PD-L1×CD40 bispecific binding proteins.
FIG. 21 shows the proliferation levels of human B cells (donor #2) promoted by PD-L1×CD40 bispecific binding proteins.
FIG. 22A and FIG. 22B respectively show the proliferation levels of human B cells (donor #3) promoted by some PD-L1×CD40 bispecific binding proteins of the present invention.
FIG. 23A and FIG. 23B respectively show the proliferation levels of human B cells (donor #4) promoted by some PD-L1 ×CD40 bispecific binding proteins of the present invention.
FIG. 24A and FIG. 24B respectively show the proliferation levels of human B cells (donor #5) promoted by some PD-L1 ×CD40 bispecific binding proteins of the present invention.
FIG. 25A shows the changes in tumor volume during the in vivo anti-tumor efficacy experiment of PD-L1×CD40 bispecific binding proteins in mice (mouse efficacy experiment #1), and FIG. 25B shows the changes in mouse weight during the in vivo anti-tumor efficacy experiment of PD-L1×CD40 bispecific binding proteins in mice (mouse efficacy experiment #1).
FIG. 26A shows the changes in tumor volume during the in vivo anti-tumor efficacy experiment of PD-L1×CD40 bispecific binding proteins in mice (mouse efficacy experiment #2), and FIG. 26B shows the changes in mouse weight during the in vivo anti-tumor efficacy experiment of PD-L1×CD40 bispecific binding proteins in mice (mouse efficacy experiment #2).
FIG. 27A shows the changes in tumor volume during the in vivo anti-tumor efficacy experiment of PD-L1×CD40 bispecific binding proteins in mice (mouse efficacy experiment #3), and FIG. 27B shows the changes in mouse weight during the in vivo anti-tumor efficacy experiment of PD-L1×CD40 bispecific binding proteins in mice (mouse efficacy experiment #3).
FIG. 28 shows the effects of PD-L1×CD40 bispecific binding proteins and Selicrelumab on mouse liver function indicators ALT and AST in mouse efficacy experiment #3.
FIG. 29 shows the results of staining analysis of mouse liver tissue sections in mouse efficacy experiment #3.
FIG. 30A shows the changes in tumor volume during the in vivo anti-tumor efficacy experiment of PD-L1×CD40 bispecific binding proteins and Selicrelumab in mice (mouse efficacy experiment #4), and FIG. 30B shows the changes in mouse weight during the in vivo anti-tumor efficacy experiment of PD-L1×CD40 bispecific binding proteins and Selicrelumab in mice (mouse efficacy experiment #4).
FIG. 31 shows the effects of PD-L1×CD40 bispecific binding proteins and Selicrelumab on mouse liver function indicators ALT and AST in mouse efficacy experiment #4.
FIG. 32 shows the cytokine (IL-6 and IFN-γ) levels in peripheral blood of mice in mouse efficacy experiment #4.
FIG. 33 shows the cytokine (TNF-α and IL12p40) levels in peripheral blood of mice in mouse efficacy experiment #4.
FIG. 34 shows the results of staining analysis of mouse liver tissue sections in mouse efficacy experiment #4.
FIG. 35A shows the pharmacokinetic of the PR007619PD-L1 ×CD40 bispecific binding protein in mice, FIG. 35B shows the pharmacokinetic of the PR007556PD-L1×CD40 bispecific binding protein in mice, FIG. 35C shows the pharmacokinetic of the PR007281PD-L1×CD40 bispecific binding protein in mice, and FIG. 35D shows the pharmacokinetic of the PR007276PD-L1×CD40 bispecific binding protein in mice, in which Total refers to Fc-end detection, and Free refers to PD-L1-end detection.

### Detailed Description of Embodiments

The embodiments of the present invention are illustrated below by using the specific examples, and those skilled in the art would have readily understood other advantages and effects of the present invention from the content disclosed in this description.

In the present application, the term "binding protein" or "antigen-binding protein" generally refers to a protein comprising a portion binding to an antigen and optionally a scaffold or framework portion that allows the antigen-binding portion to adopt a conformation that promotes binding of the antigen-binding protein to the antigen. It can typically comprise a light chain variable region (VL) of an antibody, a heavy chain variable region (VH) of an antibody, or both. VH and VL regions can be further divided into hypervariable regions called complementary determining regions (CDRs), which are scattered within more conserved regions called framework regions (FRs). Each VH and VL can be composed of three CDRs and four FRs, which can be arranged from an amino terminus to a carboxyl terminus in the following order: FR-1, CDR1, FR-2, CDR2, FR-3, CDR3 and FR-4. The variable regions of the heavy and light chains contain binding domains that interact with antigens. Three CDRs of VH are denoted as HCDR1, HCDR2, and HCDR3, respectively, and can also be denoted as VH CDR1, VH CDR2, and VH CDR3; and three CDRs of VL are denoted as LCDR1, LCDR2, and LCDR3, respectively, and can also be denoted as VL CDR1, VL CDR2, and VL CDR3. Examples of the antigen-binding protein include, but are not limited to, an antibody, an antigen-binding fragment (Fab, Fab', F(ab)₂, Fv fragment, F(ab')₂, scFv, di-scFv and/or dAb), an immunoconjugate, a multi-specific antibody (e.g., a bispecific antibody), an antibody fragment, an antibody derivative, an antibody analog, a fusion protein, and the like so long as they exhibit the desired antigen-binding activity.

In the present application, the amino acid sequences of the CDRs are shown according to the rules of Chothia definition. However, it is well known to those skilled in the art that the CDRs of an antibody can be defined in the art by a variety of methods, such as the rules of Kabat definition based on sequence variability (see, Kabat et al., Sequences of Proteins of Immunological Interest, fifth edition, National Institutes of Health, Bethesda, Maryland (1991)), and the rules of Chothia definition based on the location of a structural loop region (see JMol Biol 273: 927-48, 1997). In the technical solutions of the present invention, amino acid residues in variable domain sequences can also be determined according to the rules of Combined definition that incorporates both Kabat definition and Chothia definition. The rules of Combined definition refer to the combination of the ranges of Kabat definition and Chothia definition, based on which a larger scope is taken, see Table 2 below for details. It should be understood by those skilled in the art that unless otherwise specified, the terms "CDR" and "complementarity determining region" of a given antibody or region thereof (for example, a variable region) should be understood to encompass the complementarity determining region as defined by any of the above-mentioned known schemes as described in the present invention. Although the scope of protection as claimed in the present invention is based on the sequences shown according to the rules of Chothia definition, corresponding amino acid sequences according to the rules of other CDR definitions shall also fall within the scope of protection of the present invention.

**Table 2. Definition method for CDRs of antibodies of the present application**

| | Kabat | Chothia | Combined |
|---|---|---|---|
| LCDR1 | L24--L34 | L24--L34 | L24-L34 |
| LCDR2 | L50--L56 | L50--L56 | L50-L56 |
| LCDR3 | L89--L97 | L89--L97 | L89-L97 |
| HCDR1 | H31--H35 | H26--H32 | H26-H35 |
| HCDR2 | H50--H65 | H52--H56 | H50-H65 |
| HCDR3 | H95--H102 | H95--H102 | H95-H102 |

Laa-Lbb may refer to an amino acid sequence from position aa (Chothia numbering) to position bb (Chothia numbering), starting from the N-terminus of an antibody light chain; and Haa-Hbb may refer to an amino acid sequence from position aa (Chothia numbering) to position bb (Chothia numbering), starting from the N-terminus of an antibody heavy chain. For example, L24-L34 may refer to an amino acid sequence from position 24 to position 34 according to Chothia numbering, starting from the N-terminus of an antibody light chain; H26-H32 may refer to an amino acid sequence from position 26 to position 32 according to the Chothia coding rules, starting from the N-terminus of an antibody heavy chain. It should be known by those skilled in the art, when Chothia numbering scheme is used to number CDR, there may be cases where insertion sites are present at some locations (see http://bioinf.org.uk/abs/).

In the present application, the term "monoclonal antibody" generally refers to an antibody obtained from a population of substantially homogeneous antibodies, that is, individual antibodies in the population are the same, except for the possible small amounts of natural mutations. The monoclonal antibody is usually highly specific for a single antigenic site. Furthermore, in contrast to a conventional polyclonal antibody preparation (which usually have different antibodies for different determinants), each monoclonal antibody is for a single determinant on the antigen. In addition to the specificity the monoclonal antibody, the monoclonal antibody has the advantage that it can be synthesized by hybridoma culture and is not contaminated by other immunoglobulins. The modifier "monoclonal" indicates the characteristic of the antibody obtained from a population of substantially homogeneous antibodies, and is not to be construed as requiring production of the antibody by any particular method. For example, the monoclonal antibody used according to the present invention can be prepared in hybridoma cells, or can be prepared by the recombinant DNA method.

In the present application, the term "fully human antibody" generally refers to an antibody expressed in a genetically engineered antibody gene-deficient animal, to which all human antibody-encoding genes are transferred. All parts of the antibody, including variable and constant regions of the antibody, are encoded by human-derived genes. The fully human antibody can greatly reduce the immune side effects caused by a heterologous antibody to the human body. Methods for obtaining the fully human antibody in the art can include phage display technique, transgenic mouse technique etc.

In the present application, the term "specific binding" generally refers to the binding of an antibody to an epitope by the antigen binding domain of the antibody, and the binding requires some complementarity between the antigen binding domain and the epitope. According to the definition, an antibody is said to "specifically bind" to an antigen when the antibody more likely to bind to an epitope by the antigen binding domain of the antibody than to bind to a random, unrelated epitope. The term "epitope" refers to a specific group of atoms (e.g., sugar side chain, phosphoryl and sulfonyl) or an amino acid on an antigen that binds to an antigen-binding protein (e.g., an antibody).

In the present application, the term "Fab" generally refers to the antigen-binding portion of a conventional antibody (e.g., IgG), including the heavy chain variable region VH, light chain variable region VL, heavy chain constant region domain CH1, and light chain constant region CL of the antibody. In a conventional antibody, the C-terminus of VH is linked to the N-terminus of CH1 to form the heavy chain Fd fragment, the C-terminus of VL is linked to the N-terminus of CL to form the light chain, and the C-terminus of CH1 is further linked to the hinge region and other constant region domains of the heavy chain to form the heavy chain. In some embodiments, "Fab" also refers to the variant structure of Fab. For example, in certain embodiments, the C-terminus of VH is linked to the N-terminus of CL to form one polypeptide chain, and the C-terminus of VL is linked to the N-terminus of CH1 to form another polypeptide chain, thereby forming a Fab (cross VH/VL) structure. In certain embodiments, CH1 of Fab is not linked to the hinge region, but the C-terminus of CL is linked to the hinge region of the heavy chain to form a Fab (cross Fd/LC) structure.

In the present application, the term "VH" generally refers to the heavy chain variable region VH domain of an antibody, that is, VH can be the heavy chain variable region VH of a conventional antibody of human or other animals (H2L2 structure), can also be the heavy chain variable region VHH of a heavy chain antibody of animal, such as camelid (HCAb structure), or can be the heavy chain variable region VH of a fully human heavy chain antibody produced by Harbour HCAb transgenic mice (HCAb structure).

In the present application, the term "antigen-binding fragment" generally refers to any protein functional region that can specifically bind to an antigen, that is, the antigen-binding fragment can be "Fab", "VH", or other antigen-binding forms (e.g., lipocalins, neural cell adhesion molecules (NCAM), fibronectin, ankyrin repeat proteins (DARPins), and other derived protein structures).

In the present application, the term "tumor antigen" can refer to either a tumor specific antigen (TSA) or a tumor associated antigen (TAA). The tumor specific antigen refers to an antigen that is unique to tumor cells and do not exist on normal cells or tissues. The tumor associated antigen is not unique to tumor cells and also exists in normal cells or tissues, but is highly expressed during tumor cell proliferation.

In the present application, the term "target cell" refers to cells that need to be eliminated, mainly tumor cells, but also immunosuppressive cells and the like.

In the present application, the term "immune effector cell" generally refers to an immune cell that participates in clearing foreign antigens and exercising effector functions in an immune response, such as a plasma cell, a cytotoxic T cell and an NK cell.

In the present application, the term "co-stimulatory molecule", "co-stimulatory molecule antigen" or "stimulatory antigen" refer to a cell surface molecule and a ligand thereof which provides a co-stimulatory signal for the full activation of an immune cell such as T or B cell, and positively regulates the activation of immune cell, such as, CD28, 4-1BB, ICOS, OX40 and CD40.

In the present application, the term "co-inhibitory molecule" or "co-inhibitory molecule antigen" refers to a class of cell surface molecules and ligands thereof which negatively regulate the function of immune cells, such as, CTLA-4, PD-L1 and PD-1.

In the present application, the term "PD-L1" generally refers to programmed death ligand 1 protein, a functional variant thereof and/or a functional fragment thereof. PD-L1 is also known as cluster of differentiation 274 (CD274) or B7 homolog 1 (B7-H1), and is a protein encoded by CD274 gene (in human). The sequence of PD-L1 is known in the art. For example, the amino acid sequence of an exemplary full-length human PD-L1 protein can be found in NCBI under the accession number of NP_054862 or UniProt under the accession number of Q9NZQ7; and the sequence of an exemplary full-length cynomolgus monkey PD-L1 protein can be found in NCBI under the accession number of XP_005581836 or Uniprot under the accession number of G7PSE7.

In the present application, the term "CD40" generally refers to tumor necrosis factor receptor superfamily member 5 protein, a functional variant thereof and/or a functional fragment thereof, also known as TNFRSF5. The sequence of CD40 is known in the art. For example, the amino acid sequence of an exemplary human CD40 protein can be found in UniProt under the accession number of P25942; the sequence of an exemplary cynomolgus monkey CD40 protein can be found in Uniprot under the accession number of G7PG38; and the sequence of an exemplary mouse CD40 protein can be found in Uniprot under the accession number of P27512. CD40L is the natural trimeric ligand molecule of CD40.

### Examples

The present invention is further described below by way of examples; however, the present invention is not limited to the scope of the described examples. The examples do not include a detailed description of conventional methods, such as methods for constructing vectors and plasmids, methods for inserting protein-encoding genes into such vectors and plasmids, or methods for introducing plasmids into host cells. Such methods are well known to an ordinary person skilled in the art and are described in numerous publications. For the experimental methods in which no specific conditions are specified in the following examples, selections are made according to conventional methods and conditions or according to the product instructions.

### Example 1. Expression, purification and characterization analysis of physicochemical properties of recombinant antibodies

### Example 1.1. Expression and purification of antibodies

In this example, the general method for preparing antibodies using techniques such as mammalian host cells (e.g., human embryonic kidney cells HEK293 or Chinese hamster ovary cells CHO and derivatives thereof), transient transfection expression and affinity capture isolation was described. The present method is suitable for an antibody of interest containing an Fc region; the antibody of interest may be composed of one or more protein polypeptide chains; and the antibody of interest may be derived from one or more expression plasmids.

The amino acid sequences of antibody polypeptide chains are converted into nucleotide sequences by codon optimization; and the nucleotide sequences for encoding are respectively synthesized and cloned onto expression vectors compatible with a host cell. The plasmids encoding the antibody polypeptide chains are simultaneously transfected into a mammalian host cell according to a particular ratio, and the recombinant antibodies having correct folding and polypeptide chain assembly can be obtained using conventional recombinant protein expression and purification techniques. Specifically, FreeStyle^{™} 293-F cells (Thermo, # R79007) were subjected to scale-up culture in a FreeStyle^{™} F17 Expression Medium (Thermo, # A1383504). Before transient transfection, the cells were adjusted to a cell concentration of 6-8 × 10⁵ cells/mL, and cultured in a shaker at 37°C and 8% CO₂ for 24 hours at a cell concentration of 1.2 × 10⁶ cells/mL. 30 mL of the cultured cells was prepared. The plasmids encoding the antibody polypeptide chains (pTT5, NRC) were mixed according to a certain ratio, a total of 30 µg of plasmids (the ratio of the plasmids to the cells being 1 µg : 1 mL) were dissolved in 1.5 mL of Opti-MEM reduced serum medium (Thermo, # 31985088), and the resulting mixture was filtered through a 0.22 µm filter membrane for sterilization. Another 1.5 mL of Opti-MEM was taken and dissolved in 120 µL of 1 mg/mL PEI (Polysciences, # 23966-2), and the resulting mixture was left to stand for 5 minutes. The PEI was slowly added to the plasmids, the resulting mixture was incubated at room temperature for 10 minutes, the plasmid and PEI mixed solution was slowly dripped into the culture flask while shaking the culture flask, and cultured in a shaker at 37°C and 8% CO₂ for 5 days. The cell viability was observed after 5 days. The culture was collected, and centrifuged at a rotary speed of 3300 g for 10 minutes, and then the supernatant was taken; and the supernatant was then centrifuged at a high speed to remove impurities. A gravity column (Bio-Rad, #7311550) containing MabSelect^{™} (GE Healthcare, #71-5020-91) was equilibrated with a PBS buffer with pH 7.4, that is, the column was rinsed with 2-5 times the column volume of the buffer. The supernatant sample was loaded onto the column; and the column was rinsed with 5-10 times the column volume of PBS buffer, the protein of interest was eluted with 0.1 M glycine with pH 3.5, then adjusted with Tris-HCl with pH 8.0 until neutrality, and finally transferred to a PBS buffer or a buffer containing other components through concentration and liquid exchange by an ultrafiltration tube (Millipore, # UFC901024) to obtain a purified recombinant antibody solution. Finally, the concentration was determined using NanoDrop (Thermo, NanoDrop^{™} One), and the purified recombinant antibody solution was subpackaged and stored for later use.

### Example 1.2. Analysis of protein purity and aggregate by SEC-HPLC

In this example, analytical size exclusion chromatography (SEC) was used to analyze the purity and aggregate form of the protein samples. An analytical chromatographic column TSKgel G3000SWxl (Tosoh Bioscience, #08541, 5 µm, 7.8 mm×30 cm) was connected to a high pressure liquid chromatograph HPLC (Agilent Technologies, Agilent 1260 Infinity II), and equilibrated with a PBS buffer (pH 7.4) for at least 1 hour at room temperature. An appropriate amount of protein sample (at least 10 µg) was filtered through a 0.22 µm filter membrane and then injected into the system, and the HPLC procedure was set: a PBS buffer (pH 7.4) was used to make the sample flow through the column at a flow rate of 1.0 mL/min for up to 25 minutes. HPLC would generate an analytical report where the retention times of components having different molecular sizes in the sample were reported.

### Example 1.3. Determination of thermal stability of protein molecules by DSF

Differential scanning fluorimetry (DSF) is a common high-throughput method for determine the thermal stability of proteins. In the method, a real-time fluorescent quantitative PCR instrument is used to monitor the change in the fluorescence intensity of the dye bound to the unfolded protein molecule to reflect the process of protein denaturation, thereby reflecting the thermal stability of the protein molecule. In this example, the thermal denaturation temperature (Tm) of the protein molecule was determined by DSF. 10 µg of protein was added to a 96-well PCR plate (Thermo, # AB-0700/W), then 2 µL of 100×diluted dye SYPROTM (Invitrogen, # 2008138) was added, and then a buffer was added to achieve a final volume of 40 µL per well. The PCR plate was sealed and placed in a real-time fluorescent quantitative PCR instrument (Bio-Rad CFX96 PCR System), incubation was first performed at 25°C for 5 minutes, then the temperature gradually increased from 25°C to 95°C at a gradient of 0.2°C/0.2 min, and the temperature decreased to 25°C at the end of the test. The FRET scanning mode was used, Bio-Rad CFX Maestro software was used to perform data analysis, and the Tm of the sample was calculated.

### Example 2. Preparation of CD40 antibodies

Experimental animals, which could be mice, rats, rabbits, sheep, camels, etc., were immunized with the CD40 antigen to obtain antibody molecules that specifically bind to CD40. Usually, the resulting antibody molecules are of non-human origin. After obtaining non-human antibodies, these molecules need to be humanized using antibody engineering techniques to reduce immunogenicity and improve druggability. However, the process of humanizing antibodies has its technical complexity, and molecules that have been humanized tend to have reduced affinity for antigens. On the other hand, advances in transgenic technology allows the breeding of genetically engineered mice that carry the human immunoglobulin repertoire and in which their endogenous murine immunoglobulin repertoires are deleted. The antibody produced by this transgenic mouse has a fully human sequence, so no further humanization is required, greatly improving the efficiency of therapeutic antibody development. Harbour H2L2 mouse (Harbour antibodies BV) is a transgenic mouse carrying the human immunoglobulin repertoire, and the antibodies produced thereby have complete human antibody variable domains and rat constant domains.

### Immunization of mice

Harbour H2L2 mice were immunized in multiple rounds with a soluble recombinant human CD40 extracellular domain fusion protein (Acrobiosystems, #CD0-H5253). The antigen protein was mixed with an immunoadjuvant to form an immunogen reagent, and the immunogen reagent was then injected subcutaneously in the inguinal region or injected intraperitoneally. In each round of immunization, each mouse received a total injection dose of 100 µL. In the first round of immunization, each mouse was immunized with an immunogen reagent prepared by mixing 50 µg of the antigen protein with a complete Freund's adjuvant (Sigma, #F5881) at a volume ratio of 1 : 1. In each subsequent round of booster immunization, each mouse was immunized with an immunogen reagent prepared by mixing 25 µg of the antigen protein with Sigma Adjuvant System adjuvant (Sigma, #S6322). The interval between two rounds of booster immunization was at least two weeks, typically with no more than 5 rounds of booster immunization. The immunization times were days 0, 14, 28, 42, 56, and 70; and on days 49 and 77, the antibody titers in the sera of mice were measured. Three days before the cell fusion, the final booster immunization was performed at a dose of 25 µg of the antigen protein per mouse.

### Hybridoma screening

When the detected titer of the CD40-specific antibody in the mouse serum reached a certain level, the mouse spleen cells were taken out and fused with the myeloma cell line to obtain hybridoma cells. After multiple rounds of screening and cloning of hybridoma cells, the hybridoma expressing the CD40 monoclonal antibody molecule was isolated. The isolated hybridoma expressed the antibody molecule having heavy and light chains of complete human variable domains and rat constant domains. The above-mentioned monoclonal antibody was further identified, and several hybridoma clones were selected for sequencing based on parameters such as the binding ability to human CD40, the binding ability to cynomolgus monkey CD40, and the ability to activate CD40 downstream signaling pathway. During the screening process, Selicrelumab (also numbered PR001028 in the present application) was used as a positive control antibody. The nucleotide sequence encoding the variable domain of the antibody molecule and the corresponding amino acid sequence was obtained by conventional hybridoma sequencing means. In this example, the sequences of the variable domains of the CD40 monoclonal antibody molecule obtained from the immunized Harbour H2L2 mice were human antibody sequences. The CDR sequences of the antibody variable domains could be analyzed according to Kabat or Chothia or other CDR definition rules (Table-I).

### Recombinant IgG antibodies and sequence analysis and optimization

After obtaining the light and heavy chain variable domain sequences of the encoding antibody molecule, the light and heavy chain variable domain sequences can be expressed in fusion with the light and heavy chain constant domain sequences of corresponding human antibody using conventional recombinant DNA techniques to obtain recombinant antibody molecules using the method described in 0. In this example, an antibody heavy chain variable domain (VH) sequence was obtained by gene synthesis and cloned into a mammalian cell expression plasmid vector encoding heavy chain constant domain sequences of **human IgG1 or IgG2 antibody** to encode and produce a full-length heavy chain of an IgG antibody. A light chain variable domain (VL) sequence of an antibody was obtained by gene synthesis and cloned into a mammalian cell expression plasmid vector encoding a κ light chain constant domain sequence of human antibody Ig to encode and produce a full-length light chain of an antibody. In this example, a fully human anti-CD40 recombinant IgG antibody was obtained.

The heavy chain variable domain sequence of the antibody is derived from events such as gene rearrangement of germline gene V, D and J gene fragments of the heavy chain gene group on chromosomes and somatic hypermutation; and the light chain variable domain sequence is derived from events such as gene rearrangement of germline gene V and J gene fragments of the light chain gene group and somatic hypermutation. Gene rearrangement and somatic hypermutation are main factors that increase antibody diversity. Antibodies derived from the same germline V gene fragment may also give rise to different sequences, but the overall similarity is high. The possible germline gene fragments in the event of gene rearrangement can be deduced from the variable domain sequence of the antibody using some algorithms, such as IMGT/DomainGapAlign (http://imgt.org/3Dstructure-DB/cgi/DomainGapAlign.cgi) or NCBI/IgBLAST (https://www.ncbi.nlm.nih.gov/igblast/).

After the translation synthesis of amino acid chains of proteins or polypeptides in cells, chemical modifications are sometimes introduced, called post-translational modifications (PTMs). For antibodies, some of the PTM sites are very conserved. For example, the conserved amino acid asparagine Asn at position 297 (EU numbering) of the constant domain of human IgG1 antibody is usually glycosylated to form a saccharide chain, and this saccharide chain structure is critical for the antibody structure and related effector functions. However, if a PTM is present in a variable domain, particularly an antigen-binding region (e.g., CDR) of an antibody, the presence of such a PTM can have a greater impact on the binding to an antigen, and can also cause a change in the physicochemical properties of the antibody. For example, glycosylation, deamidation, isomerization, oxidation, etc. may increase the instability or heterogeneity of antibody molecules, thereby increasing the difficulty and risk of antibody development. Avoiding some potential PTMs is thus very important for the development of therapeutic antibodies. With the accumulation of experience, it has been found that some PTMs are highly correlated with the "pattern" of the composition of amino acid sequences, particularly of the composition of adjacent amino acids, which makes it possible to predict potential PTMs from the primary amino acid sequences of proteins. For example, an N-linked glycosylation site can be predicted according the sequence pattern N-x-S/T (asparagine at the first position, any amino acid other than proline at the second position, and serine or threonine at the third position). Amino acid sequence patterns that cause PTMs may be derived from germline gene sequences, for example, pattern NST where human germline gene fragment IGHV3-33 naturally has a glycosylation modification in the FR3 region; and they may also be derived from somatic hypermutation. Specifically, NGS or NLT may be a glycosylation site, NS may be a deamidation site, and DG may cause isomerization of aspartic acid. The amino acid sequence patterns of PTMs can be disrupted by amino acid mutations, thereby reducing or removing the formation of particular PTMs. Depending on different antibody sequences and different PTM sequence patterns, there are different mutation design methods. One method is to replace the "hot spot" amino acid (such as N or S in the NS pattern) with an amino acid having similar physicochemical properties (e.g., mutation of N to Q). Another method uses a mutation library approach to randomly mutate the PTM sequence pattern. In actual operation, multiple mutation design methods may be used for the same PTM sequence pattern.

Table 3 listed the anti-CD40 hybridoma clone and the recombinant antibody molecule (PR003379) obtained in this example, as well as the analyzed germline gene V gene segments and potential PTM sites of VH and VL.

Table 4 listed the new antibody molecules obtained by amino acid mutation of CD40 antibody PR003379. Purified recombinant antibodies were obtained from all designed mutant molecules according to the method described in 0, and further verified in subsequent functional experiments. The method for obtaining the antibodies in Table 5 comprises: the heavy chain variable region (VH) of PR003379 was linked to the heavy chain constant domain sequence of an IgG1 subtype antibody containing L234A, L235A and G237A mutations and cloned into a mammalian cell expression plasmid to encode and produce a full-length heavy chain of IgG1 (L234A, L235A and G237A); a light chain variable domain (VL) sequence of an antibody with the PTM mutation site was obtained by gene synthesis and cloned into a mammalian cell expression plasmid vector encoding a κ light chain constant domain sequence of human antibody Ig to encode and produce a full-length light chain of an antibody; and the two plasmids were co-transfected into mammalian cells, and after expression, production and purification, a fully human anti-CD40 recombinant IgG1 (L234A, L235A and G237A) antibody was obtained.

Table 5 listed the sequences of the CD40 antibodies of the present invention and the amino acid sequences of the CDRs defined according to the rules of Chothia definition. These CD40 antibodies include a positive control antibody, the CD40 antibody PR003379 of the present invention, and mutant molecules thereof.

**Table 3. Germline gene analysis and PTM site analysis of CD40 antibodies**

| **Clone No.** | **VH germline V gene** | **VL germline V gene** | **VH PTM** | **VL PTM** | **Recombinant antibody** | **Recombinant antibody subtype** |
|---|---|---|---|---|---|---|
| 18C4A12-1B1 | IGHV1-2 | IGKV1-9 | | NS (LCDR3) | **PR003379** | Human IgG2 |

**Table 4. Mutation site design of CD40 antibody PR003379**

| **Variant number** | **Mutation in variable region** | **Recombinant antibody subtype** | **Mutation in Fc** |
|---|---|---|---|
| PR006495 | L:N92A | Human IgG1 | L234A, L235A, G237A |
| PR006496 | L:N92F | Human IgG1 | L234A, L235A, G237A |
| PR006497 | L:N92Y | Human IgG1 | L234A, L235A, G237A |
| PR006504 | L:N92V | Human IgG1 | L234A, L235A, G237A |
| PR006511 | L:S93N | Human IgG1 | L234A, L235A, G237A |

**Table 5. Sequence numbering table of the sequences and CDR sequences (Chothia) for the CD40 antibodies of the present invention**

| **Molecular number** | **Light chain** | **Heavy chain** | **VL** | **VH** | **LCDR 1** | **LCDR 2** | **LCDR 3** | **HCDR 1** | **HCDR 2** | **HCDR 3** |
|---|---|---|---|---|---|---|---|---|---|---|
| PR001028 (Selicrelu mab) | 86 | 76 | 67 | 58 | 37 | 42 | 47 | 6 | 17 | 26 |
| PR003379 | 87 | 78 | 68 | 60 | 38 | 43 | 48 | 8 | 18 | 27 |
| PR006495 | 88 | 84 | 69 | 60 | 38 | 43 | 49 | 8 | 18 | 27 |
| PR006496 | 89 | 84 | 70 | 60 | 38 | 43 | 50 | 8 | 18 | 27 |
| PR006497 | 90 | 84 | 71 | 60 | 38 | 43 | 51 | 8 | 18 | 27 |
| PR006504 | 91 | 84 | 72 | 60 | 38 | 43 | 52 | 8 | 18 | 27 |
| PR006511 | 92 | 84 | 73 | 60 | 38 | 43 | 53 | 8 | 18 | 27 |

### Example 3. Binding of CD40 antigen-binding proteins to cells expressing CD40

In this example, flow cytometry (FACS) was used to study the binding activity of the antibody or antigen-binding protein targeting CD40 to cells expressing CD40. For example, the CHO-K1 cell line highly expressing human CD40, CHO-K1/hCD40 (Shanghai ChemPartner), the CHO-K1 cell line highly expressing cynomolgus monkey CD40, CHO-K1/cyCD40 (Shanghai ChemPartner), and the lymphoma cell highly expressing human CD40, Raji (ATCC, #CCL-86) were used.

Specifically, the cells expressing CD40 were digested and resuspended with complete medium (F-12K for CHO-K1 cells; and RPMI-1640 for Raji cells); and the cells were adjusted to a cell density of 1 × 10⁶ cells/mL. The cells were seeded in a 96-well V-bottom plate (Corning, #3894) at 100 µL/well, and the antibody to be tested at 2× final concentration was then added at 100 µL/well and mixed evenly, with the highest final concentration of the antibody of 300 nM, a total of 8 concentrations, and 5-fold concentration gradient dilution. hIgG (Crownbio, #C0002) was used as a negative control and Selicrelumab was used as a positive control molecule, and the concentration settings were the same as the subsequent bispecific antibody to be tested. The cells were incubated at 4°C for 1 hours in the dark. After the incubation, the cells were rinsed twice by adding pre-chilled PBS at 100 µL/well, and centrifuged at 500 g at 4°C for 5 minutes, and the supernatant was discarded. Then, a fluorescent secondary antibody (goat anti-human IgG (H+L) secondary antibody, Alexa Fluor^{®} 488 conjugate, Invitrogen, #A11013, 1 : 1000 dilution) was added at 100 µL/well, and the cells were incubated at 4°C for 30 minutes in the dark. The cells were washed twice with pre-chilled PBS at 200 µL/well, and centrifuged at 500 g at 4°C for 5 minutes, and the supernatant was discarded. Finally, the cells were resuspended in pre-chilled PBS at 200 µL/well. The fluorescence signal values were read using a BD FACS CANTOII flow cytometer or ACEA NovoCyte flow cytometer, and the data were processed and analyzed using FlowJo v10 software (FlowJo, LLC).

Data processing and graphical analysis were performed using GraphPad Prism 8 software. The binding curve of the antibody to the target cell and parameters, such as EC₅₀ value were obtained by four-parameter nonlinear fitting.

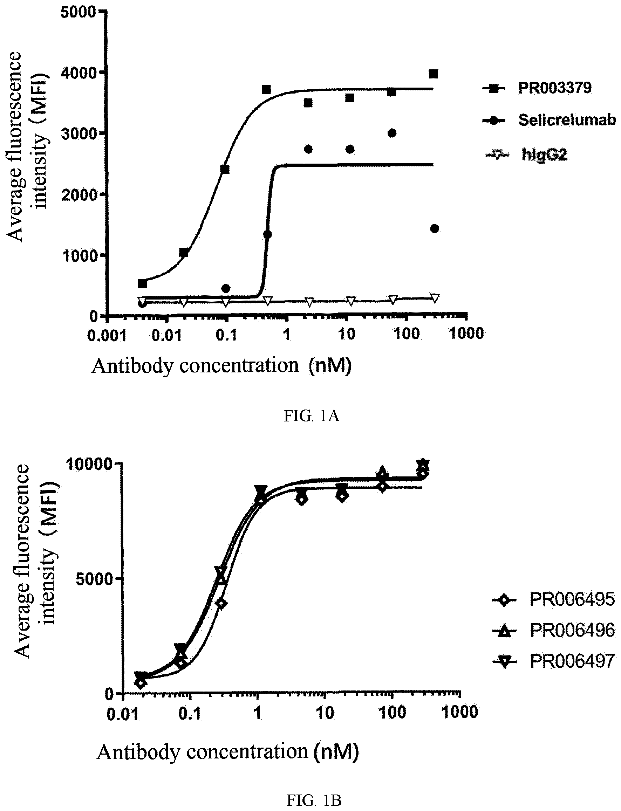

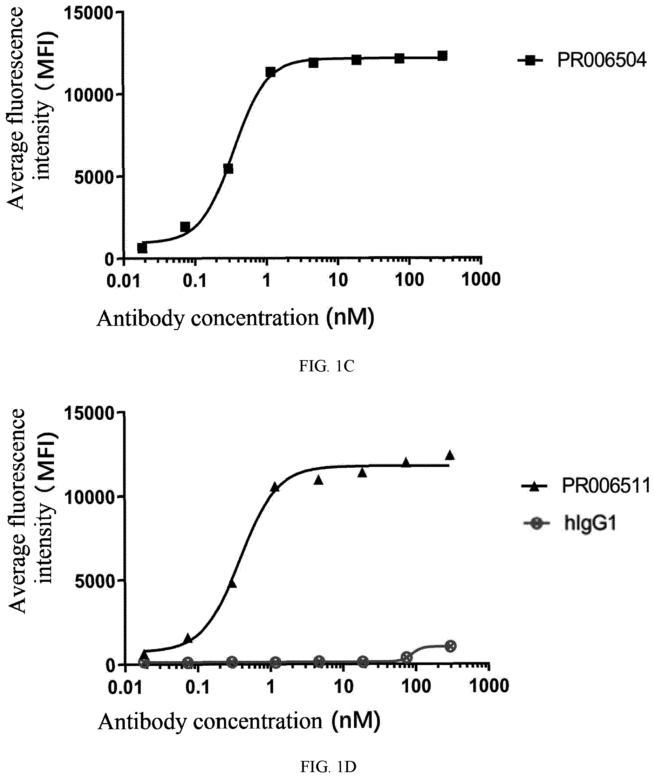

FIG. 1A-FIG. 1D and Table 6 showed the activity of CD40 antibody PR003379 and the variant molecules thereof in binding to Raji cells. The results showed that PR003379 and the variant molecules thereof could strongly bind to Raji cells; and the binding activity of PR003379 was stronger than that of Selicrelumab.

**Table 6. Binding of CD40 antibody to Raji cells (corresponding to FIG. 1A-FIG. 1D)**

| | **Antibody** | **EC₅₀ (nM)** | **Maximum MFI** | | **Antibody** | **EC₅₀ (nM)** | **Maximum MFI** |
|---|---|---|---|---|---|---|---|
| (A) | PR003379 | 0.0699 | 3696 | (B) | PR006495 | 0.3556 | 8872 |
| | Selicrelumab | 0.4837 | 2449 | | PR006496 | 0.2771 | 9297 |
| | | | | | PR006497 | 0.2524 | 9203 |
| | **Antibody** | **EC₅₀ (nM)** | **Maximum MFI** | | **Antibody** | **EC₅₀ (nM)** | **Maximum MFI** |
| (C) | PR006504 | 0.3503 | 12152 | (D) | PR006511 | 0.3859 | 11785 |

### Example 4. Activity of CD40 antigen-binding proteins in fluorescent reporter gene experiments

In this example, activation experiments of HEK293-hCD40-NFκB fluorescent reporter gene cells (BPS Bioscience, #60626) were used to study the activation effect of antibodies or antigen-binding proteins targeting CD40 in the presence of or in the absence of crosslinking on CD40. Cells highly expressing human CD32B were used to perform Fc-mediated crosslinking of the molecules to be tested, to study whether crosslinking could further enhance the ability to activate CD40.

CHO-K1 cells expressing human CD32B (CHO-K1/hCD32B, GenScript, #M00600) and CHO-K1 cells were plated at 1 × 10⁴ cells/well or 100 µL/well in a 96-well plate (PerkinElmer, #6005181) and incubated at 37°C in 5% CO₂ environment overnight. The supernatant was removed, and HEK293-hCD40-NFκB reporter gene cells expressing human CD40 and NFκB-luciferase reporter gene (BPS bioscience, #60626) were collected and added to a 96-well plate at 5×10⁴/well or 50 µL/well. 50 µL/well of the dilution solution of the antibody to be tested was added with an initial concentration of 100 nM, 5-fold concentration dilution and a total of 8 concentrations. Culture was performed at 37°C in 5% CO₂ environment for 6 hours. ONE-Glo^{™} luciferase reagent (Promega, #E6110) was added, incubation was performed at room temperature for 5 minutes, and the luminescence value was detected by a microplate reader. Data processing and graphical analysis were performed using GraphPad Prism 8 software. The curve of the antibody concentration-dependent relative fluorescence signal unit (RLU) and parameters, such as EC₅₀ value were obtained by four-parameter nonlinear fitting. The sample in which the CD40 antibody was incubated with CHO-K1/hCD32B cells was referred to as "crosslinked"; and the sample in which the CD40 antibody was incubated with CHO-K1 cells was referred to as "un-crosslinked".

As shown in FIG. 2A-FIG. 2B and Table 7, PR003379 had a more obvious "crosslinking enhancement" effect than the control antibody Selicrelumab, that is, PR003379 in the presence of CD32B-mediated antibody crosslinking could significantly amplify the signal of the fluorescent reporter gene, indicating that the activation effect of the CD40 molecule on the downstream signal molecule was significantly enhanced.

FIG. 2C-FIG. 2D and Table 8 showed the enhancement fold of fluorescence intensity of the activation of reporter gene cells by the variant molecules of PR003379 in the presence of or in the absence of crosslinking mediated by cells expressing human CD32B. The results showed that the variant molecules of PR003379 in the presence of crosslinking could significantly enhance the activation on CD40 molecule, which was manifested in that the EC₅₀ value in the presence of crosslinking was significantly reduced compared with the EC₅₀ value in the absence of crosslinking (expressed as "fold (EC₅₀ in the absence of crosslinking/EC₅₀ in the presence of crosslinking)" > 1).

**Table 7. Activity of CD40 antibody for activating the HEK293-hCD40-NFκB reporter gene cell**

| **Antibody** | **EC₅₀ (nM)** | **Maximum RLU** | **Fold (EC₅₀ in the absence of crosslinking/EC₅₀ in the presence of crosslinking))** |
|---|---|---|---|
| PR003379 | 0.3693 | 8252 | 2.07 |
| PR003379 crosslinked | 0.1784 | 19664 | |
| Selicrelumab | 0.1628 | 13355 | 3.94 |
| Selicrelumab crosslinked | 0.0413 | 17036 | |

**Table 8. Activity of PR003379 variants in the presence of or in the absence of crosslinking for activating the reporter gene cell**

| | **Crosslinked** | | **Un-crosslinked** | | |
|---|---|---|---|---|---|
| **PR003379 variant** | **EC₅₀ (nM)** | **Maximum fold (fold)** | **EC₅₀ (nM)** | **Maximum fold (fold)** | **Fold (EC₅₀ in the absence of crosslinking/EC₅₀ in the presence of crosslinking))** |
| PR006495 | 0.1092 | 5.54 | 0.9047 | 4.92 | 8.28 |
| PR006496 | 0.2172 | 5.03 | 0.4296 | 4.43 | 1.98 |
| PR006497 | 0.1582 | 4.77 | 0.4786 | 4.61 | 3.03 |

### Example 5. Preparation of PD-L1 heavy chain antibody

Harbour HCAb mouse (Harbour Antibodies BV, WO 2010/109165 A2) is a transgenic mouse carrying a human immunoglobulin immune repertoire, and can produce a heavy-chain-only antibody (HCAb) which is only half the size of conventional IgG antibody. The antibody produced thereby has only heavy chain variable domain of human antibody and Fc constant domain of mouse. Due to the characteristic of not containing light chains, the antibody almost solves the problems of light chain mispairing and heterodimerization, so that this technique platform can develop products that are difficult to achieve with conventional antibody platforms.

### Immunization of mice

6-8 week-old Harbour HCAb mice were immunized in multiple rounds with a soluble recombinant human PD-L1-mFc fusion protein (Novo Protein Inc., #CM06). In each immunization, each mouse received a total injection dose of 100 µL by subcutaneous injection in the inguinal region or by intraperitoneal injection. In the first round of immunization, each mouse was immunized with an immunogen reagent prepared by mixing 50 µg of the antigen protein with a complete Freund's adjuvant (Sigma, #F5881) at a volume ratio of 1 : 1. In each subsequent round of booster immunization, each mouse was immunized with an immunogen reagent prepared by mixing 25 µg of the antigen protein with Ribi adjuvant (Sigma Adjuvant System, #S6322). The interval between two rounds of booster immunization was at least two weeks, typically with no more than 5 rounds of booster immunization. The immunization times were days 0, 14, 28, 42, 56, and 70; and on days 49 and 77, the antibody titers in the sera of mice were measured. Five days before the isolation of splenic B cells in HCAb mice, the final booster immunization was performed at a dose of 25 µg of the antigen protein per mouse.

### Construction of HCAb library and high-throughput transient expression

When the titer of the PD-L1-specific antibody in the mouse serum reached a certain level, the mouse spleen cells were removed to isolate B cells, and CD138-positive plasma cells and human PD-1 antigen-positive B cell populations were sorted using BD FACS AriaII Cell Sorter. The human VH gene was amplified from plasma cells and B cell populations by conventional molecular biological means, and the amplified human VH gene fragments were constructed into a mammalian cell expression plasmid pCAG vector encoding the Fc region sequence of the human IgG1 antibody heavy chain. The plasmid was transfected into mammalian host cells (such as human embryonic kidney cell HEK293) for expression to obtain the supernatant containing the fully human HCAb antibody.

### Mirrorball high-throughput screening

The supernatant containing expressed HCAb was screened using Mirrorball high-throughput no-wash antibody screening platform (SPT Labtech), that is, the binding of the supernatant to the CHO-K1/hPD-L1 (GenScript, #M00543) stable cell line overexpressing human PD-L1 was detected, and the positive antibody PR000151 (Atezolizumab analog) was used as a positive control.

Specifically, CHO-K1/hPD-L1 cells were washed with serum-free F12K medium (Thermofisher, #21127022) and resuspended to 1 × 10⁶/mL. Draq5 fluorescent probe (CTS, #4048L) was added (1 µL of Draq5 being added to 1 mL of CHO-K1/hPD-L1 cells, 1 : 1000 dilution), and the cells were incubated for 30 minutes in the dark. After the cells were centrifuged, the cells were washed with culture medium and adjusted to a cell density of 1.0 × 10⁵ cells/mL. Then, the secondary antibody (Alexa Fluor^{®} 488 AffiniPure Goat Anti-Human IgG,Fcγ Fragment Specific, Jackson ImmunoResearch, #109-545-098) at 1 : 1000 dilution was added. The mixture was taken and added to a 384-well plate (Greiner, #781091) at 30 µL/well. Then, 10 µL of positive control or the supernatant containing expressed HCAb was added to the 384-well plate, and incubation was performed for 2 hours. Fluorescence values were read on Mirrorball.

The positive antibody supernatant was further identified. The binding to CHO-K1/hPD-L1 cell was detected by FACS and the cross-binding activity to cynomolgus monkey PD-L1 protein (Acro biosystems, #PD1-C52H4) was detected by ELISA. The screened HCAb was sequenced by conventional sequencing means to obtain the nucleotide sequence encoding corresponding VH and the corresponding amino acid sequence. The VH sequence of the HCAb antibody and the Fc sequence of the human IgG1 heavy chain were expressed in fusion to obtain the fully human recombinant HCAb antibody molecule.

### Sequence analysis and optimization

Table 9 listed the preferred anti-PD-L1 recombinant heavy chain antibody molecule PR000960 obtained in this example, as well as the analyzed VH germline gene V gene segment and potential post-translational modification (PTM) site. PR000960 has an N-glycosylation modification site in HCDR1, which may affect the drugability in later development. In order to remove the N-glycosylation modification site, PR000960 was subjected to amino acid mutation to obtain a new antibody molecule PR002082 (Table 10).

### Improve the binding affinity to PD-L1 by affinity maturation technique

In order to further improve the binding affinity of PR002082 to PD-L1, the PR002082 molecule was subjected to in vitro affinity maturation engineering. A yeast display antibody mutation library was established by random mutation of the CDR region, and the mutation library of PR002082 molecular was screened by BD FACS AriaIII sorting platform.

First, mutations were randomly introduced into the three CDRs of PR002082 to establish three yeast display mutation libraries (CDR1, CDR2 and CDR3); enrichment was performed using the MACS sorting method; and then multiple rounds of sorting were performed using flow cytometry FACS to enrich high-affinity mutant molecules. In the first round, yeast cells with higher binding affinity were sorted using 0.1 nM biotinylated PD-L1-his protein; and then yeast cells were further cultured for induction. In the second round, yeast cells with higher binding affinity were further sorted using 0.01 nM biotinylated PD-L1-his protein; and then yeast cells were further cultured for induction. In the third round, the antigen concentration during sorting was further reduced, and the final round of sorting was performed using 0.003 nM biotinylated PD-L1-his protein.

Then the molecules sorted in the previous three rounds were sequenced to find the mutation sites which were randomly combined, thereby establishing a combinatorial mutation library. Subsequently, mutant molecules with higher affinity were further sorted using FACS at lower antigen concentrations.

The VHs of the mutant molecules obtained by affinity maturation were subjected to gene synthesis and cloned into a mammalian cell expression plasmid vector encoding the human IgG1 heavy chain Fc sequence to produce recombinant HCAb antibody molecules. Table 11 listed the variant molecules of PR002082 obtained by affinity maturation process.

Purified recombinant antibodies were obtained from all mutant molecules (Table 10 and Table 11) according to the method described in 0, and further verified in subsequent functional experiments.

Table 12 listed the sequences of the PD-L1 antibodies of the present invention and the amino acid sequences of the CDRs defined according to the rules of Chothia definition. These PD-L1 antibodies include a positive control antibody, the PD-L1 antibody PR000960 of the present invention, and derived mutant molecules thereof.

**Table 9. Germline gene analysis and PTM site analysis of PD-L1 heavy chain antibodies**

| **Recombinant antibody** | **Molecular structure** | **IgG subtype** | **VH germline V gene** | **VH PTM** |
|---|---|---|---|---|
| PR000960 | HCAb | Human IgG1 | IGHV3-33 | NxS/T (HCDR1); DG (HCDR2) |

**Table 10. Post-translational modification (PTM) variant of PD-L1 heavy chain antibody PR000960**

| **Initial antibody** | **Variant molecule** | **Mutation in variable region** | **VH PTM** |
|---|---|---|---|
| PR000960 | PR002082 | N28T, N31S | DG (HCDR2) |

**Table 11. Variant molecules of PR002082 obtained by affinity maturation process**

| **Initial antibody** | **Variant molecule** | **Mutation in variable region** |
|---|---|---|
| PR002082 | PR005878 | I50S, W52R, D54T, K57E, I102L, V106I, A109D |
| PR002082 | PR006245 | T28D, I50S, W52R, D54T, K57E, |
| PR002082 | PR006246 | T28D, I50S, W52R, D54T, K57E, V106I, A109D |
| PR002082 | PR006247 | T28D, I50S, W52R, D54T, K57E, I102L, V106I |
| PR002082 | PR006248 | T28D, I50S, W52R, D54T, K57E, I102L, V106I, A109D |

**Table 12. Sequence numbering table of the sequences and CDR sequences (Chothia) for the PD-L1 antibodies of the present invention**

| **Molecular number** | **Light chain** | **Heavy chain** | **VL** | **VH** | **LCDR1** | **LCDR2** | **LCDR3** | **HCDR1** | **HCDR2** | **HCDR3** |
|---|---|---|---|---|---|---|---|---|---|---|
| PR000151 (Atezolizum ab) | 85 | 74 | 66 | 56 | 36 | 41 | 46 | 4 | 15 | 24 |
| PR000960 | | 75 | | 57 | | | | 5 | 16 | 25 |
| PR002082 | | 77 | | 59 | | | | 7 | 16 | 25 |
| PR005878 | | 79 | | 61 | | | | 7 | 19 | 28 |
| PR006245 | | 80 | | 62 | | | | 9 | 19 | 25 |
| PR006246 | | 81 | | 63 | | | | 9 | 19 | 29 |
| PR006247 | | 82 | | 64 | | | | 9 | 19 | 30 |
| PR006248 | | 83 | | 65 | | | | 9 | 19 | 28 |

### Example 6. Binding of PD-L1 antigen-binding proteins to cells expressing PD-L1

In this example, flow cytometry (FACS) was used to study the binding activity of the antibody or antigen-binding protein targeting PD-L1 to cells expressing PD-L1. For example, the CHO-K1 cell line highly expressing human PD-L1, CHO-K1/hPD-L1 (Shanghai ChemPartner), the CHO-K1 cell line highly expressing cynomolgus monkey PD-L1, CHO-K1/cyPD-L1 (Shanghai ChemPartner), the lung cancer cell highly expressing human PD-L1, NCI-H226 (ATCC, #CRL-5826), and the breast cancer cell highly expressing human PD-L1, MDA-MB-231 (ATCC, #CRM-HTB-26) were used.

Specifically, the cells expressing PD-L1 were digested and resuspended in PBS buffer containing 2% BSA. The cells were adjusted to a cell density of 1×10⁶ cells/mL, respectively. The cells were seeded in a 96-well V-bottom plate (Corning, #3894) at 100 µL/well, and the antibody to be tested at 2×final concentration with 4-fold concentration gradient dilution was then added at 100 µL/well. The cells were incubated at 4°C for 2 hours in the dark. After the incubation, the cells were rinsed twice by adding pre-chilled PBS containing 2% BSA at 100 µL/well, and centrifuged at 500 g at 4°C for 5 minutes, and the supernatant was discarded. A fluorescent secondary antibody (Alexa Fluor 488-conjugated AffiniPure Goat Anti-Human IgG, Fcγ Fragment Specific, Jackson, #109-545-098, 1 : 500 dilution) was then added at 100 µL/well, and the cells were incubated at 4°C for 1 hour in the dark. The cells were washed twice by adding pre-chilled PBS containing 2% BSA at 100 µL/well, and centrifuged at 500 g at 4°C for 5 minutes, and the supernatant was discarded. Finally, the cells were resuspended in pre-chilled PBS buffer containing 2% BSA at 200 µL/well. The fluorescence signal values were read using a BD FACS CANTOII flow cytometer or ACEA NovoCyte flow cytometer, and the data were processed and analyzed using FlowJo v10 software (FlowJo, LLC).

Data processing and graphical analysis were performed using GraphPad Prism 8 software. The binding curve of the antibody to the target cell and parameters, such as EC₅₀ value were obtained by four-parameter nonlinear fitting.

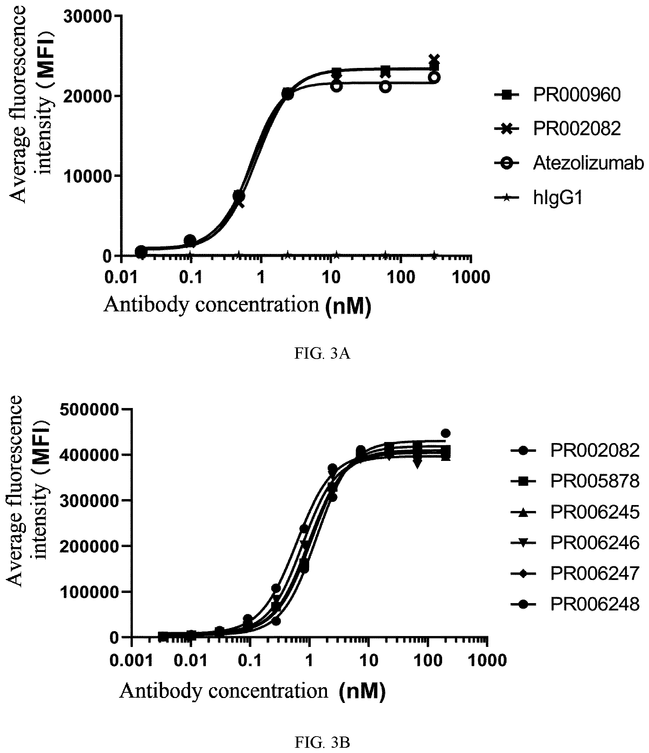

FIG. 3A and FIG. 3B showed the binding curves of PD-L1 heavy chain antibodies PR000960 and PR002082, and the variant molecules thereof to CHO-K1/hPD-L1 cells, and Table 13 listed EC₅₀ values and the maximum MFI signal of the corresponding curves. The results indicated that the binding ability of PR000960 and PR002082 to human PD-L1 was comparable to that of the control antibody Atezolizumab; and the binding abilities of affinity matured variant molecules of PR002082 were slightly better than that of PR002082.

**Table 13. Binding of PD-L1 antibody to CHO-K1/hPD-L1 cells (corresponding to FIG. 3A-FIG. 3B)**

| | **Antibody** | **EC₅₀ (nM)** | **Maximum MFI** | | **Antibody** | **EC₅₀ (nM)** | **Maximu m MFI** |
|---|---|---|---|---|---|---|---|
| (A) | PR000960 | 0.79 | 23730 | (B) | PR002082 | 1.313 | 430130 |
| | PR002082 | 0.86 | 24529 | | PR005878 | 1.081 | 418658 |
| | Atezolizumab | 0.7 | 22303 | | PR006245 | 0.964 | 404629 |
| | hIgG1 | | 86 | | PR006246 | 0.764 | 396497 |
| | | | | | PR006247 | 1.012 | 409736 |
| | | | | | PR006248 | 0.606 | 404274 |

### Example 7. Activity of PD-L1 antigen-binding proteins in fluorescent reporter gene experiments

In this example, activation experiments of Jurkat-NFAT-hPD1 fluorescent reporter gene cells (Harbour BioMed) were used to study inhibitory effects of antibodies or antigen-binding proteins targeting PD-L1 on the PD-1 signaling pathway. PD-L1 antibodies or antigen-binding proteins removed the inhibitory effect of PD-1 on T cells by blocking the interaction between PD-L1 and PD-1.

293T cell overexpressing PD-L1 and OS8 (CD3 single-chain antibody transmembrane protein), 293T-OS8-hPDL1 (Beijing Kyinno Biotechnology, #KC-1148) or Jurkat reporter cells co-expressing PD-1 and NFAT-luciferase reporter gene, Jurkat-NFAT-hPD1 (Harbour BioMed) were cultured and collected. The Jurkat-NFAT-hPD1 cells were adjusted to a cell density of 1 × 10⁶ cells/mL, and 293T-OS8-hPDL1 cells were adjusted to a cell density of 5 × 10⁵ cells/mL. Then, the same volume of cell suspensions was mixed and added to a 96-well U-bottom plate at 100 µL/well, where the ratio of effector cells Jurkat-NFAT-hPD1 to target cells 293T-OS8-hPDL1 was 2:1. Pre-gradiently diluted antigen-binding protein to be tested was added at 50 µL/well. The 96-well plate was placed in a cell incubator and incubated at 37°C and 5% CO₂ for six hours. ONE-Glo^{™} luciferase solution (Promega, #E6120) was added at 50 µL/well, the plate was gently slapped for uniformly mixing, and the mixture was reacted for ten minutes at room temperature in the dark. Then, 100 µL of the liquid was transferred into a 96-well flat-bottom plate (PerkinElmer, #6005225), and finally fluorescence values were read on Envision^{™} plate reader (PerkinElmer).

Data processing and graphical analysis were performed using GraphPad Prism 8 software. The binding curve of the antibody to the target cell and parameters, such as EC₅₀ value were obtained by four-parameter nonlinear fitting.

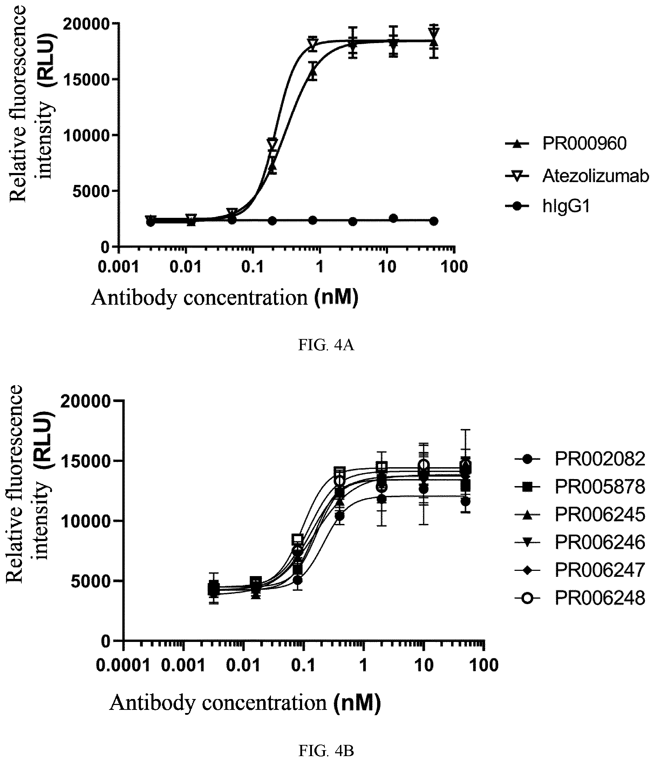

FIG. 4A and FIG. 4B and Table 14 showed that in the fluorescent reporter gene experiments, PD-L1 heavy chain antibodies PR000960 and PR002082 and variant molecules thereof inhibited the PD-1 signaling pathway, and therefore restored the NFAT signal in Jurkat. The results indicated that PR000960 and the control antibody Atezolizumab had comparable inhibitory activity; and the inhibitory effects of affinity matured variant molecules of PR002082 were slightly better than that of PR000960.

**Table 14. Inhibition of the PD-1 signaling pathway of Jurkat-NFAT-hPD1 by PD-L1 antibodies (corresponding to FIG. 4A and FIG. 4B)**

| | **Antibody** | **EC₅₀ (nM)** | **Maximum RLU** | | **Antibody** | **EC₅₀ (nM)** | **Maximum RLU** |
|---|---|---|---|---|---|---|---|
| (A) | PR000960 | 0.31 | 18445 | (B) | PR002082 | 0.217 | 12066 |
| | Atezolizumab | 0.22 | 18473 | | PR005878 | 0.166 | 13424 |
| | hIgG1 | | 2369 | | PR006245 | 0.151 | 13840 |
| | | | | | PR006246 | 0.132 | 13745 |
| | | | | | PR006247 | 0.15 | 13432 |
| | | | | | PR006248 | 0.121 | 14134 |

### Example 8. Construction of PD-L1×CD40 bispecific antigen-binding proteins

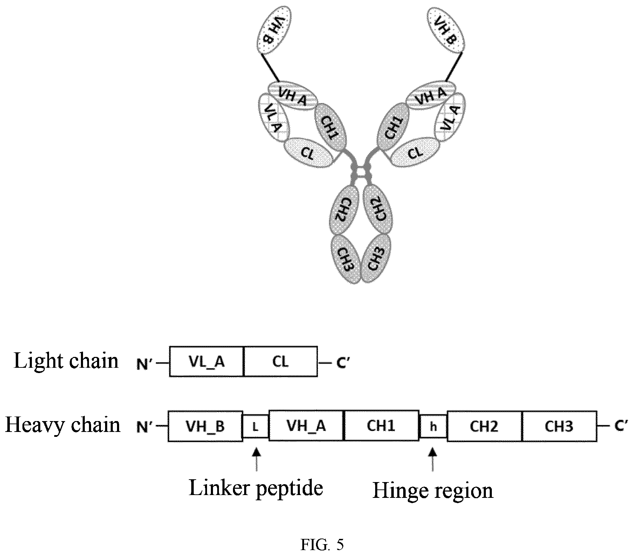

In this example, the CD40 antibody from 0 and the PD-L1 heavy chain antibody from 0 were used to construct a PD-L1×CD40 bispecific antigen-binding protein with the structure shown in FIG. 5.

As shown in FIG. 5, the Fab end is derived from conventional antibody A, and VH_A and VL_A are the heavy chain variable region and light chain variable region of antibody A, respectively. The VH end is derived from heavy chain antibody B, and VH_B is the heavy chain variable region of heavy chain antibody B. CL is the light chain constant region domain. CH1, CH2 and CH3 are the first, second and third domains of the heavy chain constant region, respectively. h is the hinge region of IgG antibody or a derived sequence, and L is a linker peptide. The binding protein having the shown structure comprises two different polypeptide chains: a light chain, from amino terminus to carboxyl terminus, comprising VL_A-CL; and a heavy chain, from amino terminus to carboxyl terminus, comprising VH _B-L-VH_A-CH1-h-CH2-CH3. In certain embodiments, VH_B of the heavy chain is directly fused to VH_A, i.e., the length of L is 0; and in other embodiments, VH_B of the heavy chain is linked to VH_A via a linker peptide L. L can be a sequence in Table 15.

**Table 15. Linker peptide sequence**

| **Linker peptide name** | **Length** | **Sequence** | **SEQ ID NO** |
|---|---|---|---|
| GS_2 | 2 | GS | |
| GS_3 | 3 | GGS | |
| GS_4 | 4 | GSGS | 100 |
| GS_5 | 5 | GGGGS | 101 |
| GS_7 | 7 | GGGGSGS | 102 |
| GS_15 | 15 | GGGGSGGGGSGGGGS | 103 |
| H1_15 | 15 | EPKSSDKTHTPPPPP | 104 |
| LH1 | 10 | DKTHTCPPCP | 105 |
| G5-LH | 15 | GGGGGDKTHTCPPCP | 106 |

In this example, VH_A and VL_A in FIG. 5 were derived from the antigen-binding fragment Fab of the H2L2 (IgG) antibodies PR006495, PR006496, PR006497, PR006504 and PR006511 that bind to CD40; and VH_B was derived from the antigen-binding fragment VH of the heavy chain antibodies PR005878, PR006245, PR006246, PR006247 and PR006248 that bind to PD-L1. Based on these PD-L1 and/or CD40 antigen-binding fragments, several PD-L1×CD40 bispecific antigen-binding proteins having the structure as shown in FIG. 5 (listed in Table 16) were constructed. These molecules all had similar structures and had the human IgG1 constant region sequence; and three amino acid mutations L234A/L235A/G237A (Eu encoding) were introduced into the Fc region to eliminate Fc-mediated effector function such as ADCC. Table 18 listed the sequence numbers of the polypeptide chain amino acid sequences of PD-L1×CD40. The corresponding protein samples of these PD-L1 ×CD40 molecules were prepared and analyzed according to the method described in 0, and the results were summarized in Table 17. The PD-L1×CD40 molecules constructed in this example had high yield and purity (see Example 1 for the expression and purity detection method of the PD-L1×CD40 molecules), and the purity of most protein samples exceeded 95%.

**Table 16. PD-L1×CD40 bispecific antigen-binding proteins of the present invention**

| **Bispecific antibody molecule** | **CD40 antibody** | **PD-L1 antibody from which VH was derived** | **Fc type (mutation)** | **Linker peptide** |
|---|---|---|---|---|
| PR006919 | PR006496 | PR006246 | Human IgG1 (L234A/L235A/G237A) | GS_5 |
| PR007070 | PR006495 | PR005878 | | GS_5 |
| PR007151 | PR006504 | PR005878 | | GS_5 |
| PR007268 | PR006497 | PR006246 | | GS_5 |
| PR007269 | PR006511 | PR006246 | | GS_5 |
| PR007273 | PR006495 | PR006245 | | GS_5 |
| PR007275 | PR006495 | PR006247 | | GS_5 |
| PR007276 | PR006495 | PR006248 | | GS_5 |
| PR007280 | PR006504 | PR006245 | | GS_5 |
| PR007281 | PR006504 | PR006246 | | GS_5 |
| PR007282 | PR006504 | PR006247 | | GS_5 |
| PR007556 | PR006496 | PR006248 | | GS_5 |
| PR007616 | PR006497 | PR006248 | | GS_5 |
| PR007617 | PR006495 | PR006246 | | GS_3 |
| PR007618 | PR006495 | PR006248 | | GS_3 |
| PR007619 | PR006504 | PR006246 | | GS_3 |
| PR007620 | PR006504 | PR006248 | | GS_3 |

**Table 17. Transient expression of PD-L1×CD40 bispecific antibody molecule**

| **Bispecific antibody molecule** | **Expressio n host cell** | **Expressio n volume (mL)** | **Sample number** | **Sample concentr ation (mg/mL)** | **SEC-HPLC concentratio n (%)** | **Yield (mg/L)** |
|---|---|---|---|---|---|---|
| PR006919 | CHO | 500 | 20210314A011 | 2.85 | 100 | 42.5 |
| PR007070 | CHO | 1000 | 20210406A001 | 6.36 | 98.60 | 46.5 |
| PR007151 | CHO | 1000 | 20210405A003 | 6.34 | 96.86 | 59.4 |
| PR007268 | HEK293 | 100 | 20210502A006 | 1.35 | 99.59 | 32.2 |
| PR007269 | HEK293 | 100 | 20210502A007 | 0.91 | 99.65 | 22.2 |
| PR007273 | HEK293 | 100 | 20210502A011 | 1.52 | 99.62 | 32.5 |
| PR007275 | HEK293 | 100 | 20210502A012 | 1.26 | 99.57 | 26.9 |
| PR007276 | HEK293 | 100 | 20210502A013 | 1.52 | 99.45 | 34.0 |
| PR007280 | HEK293 | 100 | 20210502A017 | 0.72 | 99.77 | 18.0 |
| PR007281 | HEK293 | 100 | 20210501A003 | 1.9 | 99.23 | 37.1 |
| PR007282 | HEK293 | 100 | 20210502A018 | 1.1 | 99.60 | 36.6 |
| PR007556 | CHO | 1000 | 20210619A006 | 10.25 | 92.33 | 251.2 |
| PR007616 | CHO | 1000 | 20210623A004 | 8.31 | 99.84 | 250.2 |
| PR007617 | CHO | 1000 | 20210627A001 | 5.31 | 97.09 | 163.6 |
| PR007618 | CHO | 1000 | 20210627A002 | 5.45 | 96.58 | 160.3 |
| PR007619 | CHO | 1000 | 20210627A003 | 4.64 | 95.24 | 136.4 |
| PR007620 | CHO | 1000 | 20210627A004 | 5.34 | 93.02 | 144.2 |

**Table 18. Sequence numbering table of polypeptide chains of the PD-L1×CD40 bispecific antibody molecules of the present invention**

| **Molecular number** | **Heavy chain** | **Light chain** |
|---|---|---|
| PR006919 | 93 | 89 |
| PR007070 | 94 | 88 |
| PR007151 | 94 | 91 |
| PR007268 | 93 | 90 |
| PR007269 | 93 | 92 |
| PR007273 | 95 | 88 |
| PR007275 | 96 | 88 |
| PR007276 | 97 | 88 |
| PR007280 | 95 | 91 |
| PR007281 | 93 | 91 |
| PR007282 | 96 | 91 |
| PR007556 | 97 | 89 |
| PR007616 | 97 | 90 |
| PR007617 | 98 | 88 |
| PR007618 | 99 | 88 |
| PR007619 | 98 | 91 |
| PR007620 | 99 | 91 |

### Example 9. Binding of PD-L1×CD40 to cells expressing PD-L1 or CD40

In this example, 0 and the method described in 0 were used to test the binding ability of the PD-L1×CD40 bispecific binding proteins to various cells expressing PD-L1 or CD40. These cells include: CHO-K1 cell that does not express PD-L1 or CD40, CHO-K1 cell line highly expressing human PD-L1, CHO-K1/hPD-L1 (Shanghai ChemPartner), CHO-K1 cell line highly expressing cynomolgus monkey PD-L1, CHO-K1/cyPD-L1 (Shanghai ChemPartner), CHO-K1 cell line highly expressing human CD40, CHO-K1/hCD40 (Shanghai ChemPartner), CHO-K1 cell line highly expressing cynomolgus monkey CD40, CHO-K1/cyCD40 (Shanghai ChemPartner), lymphoma cell highly expressing human CD40, Raji (ATCC, #CCL-86), lung cancer cell highly expressing human PD-L1, NCI-H226 (ATCC, #CRL-5826) and breast cancer cell highly expressing human PD-L1 and CD40, MDA-MB-231 (ATCC, #CRM-HTB-26).

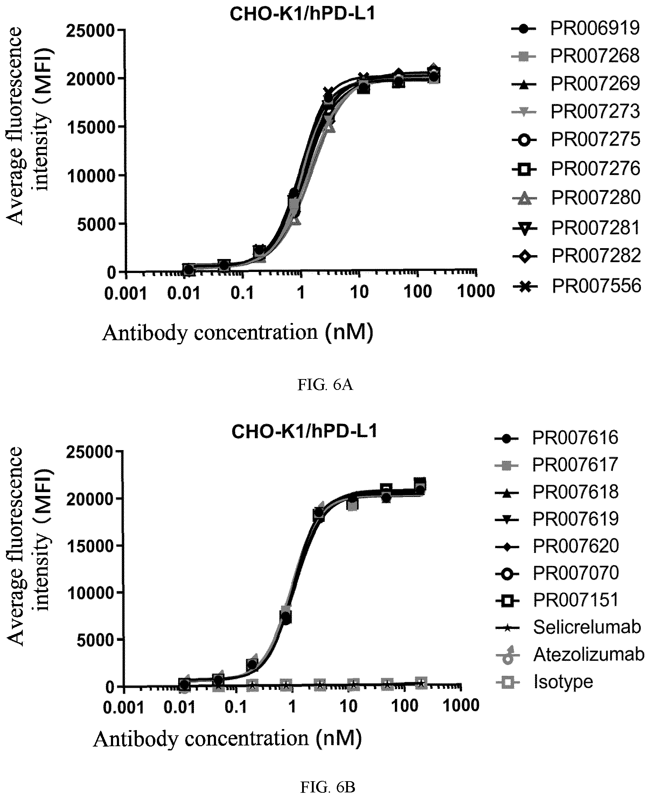

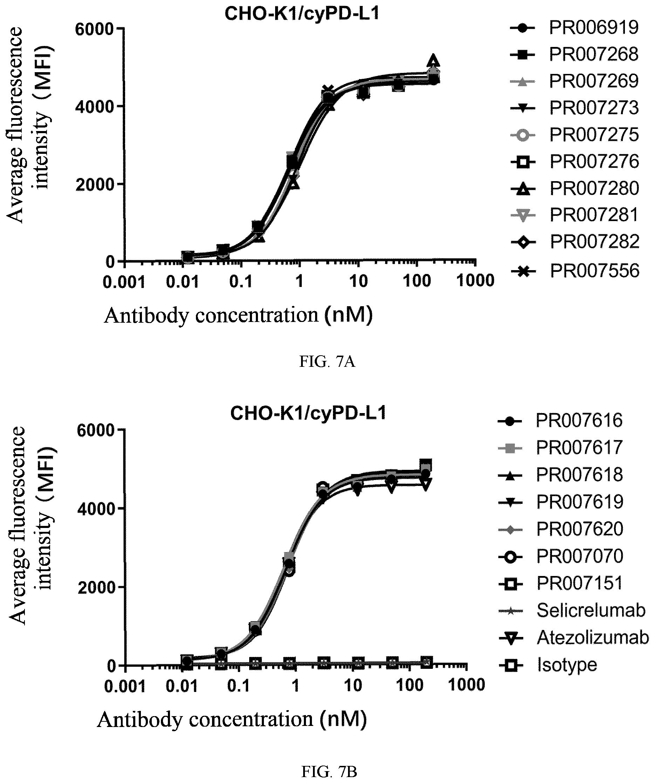

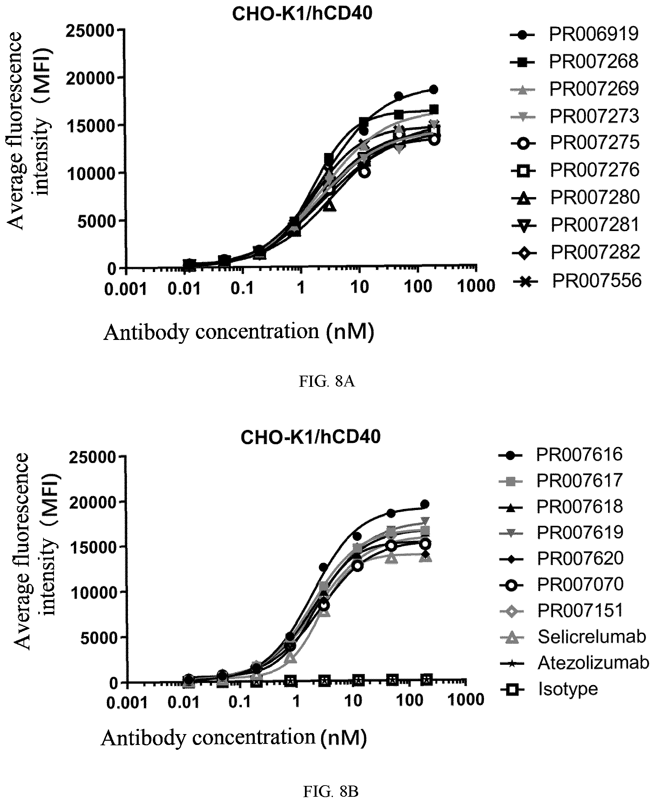

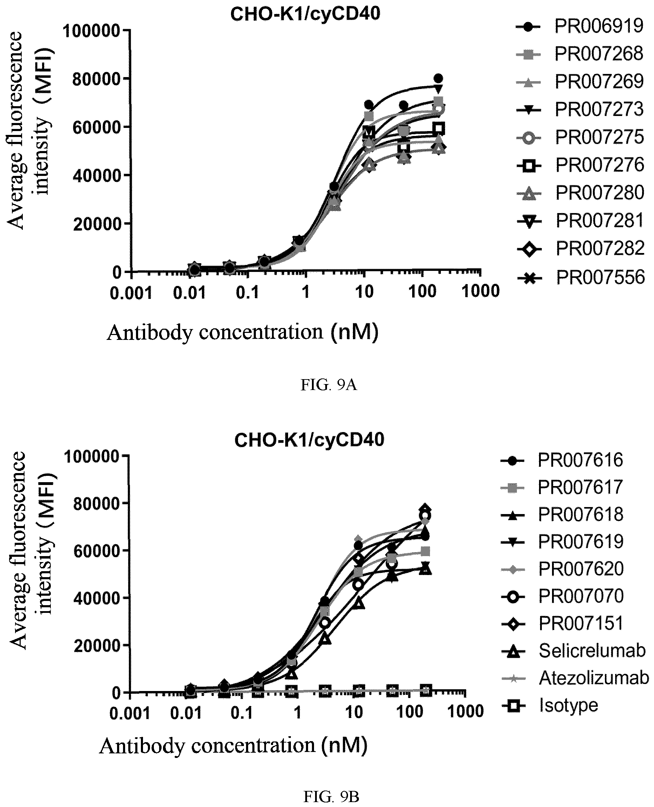

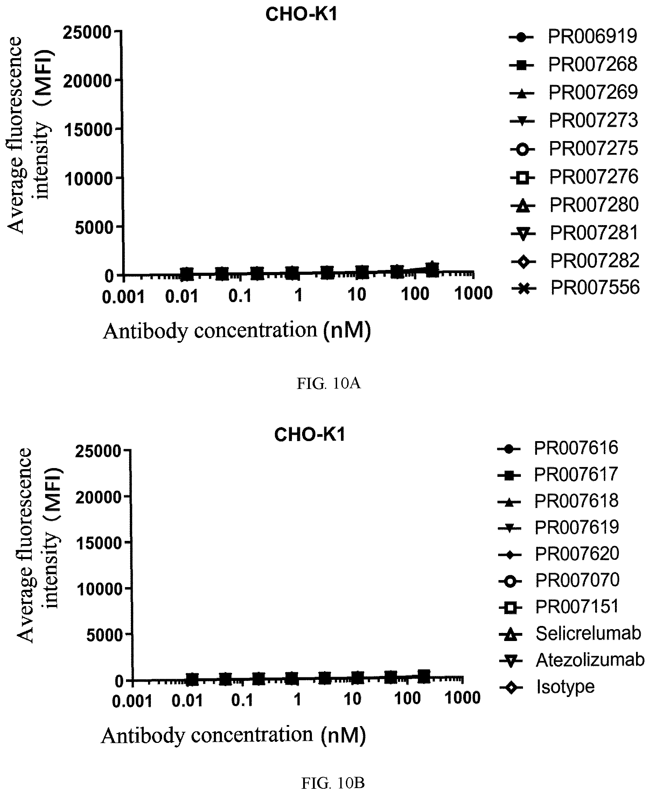

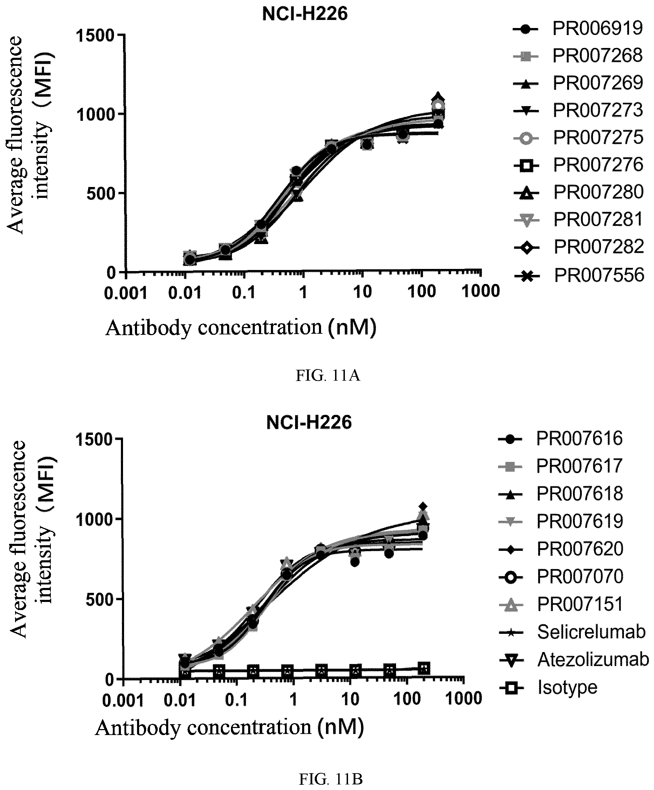

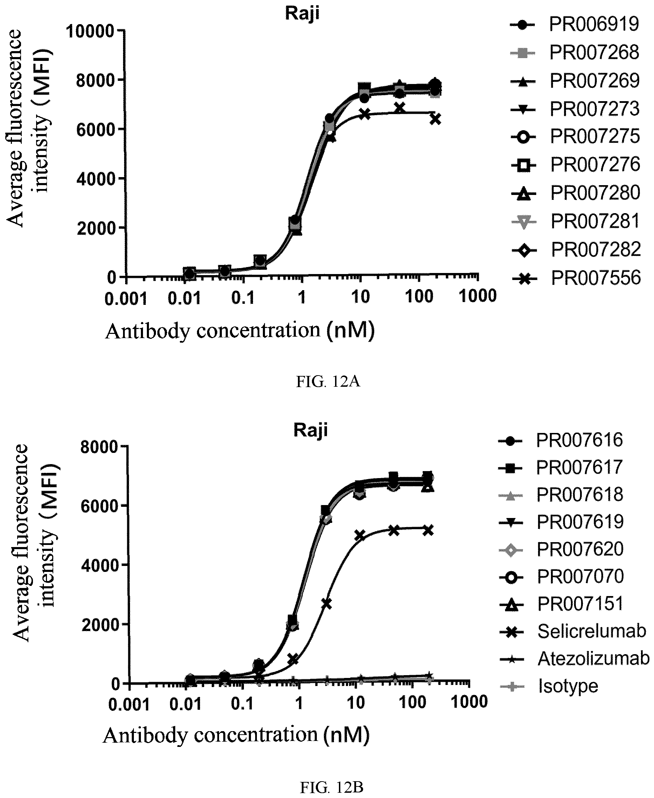

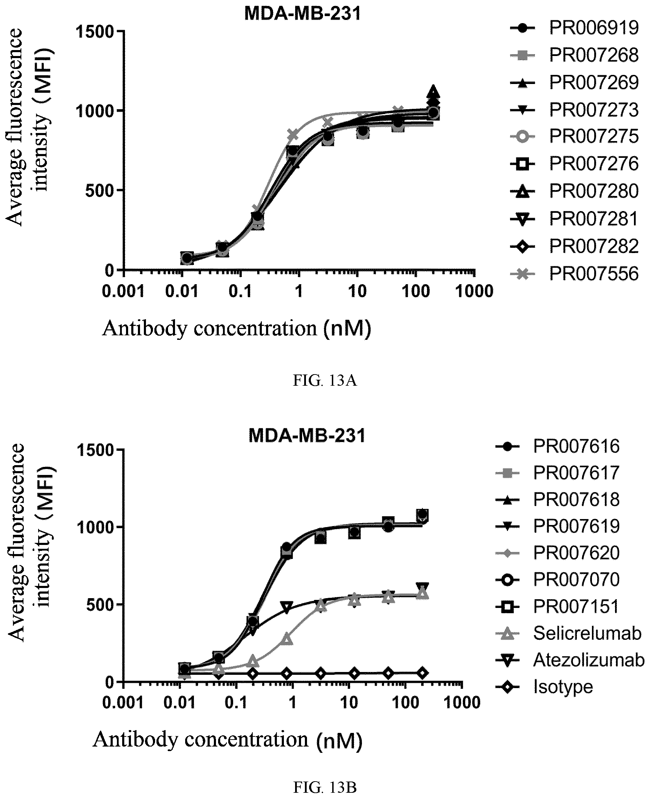

Table 19 summarized the corresponding results of binding of PD-L1×CD40 bispecific binding proteins and control molecule to various cells, corresponding figures showed the binding curves of the antigen-binding proteins to the target cell, and the corresponding tables listed the binding force parameters obtained by fitting the corresponding curves (EC₅₀ value and maximum MFI signal). FIG. 10A and FIG. 10B showed that PD-L1×CD40 did not bind to the CHO-K1 cell. FIG. 6A, FIG. 6B and Table 20 showed that PD-L1×CD40 bound to the CHO-K1/hPD-L1 cell. FIG. 7A, FIG. 7B and Table 21 showed that PD-L1×CD40 bound to the cynomolgus monkey PD-L1 cell. FIG. 8A, FIG. 8B and Table 22 showed that PD-L1×CD40 bound to the CHO-K1/hCD40 cell. FIG. 9A, FIG. 9B and Table 23 showed that PD-L1×CD40 bound to the cynomolgus monkey CD40 cell. FIG. 11A, FIG. 11B and Table 24 showed that PD-L1×CD40 bound to the NCI-H226 cell. FIG. 12A, FIG. 12B and Table 25 showed that PD-L1×CD40 bound to the Raji cell. FIG. 13A, FIG. 13B and Table 26 showed that PD-L1×CD40 bound to the MDA-MB-231 cell.

The results indicated that all PD-L1×CD40 bispecific antibodies could efficiently bind to the cell lines expressing PD-L1, such as CHO-K1/hPD-L1 (EC₅₀ being about 1 nM), CHO-K1/cy PD-L1 (EC₅₀ being less than 1 nM) and NCI-226 (EC₅₀ being less than 1 nM), the binding activities thereof were comparable to that of the positive control Atezolizumab; the PD-L1 ×CD40 bispecific antibodies did not bind to the CHO-K1 cell without expressing the target; the PD-L1×CD40 bispecific antibodies also efficiently bound to the MDA-MB-231 cell (EC₅₀ being less than 1 nM), and the binding effects thereof were significantly stronger than that of the positive control Atezolizumab (having a higher MFI signal).

Moreover, all PD-L1×CD40 bispecific antibodies could efficiently bind to CD40-expressing cell lines, such as CHO-K1/hCD40 (EC₅₀ being about 1-2 nM), CHO-K1/cyCD40 (EC₅₀ being about 1-4 nM) and Raji (EC₅₀ being about 1-2 nM), the binding activities thereof were stronger than or equivalent to that of the positive control Selicrelumab; the PD-L1×CD40 bispecific antibodies also efficiently bound to the MDA-MB-231 cell, and the binding effects thereof were significantly stronger than that of the positive control Selicrelumab.

**Table 19. Cell lines used in this example and corresponding figures of the FACS binding test data**

| **Cell line** | **PD-L1 positive** | **CD40 positive** | **Figure** | **Binding force parameter** |
|---|---|---|---|---|
| CHO-K1 | No | No | FIG. 10A-FIG. 10B | |
| CHO-K1/hPD-L1 | Human PD-L1 | No | FIG. 6A-FIG. 6B | Table 20 |
| CHO-K1/cyPD-L1 | Cynomolgus monkey PD-L1 | No | FIG. 7A-FIG. 7B | Table 21 |
| CHO-K1/hCD40 | No | Human CD40 | FIG. 8A-FIG. 8B | Table 22 |
| CHO-K1/cyCD40 | No | Cynomolgus monkey CD40 | FIG. 9A-FIG. 9B | Table 23 |
| NCI-H226 | Human PD-L1 | No | FIG. 11A-FIG. 11B | Table 24 |
| Raj i | No | Human CD40 | FIG. 12A-FIG. 12B | Table 25 |
| MDA-MB-231 | Human PD-L1 | Human CD40 | FIG. 13A-FIG. 13B | Table 26 |

**Table 20. Binding of PD-L1×CD40 to CHO-K1/hPD-L1 cell (corresponding to FIG. 6A and FIG. 6B)**

| | **Antibody** | **EC₅₀ (nM)** | **Maximum MFI** | | **Antibody** | **EC₅₀ (nM)** | **Maximum MFI** |
|---|---|---|---|---|---|---|---|
| (A) | PR006919 | 0.9602 | 19603 | (B) | PR007616 | 1.074 | 20292 |
| | PR007268 | 1.084 | 19580 | | PR007617 | 1.006 | 20174 |
| | PR007269 | 1.123 | 19691 | | PR007618 | 1.037 | 20103 |
| | PR007273 | 1.387 | 19836 | | PR007619 | 1.019 | 20141 |
| | PR007275 | 1.293 | 20036 | | PR007620 | 1.146 | 20323 |
| | PR007276 | 1.178 | 19692 | | PR007070 | 1.149 | 20509 |
| | PR007280 | 1.584 | 20168 | | PR007151 | 1.136 | 20719 |
| | PR007281 | 1.128 | 19888 | | Selicrelumab | | 127 |
| | PR007282 | 1.456 | 20425 | | Atezolizumab | 1.007 | 20497 |
| | PR007556 | 1.027 | 20058 | | Isotype | | 170 |

**Table 21. Binding of PD-L1×CD40 to CHO-K1/cyPD-L1 cell (corresponding to FIG. 7A and FIG. 7B)**

| | **Antibody** | **EC₅₀ (nM)** | **Maximum MFI** | | **Antibody** | **EC₅₀ (nM)** | **Maximum MFI** |
|---|---|---|---|---|---|---|---|
| (A) | PR006919 | 0.6221 | 4540 | (B) | PR007616 | 0.6867 | 4754 |
| | PR007268 | 0.6797 | 4553 | | PR007617 | 0.6351 | 4832 |
| | PR007269 | 0.7138 | 4672 | | PR007618 | 0.6904 | 4868 |
| | PR007273 | 0.8901 | 4621 | | PR007619 | 0.6292 | 4815 |
| | PR007275 | 0.8068 | 4617 | | PR007620 | 0.7572 | 4837 |
| | PR007276 | 0.656 | 4567 | | PR007070 | 0.7794 | 4873 |
| | PR007280 | 1.021 | 4827 | | PR007151 | 0.7538 | 4926 |
| | PR007281 | 0.6324 | 4611 | | Selicrelumab | | 94 |
| | PR007282 | 0.8344 | 4624 | | Atezolizumab | 0.6486 | 4567 |
| | PR007556 | 0.6831 | 4716 | | Isotype | | 50 |

**Table 22. Binding of PD-L1 ×CD40 to CHO-K1/hCD40 cell (corresponding to FIG. 8A and FIG. 8B)**

| | **Antibody** | **EC₅₀ (nM)** | **Maximum MFI** | | **Antibody** | **EC₅₀ (nM)** | **Maximum MFI** |
|---|---|---|---|---|---|---|---|
| (A) | PR006919 | 2.527 | 18896 | (B) | PR007616 | 1.987 | 19156 |
| | PR007268 | 1.648 | 16269 | | PR007617 | 1.962 | 16759 |
| | PR007269 | 2.401 | 16366 | | PR007618 | 2.223 | 16779 |
| | PR007273 | 2.046 | 14276 | | PR007619 | 2.498 | 17653 |
| | PR007275 | 2.57 | 14195 | | PR007620 | 2.202 | 15328 |
| | PR007276 | 2.345 | 14269 | | PR007070 | 2.672 | 15443 |
| | PR007280 | 3.829 | 14965 | | PR007151 | 2.362 | 15996 |
| | PR007281 | 2.131 | 14187 | | Selicrelumab | 2.579 | 13925 |
| | PR007282 | 1.884 | 13455 | | Atezolizumab | | 34 |
| | PR007556 | 1.686 | 14593 | | Isotype | | 23 |

**Table 23. Binding of PD-L1 ×CD40 to CHO-K1/cyCD40 cell (corresponding to FIG. 9A and FIG. 9B)**

| | **Antibody** | **EC₅₀ (nM)** | **Maximum MFI** | | **Antibody** | **EC₅₀ (nM)** | **Maximum MFI** |
|---|---|---|---|---|---|---|---|
| (A) | PR006919 | 3.372 | 76218 | (B) | PR007616 | 2.221 | 64689 |
| | PR007268 | 2.798 | 65674 | | PR007617 | 2.452 | 58657 |
| | PR007269 | 2.566 | 52983 | | PR007618 | 3.069 | 67893 |
| | PR007273 | 4.201 | 71338 | | PR007619 | 1.768 | 51274 |
| | PR007275 | 4.119 | 65963 | | PR007620 | 2.483 | 68025 |
| | PR007276 | 2.623 | 56976 | | PR007070 | 15.61 | 93201 |
| | PR007280 | 2.649 | 50086 | | PR007151 | 3.759 | 75424 |
| | PR007281 | 3.628 | 64981 | | Selicrelumab | 4.737 | 53385 |
| | PR007282 | 2.434 | 50135 | | Atezolizumab | | 1054 |
| | PR007556 | 2.584 | 55548 | | Isotype | | 59 |

**Table 24. Binding of PD-L1 ×CD40 to NCI-H226 cell (corresponding to FIG. 11A and FIG. 11B)**

| | **Antibody** | **EC₅₀ (nM)** | **Maximum MFI** | | **Antibody** | **EC₅₀ (nM)** | **Maximum MFI** |
|---|---|---|---|---|---|---|---|
| (A) | PR006919 | 0.3887 | 870 | (B) | PR007616 | 0.3033 | 800 |
| | PR007268 | 0.4204 | 869 | | PR007617 | 0.337 | 831 |
| | PR007269 | 0.5208 | 869 | | PR007618 | 0.3218 | 902 |
| | PR007273 | 0.7726 | 914 | | PR007619 | 0.3657 | 914 |
| | PR007275 | 0.7638 | 959 | | PR007620 | 0.2812 | 1034 |
| | PR007276 | 0.5693 | 927 | | PR007070 | 0.3131 | 862 |
| | PR007280 | 1.019 | 1020 | | PR007151 | 0.2151 | 928 |
| | PR007281 | 0.5027 | 921 | | Selicrelumab | | 48 |
| | PR007282 | 0.6104 | 983 | | Atezolizumab | 0.2411 | 842 |
| | PR007556 | 0.4522 | 861 | | Isotype | | 55 |

**Table 25. Binding of PD-L1 ×CD40 to Raji cell (corresponding to FIG. 12A and FIG. 12B)**

| | **Antibody** | **EC₅₀ (nM)** | **Maximum MFI** | | **Antibody** | **EC₅₀ (nM)** | **Maximum MFI** |
|---|---|---|---|---|---|---|---|
| (A) | PR006919 | 1.237 | 7359 | (B) | PR007616 | 1.24 | 6677 |
| | PR007268 | 1.379 | 7431 | | PR007617 | 1.275 | 6846 |
| | PR007269 | 1.46 | 7571 | | PR007618 | 1.228 | 6695 |
| | PR007273 | 1.431 | 7582 | | PR007619 | 1.261 | 6794 |
| | PR007275 | 1.576 | 7647 | | PR007620 | 1.319 | 6709 |
| | PR007276 | 1.449 | 7573 | | PR007070 | 1.356 | 6668 |
| | PR007280 | 1.647 | 7695 | | PR007151 | 1.296 | 6614 |
| | PR007281 | 1.462 | 7600 | | Selicrelumab | 3.019 | 5174 |
| | PR007282 | 1.446 | 7516 | | Atezolizumab | | 221 |
| | PR007556 | 1.284 | 6564 | | Isotype | | 78 |

**Table 26. Binding of PD-L1×CD40 to MDA-MB-231 cell (corresponding to FIG. 13A and FIG. 13B)**

| | **Antibody** | **EC₅₀ (nM)** | **Maximum MFI** | | **Antibody** | **EC₅₀ (nM)** | **Maximum MFI** |
|---|---|---|---|---|---|---|---|
| (A) | PR006919 | 0.3256 | 924 | (B) | PR007616 | 0.292 | 1005 |
| | PR007268 | 0.34 | 907 | | PR007617 | 0.3012 | 1014 |
| | PR007269 | 0.369 | 960 | | PR007618 | 0.3177 | 1023 |
| | PR007273 | 0.4338 | 983 | | PR007619 | 0.2828 | 1010 |
| | PR007275 | 0.3886 | 917 | | PR007620 | 0.3262 | 1016 |
| | PR007276 | 0.3343 | 908 | | PR007070 | 0.314 | 1006 |
| | PR007280 | 0.4724 | 1014 | | PR007151 | 0.3143 | 1016 |
| | PR007281 | 0.3465 | 923 | | Selicrelumab | 0.9358 | 563 |
| | PR007282 | 0.4043 | 952 | | Atezolizumab | 0.1392 | 554 |
| | PR007556 | 0.2996 | 991 | | Isotype | | 58 |

### Example 10. Inhibition of PD-1 signaling pathway by PD-L1×CD40 in fluorescent reporter gene experiments

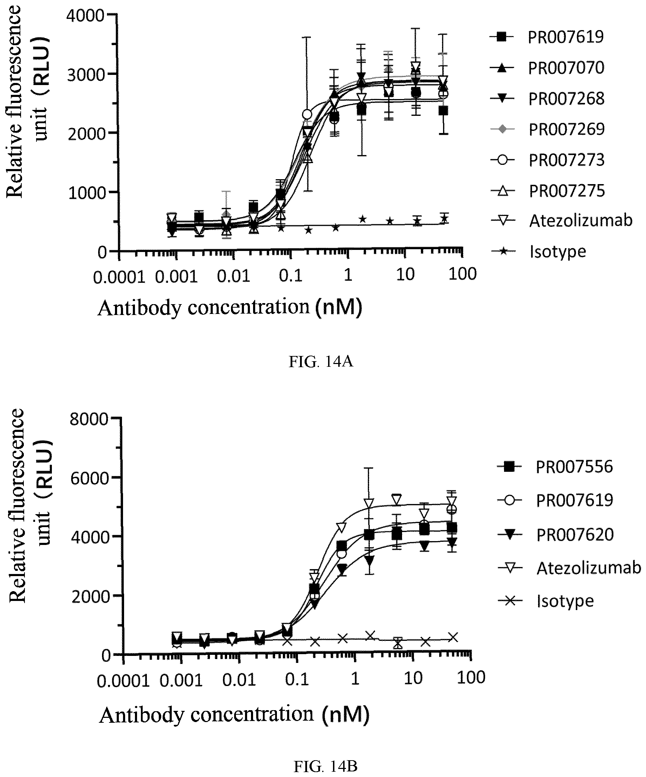

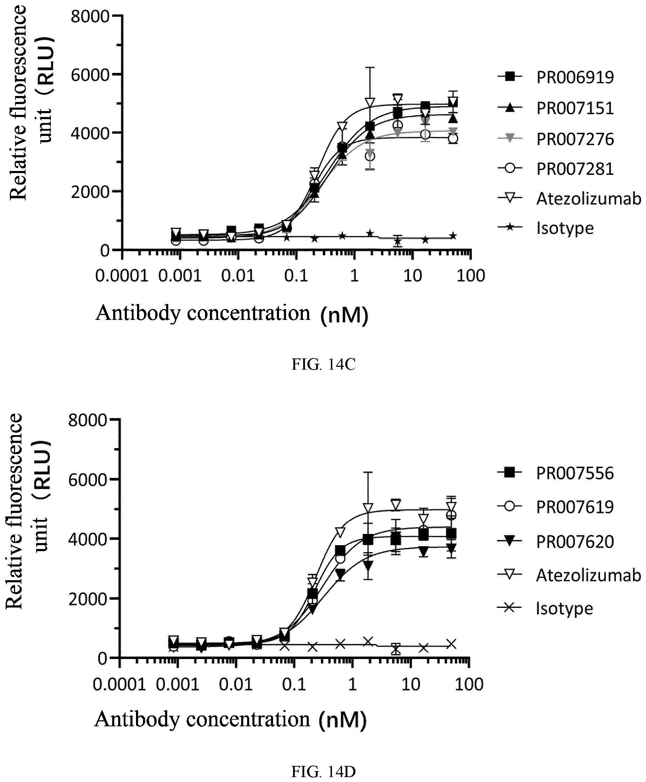

In this example, the method described in 0 was used to test the inhibitory effect of the PD-L1 ×CD40 bispecific binding proteins on the PD-1 signaling pathway. The results were as shown in FIG. 14A-FIG. 14D and Table 27. All PD-L1×CD40 bispecific antibodies could effectively block PD-1/PD-L1 interaction and activate TCR and NFAT signals, and the activities were comparable to that of the positive control antibody Atezolizumab (EC₅₀ being about 0.1 - 0.3 nM).

**Table 27. Inhibition of the PD-1 signaling pathway of Jurkat-NFAT-hPD1 by PD-L1×CD40 (corresponding to FIG. 14A-FIG. 14D)**

| | **Antibody** | **EC₅₀ (nM)** | **Maximum (RLU)** | | **Antibody** | **EC₅₀ (nM)** | **Maximum (RLU)** |
|---|---|---|---|---|---|---|---|
| (A) | PR007619 | 0.1222 | 2481 | (B) | PR007280 | 0.2952 | 2559 |
| | PR007070 | 0.1753 | 2828 | | PR007282 | 0.1884 | 2480 |
| | PR007268 | 0.1815 | 2813 | | PR007616 | 0.2037 | 2586 |
| | PR007269 | 0.1621 | 2912 | | PR007617 | 0.1569 | 2192 |
| | PR007273 | 0.1092 | 2520 | | PR007618 | 0.1204 | 2473 |
| | PR007275 | 0.2342 | 2840 | | PR007619 | 0.1222 | 2481 |
| | Atezolizumab | 0.1594 | 2761 | | Atezolizumab | 0.1594 | 2761 |
| | Isotype | | 560 | | Isotype | | 560 |
| (C) | PR006919 | 0.3586 | 4916 | (D) | PR007556 | 0.2251 | 4078 |
| | PR007151 | 0.3498 | 4636 | | PR007619 | 0.309 | 4394 |
| | PR007276 | 0.2732 | 4062 | | PR007620 | 0.3193 | 3735 |
| | PR007281 | 0.19 | 3837 | | Atezolizumab | 0.2379 | 4981 |
| | Atezolizumab | 0.2379 | 4981 | | Isotype | | 403 |
| | Isotype | | 403 | | | | |

### Example 11. Activation of CD40 signaling pathway by PD-L1×CD40 in fluorescent reporter gene experiments

In this example, the method described in 0 was used to test the activation effect of PD-L1×CD40 bispecific binding proteins in the presence of or in the absence of crosslinking mediated by PD-L1 on CD40. The cell highly expressing PD-L1, CHO-K1/hPD-L1 was used to perform crosslinking of the molecules to be tested, to study whether crosslinking could further enhance the ability to activate CD40. In this experiment, CHO-K1/hPD-L1 cell highly expressing PD-L1 could crosslink the PD-L1×CD40 bispecific antibody molecules by using PD-L1, while the CHO-K1 cell that do not express PD-L1or CD40 could not bind to the molecules to be tested. The activations of HEK293-hCD40-NFκB reporter gene system by the molecules to be tested after incubation with CHO-K1/hPD-L1 cell or with CHO-K1 cell were tested respectively.

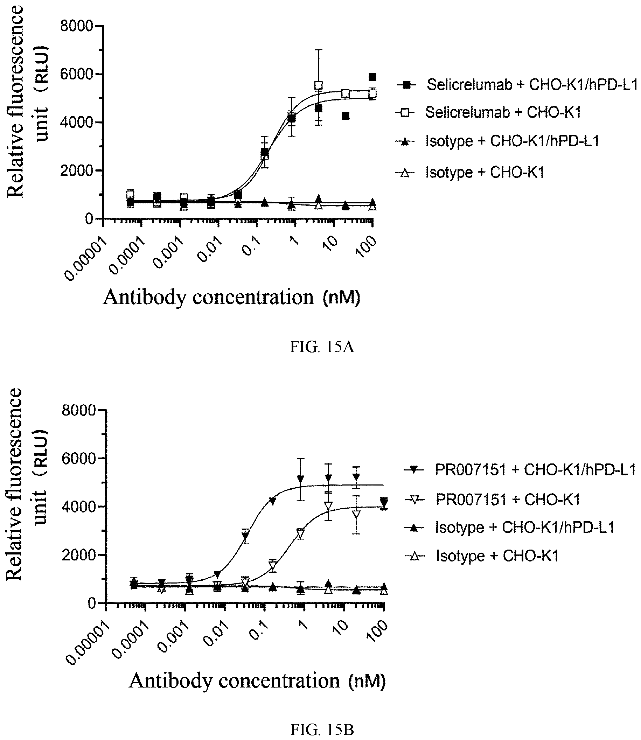

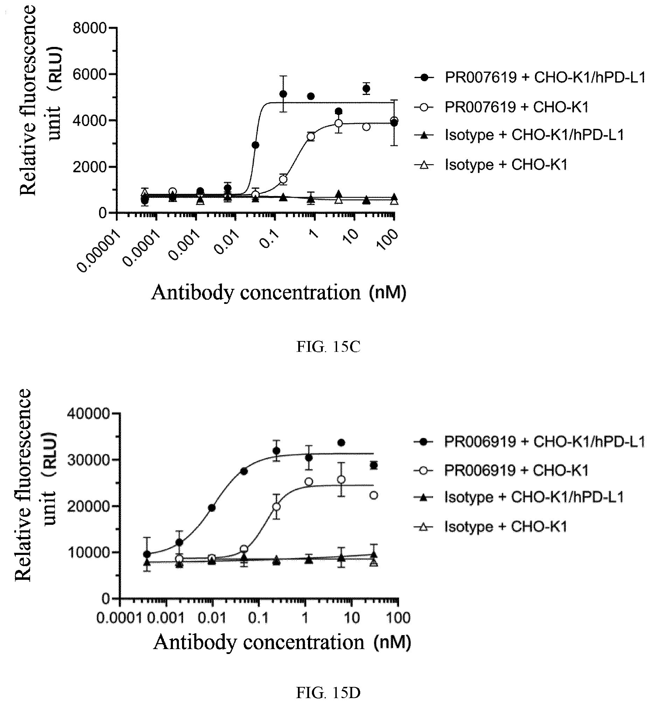

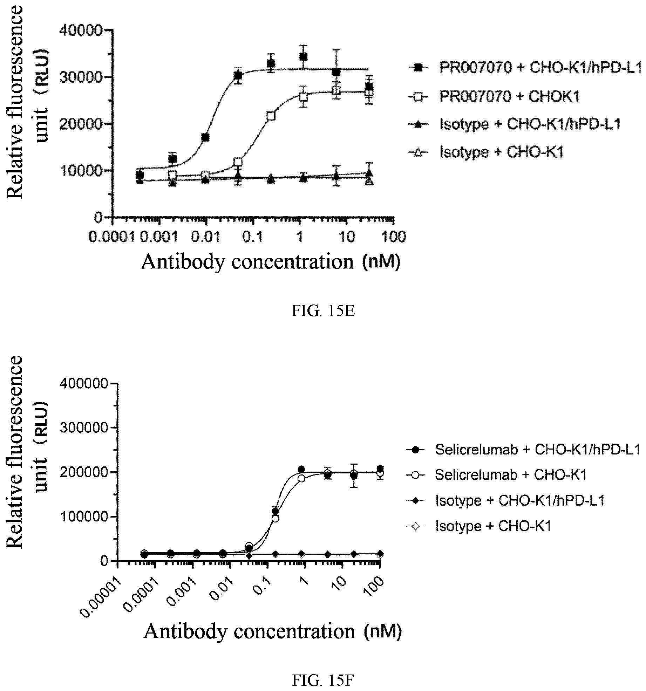

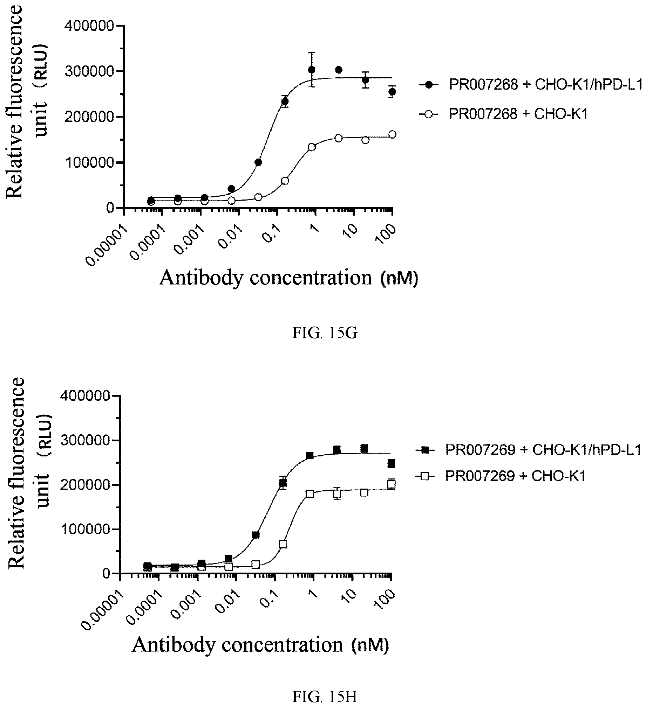

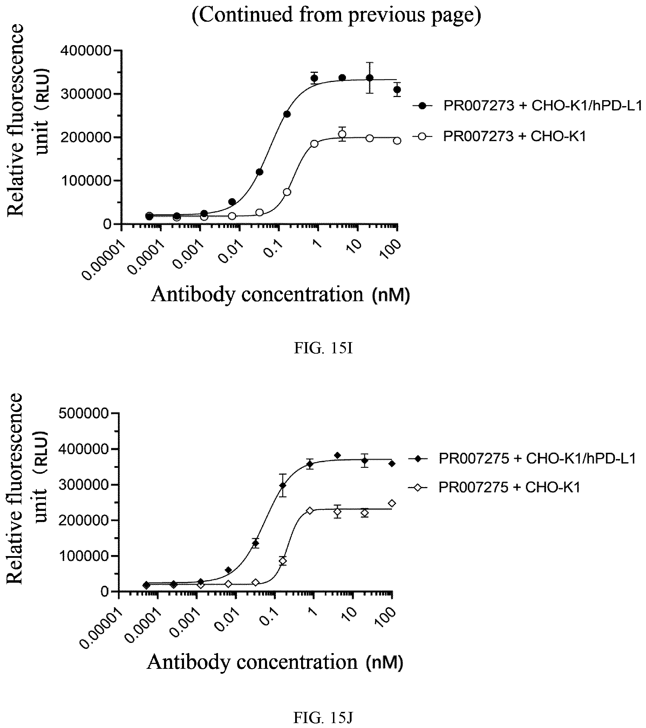

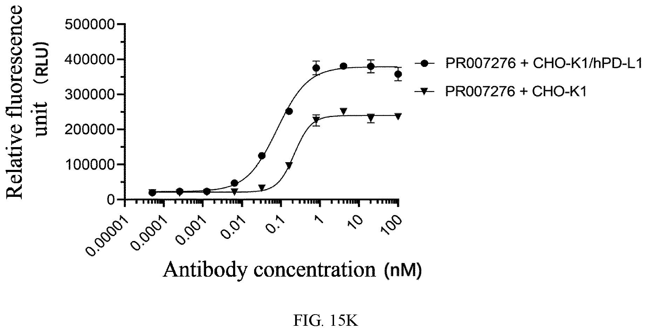

The results, as shown in FIG. 15A-FIG. 15K and Table 28, indicated that the CD40 antibody Selicrelumab could activate the downstream signaling pathway of CD40, and this activation was independent of the presence of exogenous crosslinking, where EC₅₀ was about 0.2 nM. However, the activation effect of the PD-L1×CD40 bispecific antibodies (such as PR007151 and PR007619) in the presence of PD-L1 crosslinking (EC₅₀ being about 0.03 nM) on the downstream signaling pathway of CD40 was significantly stronger than that in the absence of PD-L1 crosslinking (EC₅₀ being about 0.3-0.4 nM). The results of the remaining experiments, such as the tests on PR006919 and PR007070, and the tests on PR007268, PR007269, PR007273, PR007275 and PR007276, were similar, that is, the PD-L1×CD40 bispecific antibodies could better activate the CD40 downstream signaling pathway by crosslinking of CD40 to PD-L1.

**Table 28. Activation of CD40 signaling pathway in HEK293-hCD40-NFκB by PD-L1 ×CD40 in the presence of or in the absence of crosslinking (corresponding to FIG. 15A-FIG. 15K).**

| | **Antibody** | **EC₅₀ (nM)** | **Maximum (RLU)** |
|---|---|---|---|
| (A) | Selicrelumab + CHO-K1/hPD-L1 | 0.2047 | 5010 |
| | Selicrelumab + CHO-K1 | 0.2409 | 5318 |
| (B) | PR007151 + CHO-K1/hPD-L1 | 0.0362 | 4897 |
| | PR007151 + CHO-K1 | 0.4249 | 3995 |
| (C) | PR007619 + CHO-K1/hPD-L1 | 0.03122 | 4776 |
| | PR007619 + CHO-K1 | 0.3342 | 3883 |
| (D) | PR006919 + CHO-K1/hPD-L1 | 0.0106 | 31360 |
| | PR006919 + CHO-K1 | 0.1442 | 24537 |
| (E) | PR007070 + CHO-K1/hPD-L1 | 0.0138 | 31678 |
| | PR007070 + CHO-K1 | 0.1356 | 26870 |
| (F) | Selicrelumab + CHO-K1/hPD-L1 | 0.1555 | 200391 |
| | Selicrelumab + CHO-K1 | 0.1828 | 199114 |
| (G) | PR007268 + CHO-K1/hPD-L1 | 0.05714 | 286353 |
| | PR007268 + CHO-K1 | 0.2658 | 156013 |
| (H) | PR007269 + CHO-K1/hPD-L1 | 0.06915 | 270981 |
| | PR007269 + CHO-K1 | 0.2345 | 188712 |
| (I) | PR007273 + CHO-K1/hPD-L1 | 0.05979 | 333115 |
| | PR007273 + CHO-K1 | 0.2383 | 199499 |
| (J) | PR007275 + CHO-K1/hPD-L1 | 0.05575 | 370815 |
| | PR007275 + CHO-K1 | 0.2133 | 231364 |
| (K) | PR007276 + CHO-K1/hPD-L1 | 0.08001 | 378878 |
| | PR007276 + CHO-K1 | 0.2202 | 239239 |

### Example 12. Activation effect of PD-L1×CD40 on DC cell

In this example, the activation effect of PD-L1×CD40 bispecific binding proteins in the presence of or in the absence of crosslinking mediated by PD-L1 on dendritic cell (DC) was studied.

In this example, PBMC-derived monocytes were induced into DCs which were used in activation experiments. First, monocytes were sorted from PBMCs using a human CD14 sorting kit (Miltenyi, #130-050-201), cultured with RPMI1640 complete medium, and induced with 50 ng/mL of IL-4 (R&D Systems, #204-IL) and 100 ng/mL of GM-CSF (R&D Systems, #215-GM) cytokine for six days to become immature dendritic cells (iDCs). Half of the iDCs were induced with 1 µg/mL of LPS (Sigma, #L2880-10MG) for 24 hours to become mature dendritic cells (mDCs). mDCs from donors #1 and #2 were used directly after 24 hours of LPS induction. mDCs from donor #4 were rested for 24 hours for use after LPS was washed out and RPMI1640 complete medium, 50 ng/mL of IL-4 and 100 ng/mL of GM-CSF cytokine were added again.

CHO-K1 and CHO-K1/hPD-L1 cells were seeded in a 96-well flat-bottom plate (Corning, #3599) one day before mDC induction was completed. First, CHO-K1 and CHO-K1/hPD-L1 cells were collected, 50 µg/mL of mitomycin C (Selleckchem, #S8146) was added, and then reaction was carried out at 37°C for 1 hour to inhibit cell proliferation. After washing, the cells were adjusted to a cell density of 1×10⁵ cells/mL using F-12K complete medium and added to a 96-well flat-bottom plate at 100 µL/well, and the plate was finally placed in a cell incubator overnight. The next day, the antibody to be tested was diluted to 2× final concentration using RPMI1640 complete medium, and then subjected to 10-fold or 3-fold gradient dilution. Selicrelumab and hIgG1 were used as positive and negative controls, respectively. The culture media of CHO-K1 and CHO-K1/hPD-L1 cells were discarded and the antibody was added at 100 µL/well. iDCs and mDCs were then collected, adjusted to a cell density of 5 × 10⁵ cells/mL, added to a 96-well plate at 100 µL/well and mixed with the above-mentioned antibodies to be tested, and the plate was finally placed in a cell incubator for culture. Three days later, the cell supernatant was collected and the secretion level of IL12p40 was detected using a human IL-12/-23 (p40) ELISA kit (MABTECH, #3450-1H-20). Data processing and graphical analysis were performed using GraphPad Prism 8 software to show the IL12p40 secretion level related to antibody concentration.

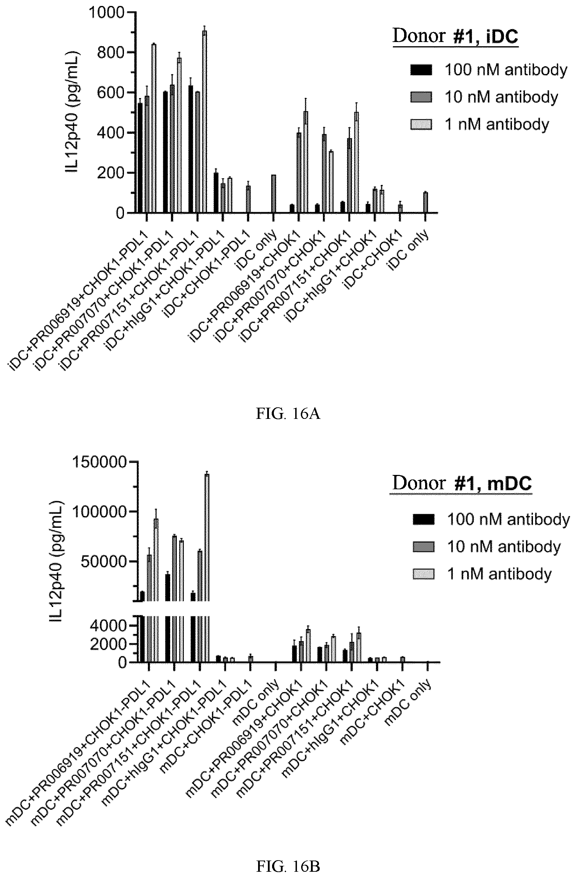

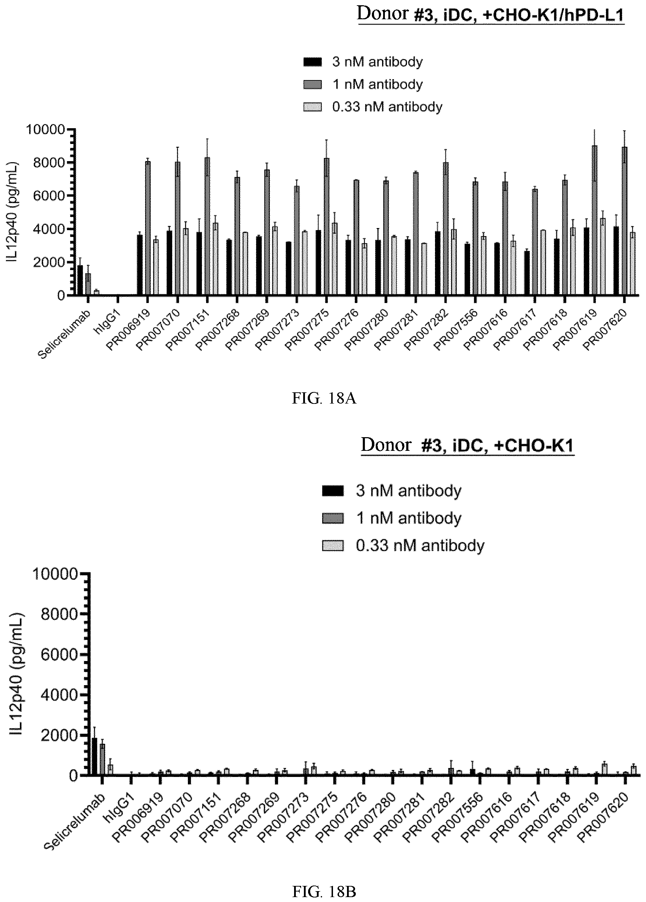

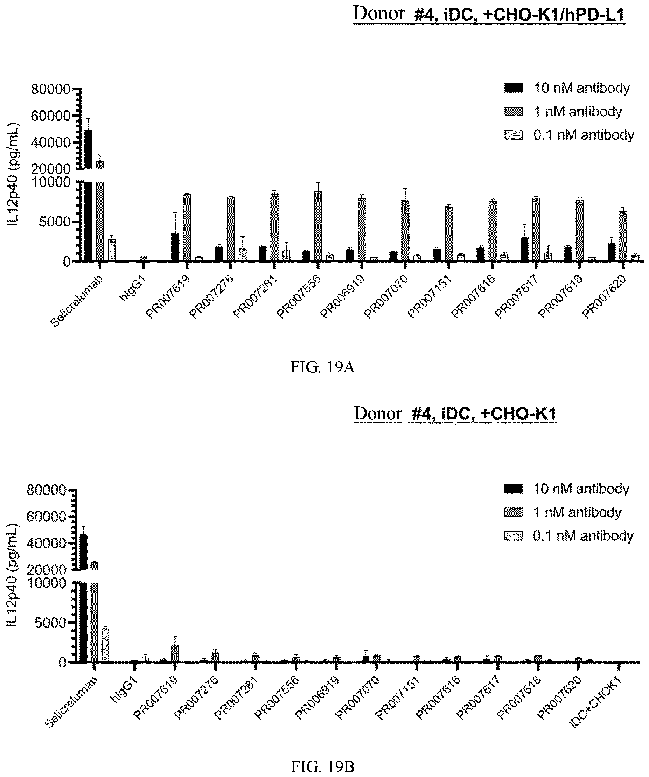

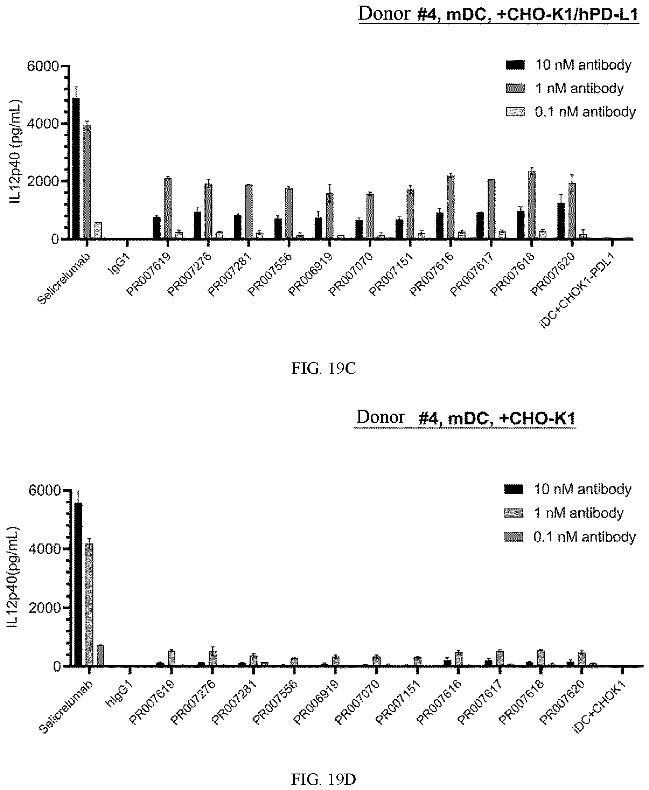

In this example, the PD-L1×CD40 bispecific antibodies were tested multiple times using different types of DC cells from multiple donors, and the results are shown in FIG. 16A and FIG. 16B, FIG. 17A and FIG. 17B, FIG. 18A and FIG. 18B, and FIG. 19A-FIG. 19D. Table 29 summarized the important parameters of the experiments corresponding to each figure, such as DC source, target cell for crosslinking, and tested molecule.

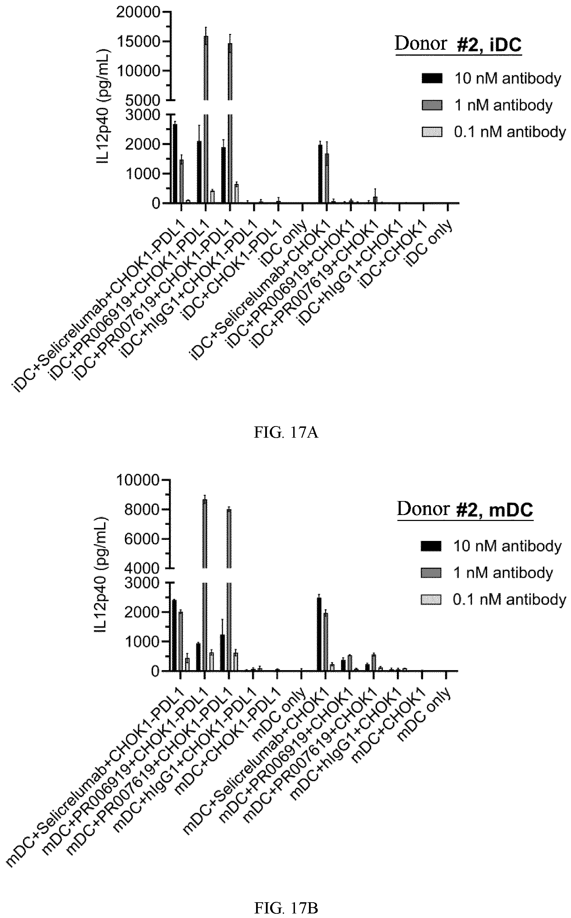

The results indicated that the positive control antibody Selicrelumab could effectively activate both iDC cells and mDC cells, and this activation effect was not affected by the tumor antigen PD-L1. The PD-L1×CD40 bispecific antibodies, such as PR006919, PR007070 and PR007151, all conditionally activated iDC and mDC cells; that is, DCs could be efficiently activated in the presence of PD-L1 crosslinking; and DCs were not activated or activated at a lower level in the absence of PD-L1 crosslinking. The property of this selective activation effect of the PD-L1×CD40 bispecific antibodies is not available to Selicrelumab, and also provides a basis for bispecific antibody molecules to exert efficacy efficiently and safely. In addition, as shown in FIG. 17A-FIG. 17B, the activation effect of PD-L1×CD40 bispecific antibodies was significantly stronger than that of Selicrelumab in experiments of some donor DCs.

**Table 29. Figures corresponding to different batches of DC activation experiments in this example**

| **Figure** | **DC source** | **Target cell** | **Test molecule** |
|---|---|---|---|
| FIG. 16A | Donor #1, iDC | CHO-K1/hPD-L1 | PR006919, PR007070, PR007151 |
| | | CHO-K1 | |
| FIG. 16B | Donor #1, mDC | CHO-K1/hPD-L1 | |
| | | CHO-K1 | |
| FIG. 17A | Donor #2, iDC | CHO-K1/hPD-L1 | PR006919, PR007619, Selicrelumab |
| | | CHO-K1 | |
| FIG. 17B | Donor #2, mDC | CHO-K1/hPD-L1 | |
| | | CHO-K1 | |
| FIG. 18A | Donor #3, iDC | CHO-K1/hPD-L1 | Selicrelumab, PR006919, PR007070, PR007151, PR007268, PR007269, PR007273, PR007275, PR007276, PR007280, PR007281, PR007282, PR007556, PR007616, PR007617, PR007618, PR007619, PR007620 |
| FIG. 18B | Donor #3, iDC | CHO-K1 | |
| FIG. 19A | Donor #4, iDC | CHO-K1/hPD-L1 | Selicrelumab, PR007619, PR007276, PR007281, PR007556, PR006919, PR007070, PR007151, PR007616, PR007617, PR007618, PR007620 |
| FIG. 19B | Donor #4, iDC | CHO-K1 | |
| FIG. 19C | Donor #4, mDC | CHO-K1/hPD-L1 | |
| FIG. 19D | Donor #4, mDC | CHO-K1 | |

### Example 13. Activation effect of PD-L1×CD40 on B cell

In this example, the effect of PD-L1×CD40 bispecific binding proteins in the presence of or in the absence of crosslinking mediated by PD-L1 on B cell proliferation was studied.

CHO-K1 and CHO-K1/hPD-L1 cells were seeded in a 96-well flat-bottom plate (Corning, #3599) one day in advance. First, CHO-K1 and CHO-K1/hPD-L1 cells were collected, 50 µg/mL of mitomycin C (Selleckchem, #S8146) was added, and then reaction was carried out at 37°C for 1 hour to inhibit cell proliferation. After washing, the cells were adjusted to a cell density of 1×10⁵ cells/mL using F-12K complete medium and added to a 96-well flat-bottom plate at 100 µL/well, and the plate was placed in a cell incubator overnight. The next day, the antibody to be tested was diluted to 2× final concentration using RPMI1640 complete medium, and then subjected to 10-fold gradient dilution. Selicrelumab and hIgG1 were used as positive and negative controls, respectively. The working concentrations of the antibodies to be tested were 100 nM, 10 nM, and 1 nM. The culture media of CHO-K1 and CHO-K1/hPD-L1 cells were discarded and the antibody was added at 100 µL/well. B cells were then sorted from donor PBMCs using a human CD19 sorting kit (Miltenyi, #130-050-301); and 1 µM CFSE (Invitrogen, #34554) was added for labeling at 37°C for 20 minutes. After washing twice, the cells were adjusted to a cell density of 1×10⁶ cells/mL, added to a 96-well plate at 100 µL/well and mixed with the above-mentioned antibodies to be tested, and the plate was finally placed in a cell incubator for culture. Six days later, the cells were transferred to a 96-well U-bottom plate (Corning, #3799) and centrifuged at 500 g for 4 min at 4°C. The supernatant was discarded, dead cell dye (Invitrogen, #L34976A, 1:1000 dilution) was added at 100 µL/well, and the cells were incubated at 4°C in the dark for 15 minutes. The cells were washed twice with PBS at 200 µL/well, resuspended with 100 µL of Fc Block antibody (BD, #564220, 1:50 dilution), and incubated at 4°C in the dark for 15 minutes. Then, anti-human CD19 antibody (Biolegend, #302234) was added at 2 µL/well, and the cells were incubated at 4°C in the dark for 30 minutes. The cells were washed twice with 2% FBS PBS at 200 µL/well, and finally resuspended with 100 µL of 2% FBS PBS. The fluorescence signal values were read using a BD FACS CANTOII flow cytometer. Data processing and graphical analysis were performed using GraphPad Prism 8 software.

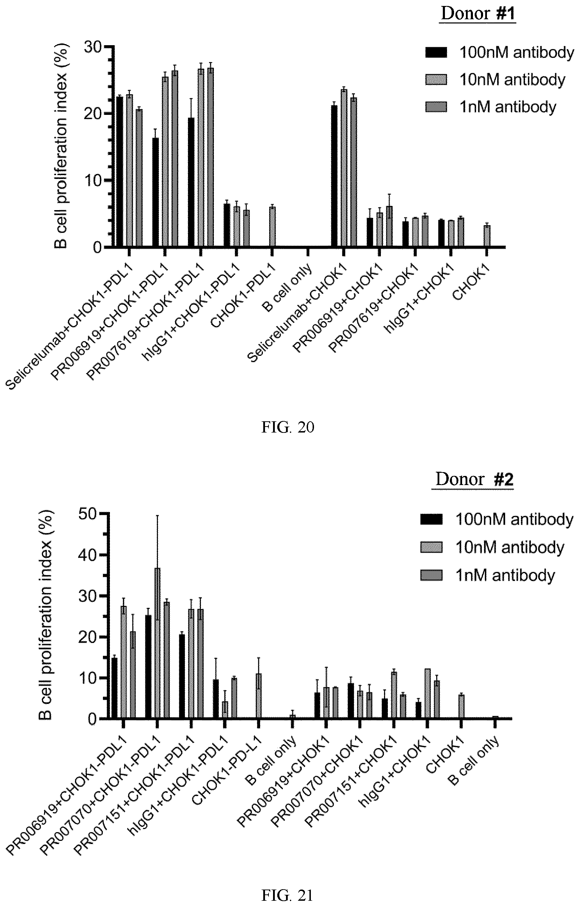

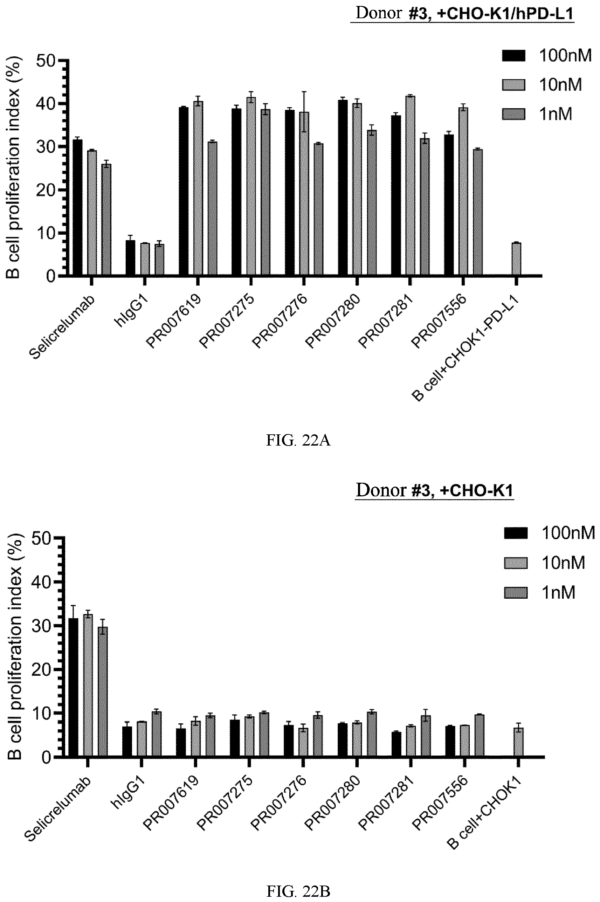

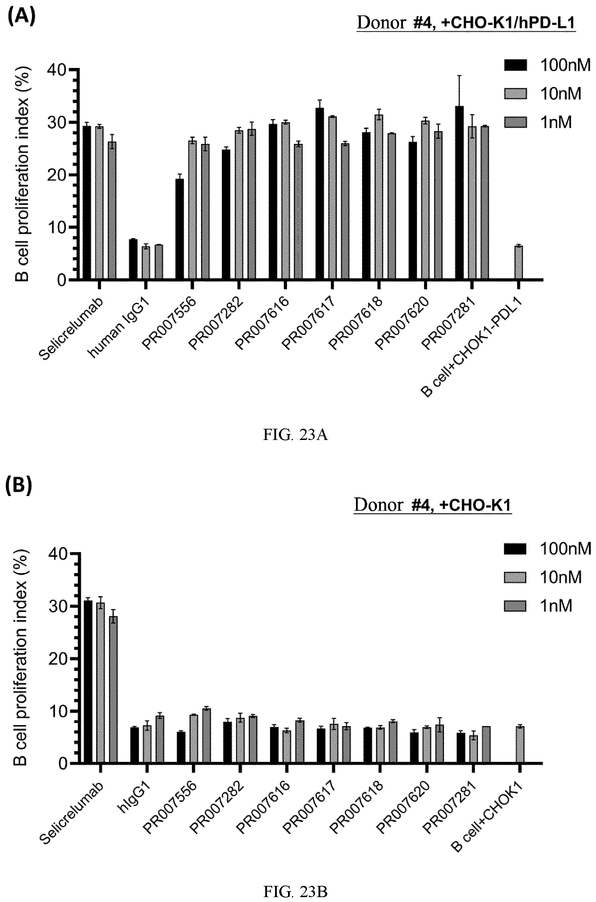

In this example, the PD-L1×CD40 bispecific antibodies were tested multiple times using B cells from multiple donors, and the results are shown in FIG. 20A and FIG. 20B, FIG. 21A and FIG. 21B, FIG. 22A and FIG. 22B, FIG. 23A and FIG. 23B, and FIG. 24A and FIG. 24B. Table 30 summarized the important parameters of the experiments corresponding to each figure, such as B cell source, target cell for crosslinking, and tested molecule.

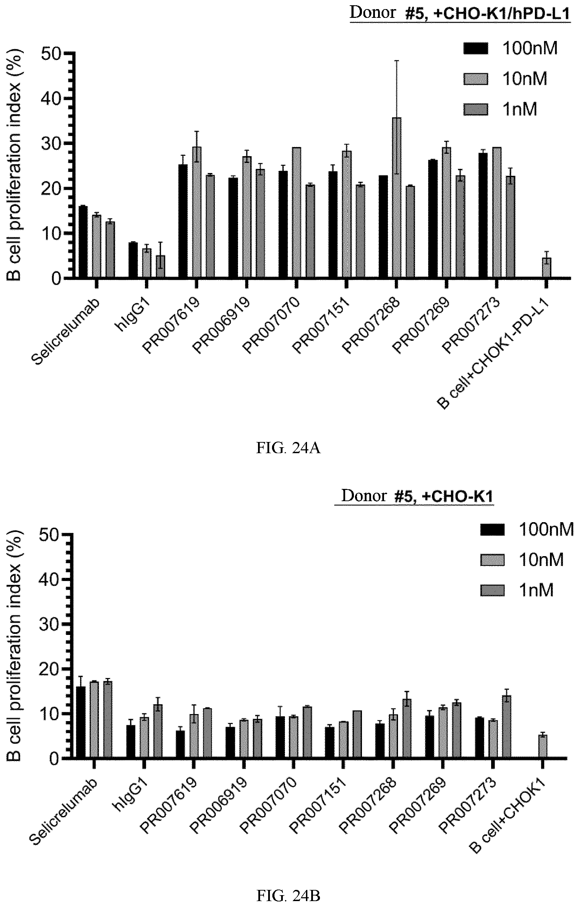

The results indicated that the positive control antibody Selicrelumab could effectively promote B cell proliferation, but this promoting effect was not affected by the tumor antigen PD-L1. The PD-L1×CD40 bispecific antibodies, such as PR006919, PR007070 and PR007151, all conditionally promoted B cell proliferation; that is, B cell proliferation could be efficiently promoted in the presence of PD-L1 crosslinking; and there was almost no promotion effect or only a weak promotion effect in the absence of PD-L1 crosslinking. The property of this selective activation effect of the PD-L1 ×CD40 bispecific antibodies is not available to Selicrelumab, and also provides a basis for bispecific antibody molecules to exert efficacy efficiently and safely. In addition, as shown in FIG. 24A-FIG. 24B, the proliferation-promoting effect of PD-L1×CD40 bispecific antibodies was significantly stronger than that of Selicrelumab in proliferation experiments of some donor B cell.

**Table 30. Figures corresponding to different batches of B cell proliferation experiments in this example**

| **Figure** | **B cell source** | **Target cell** | **Test molecule** |
|---|---|---|---|
| FIG. 20 | Donor #1 | CHO-K1/hPD-L1 | Selicrelumab, PR006919, PR007619 |
| | | CHO-K1 | |
| FIG. 21 | Donor #2 | CHO-K1/hPD-L1 | PR006919, PR007070, PR007151 |
| | | CHO-K1 | |
| FIG. 22A | Donor #3 | CHO-K1/hPD-L1 | Selicrelumab, PR007619, PR007275, PR007276, PR007280, PR007281, PR007556 |
| FIG. 22B | Donor #3 | CHO-K1 | |
| FIG. 23A | Donor #4 | CHO-K1/hPD-L1 | Selicrelumab, PR007556, PR007282, PR007616, PR007617, PR007618, PR007620, PR007281 |
| FIG. 23B | Donor #4 | CHO-K1 | |
| FIG. 24A | Donor #5 | CHO-K1/hPD-L1 | Selicrelumab, PR007619, PR006919, PR007070, PR007151, PR007268, PR007269, PR007273 |
| FIG. 24B | Donor #5 | CHO-K1 | |

### Example 14. Study on anti-tumor efficacy and safety of PD-L1×CD40s

In this example, the anti-tumor efficacy of PD-L1×CD40 bispecific binding proteins in the hPD1×hCD40-C57BL/6J mouse MC38-hPDL1 tumor model was studied. MC38-hPDL1 cell is an MC38 mouse colon cancer cell overexpressing human PD-L1. hPD1×hCD40-C57BL/6J mouse is a PD1/CD40-humanized transgenic mouse obtained by knocking human PD-1 and human CD40 genes into C57BL/6J mouse (provided by Shanghai Model Organisms Center, Inc.).

MC38-hPDL1 cells were cultured in an incubator at 37°C and 5% CO₂, where the medium was DMEM medium containing 10% inactivated fetal bovine serum. The cells grew to fill the culture dish every 3 to 4 days, and then were split to separate dish(s) for subculture. MC38-hPDL1 in the logarithmic growth phase was taken, resuspended in PBS, counted, and adjusted to a cell concentration of 1.0×10⁷/mL. The cell suspension was inoculated subcutaneously in the right lateral thorax of hPD1×hCD40-C57BL/6J mice at 100 µL/mouse using a 1 mL syringe, with about 1.0×10⁶ tumor cells inoculated per mouse. When the average tumor volume reached a certain volume, mice with a moderate tumor volume were picked and randomly divided to each experimental group according to tumor volumes, with N mice per group. The mice were administrated from the day of grouping, twice a week (BIW), for a total of 7 times, by intraperitoneal injection. On the day of administration, the tumor volume was measured and the mouse body weight was recorded.

### Example 14.1. Mouse efficacy experiment #1

In the first experiment, when the average tumor volume reached 110 mm³, mice with a moderate tumor volume were picked, and grouped and administrated according to the schemes in Table 31.

**Table 31. Experimental protocol design and tumor inhibition rate of each administration group**

| **Group** | **Number of mice (N)** | **Drug, dosage** | **Administration frequency** | **Tumor inhibition rate (%)** |
|---|---|---|---|---|
| Group 1 | 10 | Isotype, 3 mg/kg | BIW, 7 times | |
| Group 2 | 10 | Selicrelumab, 3 mg/kg | BIW, 7 times | 93.89 |
| Group 3 | 10 | Atezolizumab, 3 mg/kg | BIW, 7 times | 30.81 |
| Group 4 | 10 | [Selicrelumab, 3 mg/kg] + [Atezolizumab, 3 mg/kg] | BIW, 7 times | 102.78 |
| Group 5 | 10 | PR007070, 3.6 mg/kg | BIW, 7 times | 74.41 |
| Group 6 | 10 | PR007151, 3.6 mg/kg | BIW, 7 times | 81.10 |

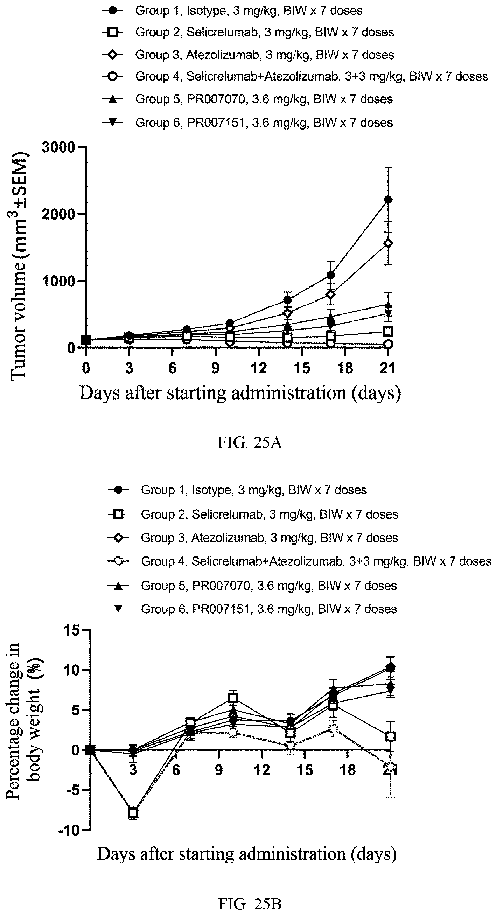

The results are shown in FIG. 25A and FIG. 25B. On day 21 after starting administration, the average tumor volume of the control group Group 1 (Isotype) was 2210.35 ± 487.58 mm³. The tumor volumes of administration groups Group 2 (Selicrelumab), Group 4 (Selicrelumab+Atezolizumab), Group 5 (PR007070) and Group 6 (PR007151) were 239.07 ± 73.25 mm³, 52.33 ± 18.99 mm³, 647.92 ± 176.36 mm³ and 507.58 ± 116.91 mm³, respectively, and the corresponding tumor inhibition rates were 93.89%, 102.78%, 74.41% and 81.10%, respectively, which has a significant statistical difference compared with that of Group 1 (Isotype) (P < 0.01). For Group 3 (Atezolizumab), the tumor volume was 1563.33 ± 325.77 mm³ and the tumor inhibition rate was 30.81%.

On day 3 after starting administration, the body weights of the mice in Group 2 (Selicrelumab) and Group 4 (Selicrelumab + Atezolizumab) were decreased significantly (nearly 10%), while the body weight of the mice in Group 3 (Atezolizumab) was normal. This suggested that Selicrelumab had side effects and toxicity that significantly cause weight loss in mice. The body weights of mice in PD-L1×CD40 bispecific antibody groups Group 5 (PR007070) and Group 6 (PR007151) were normal, indicating good safety.

The above results indicated that the PD-L1×CD40 bispecific antibodies PR007070 and PR007151 had significant anti-tumor activity and are significantly better than Atezolizumab. On the other hand, according to changes in mouse body weight, the safety of PD-L1 ×CD40 bispecific antibodies PR007070 and PR007151 was significantly better than that of Selicrelumab and the combination of Selicrelumab and Atezolizumab.

### Example 14.2. Mouse efficacy experiment #2

In the second experiment, when the average tumor volume reached 89 mm³, mice with a moderate tumor volume were picked, and grouped and administrated according to the schemes in Table 32.

**Table 32. Experimental protocol design and tumor inhibition rate of each administration group**

| **Group** | **Number of mice (N)** | **Drug, dosage** | **Administration frequency** | **Tumor inhibition rate (%)** |
|---|---|---|---|---|
| Group 1 | 7 | hIgG1, 10 mg/kg | BIW, 6 times | |
| Group 2 | 7 | PR007619, 12 mg/kg | BIW, 6 times | 82.73 |
| Group 3 | 7 | PR007619, 6 mg/kg | BIW, 6 times | 72.05 |
| Group 4 | 7 | PR007281, 12 mg/kg | BIW, 6 times | 68.75 |
| Group 5 | 7 | PR007281, 6 mg/kg | BIW, 6 times | 96.44 |
| Group 6 | 7 | PR007556, 12 mg/kg | BIW, 6 times | 81.97 |
| Group 7 | 7 | PR007556, 6 mg/kg | BIW, 6 times | 86.59 |
| Group 8 | 7 | PR007276, 12 mg/kg | BIW, 6 times | 88.57 |
| Group 9 | 7 | PR007276, 6 mg/kg | BIW, 6 times | 96.67 |

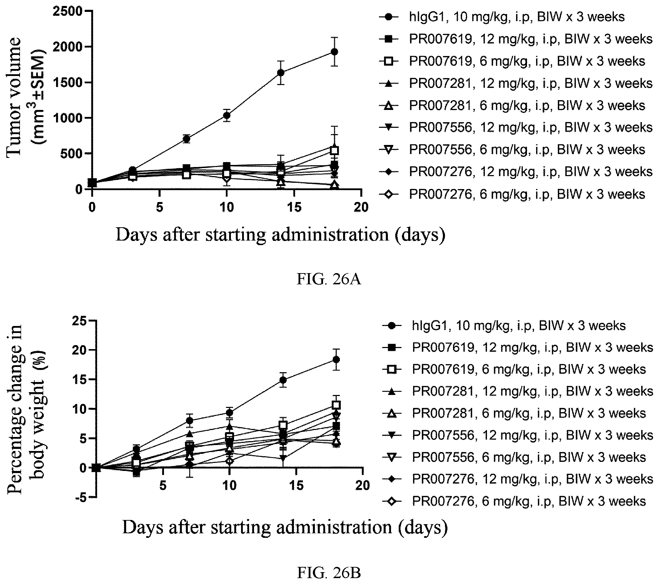

The results are shown in FIG. 26A and FIG. 26B. On day 18 after starting administration, the average tumor volume of the control group Group 1 (hIgG1, 10 mg/kg) was 1927.77 ± 200.44 mm³. The tumor volumes of Group 2 (PR007619, 12 mg/kg) and Group 3 (PR007619, 6 mg/kg) were 333.00 ± 103.06 mm³ and 538.85 ± 224.35 mm³, respectively, and the corresponding tumor inhibition rates were 82.73% and 72.05%, respectively. The tumor volumes of Group 4 (PR007281, 12 mg/kg) and Group 5 (PR007281, 6 mg/kg) were 602.40 ± 278.52 mm³ and 68.66 ± 11.68 mm³, respectively, and the corresponding tumor inhibition rates were 68.75% and 96.44%, respectively. The tumor volumes of Group 6 (PR007556, 12 mg/kg) and Group 7 (PR007556, 6 mg/kg) were 347.56 ± 167.80 mm³ and 258.51 ± 102.12 mm³, respectively, and the corresponding tumor inhibition rates were 81.97% and 86.59%, respectively. The tumor volumes of Group 8 (PR007276, 12 mg/kg) and Group 9 (PR007276, 6 mg/kg) were 200.34 ± 59.12 mm³ and 58.33 ± 42.67 mm³, respectively, and the corresponding tumor inhibition rates were 88.57% and 96.97%, respectively. During the whole experiment, the body weight of the mice in the administration group was normal, and the drug had no significant effect on the mice condition.

The above results indicated that PD-L1×CD40 bispecific antibodies PR007619, PR007281, PR007556, and PR007276 all exhibited excellent anti-tumor activity and safety.

### Example 14.3. Mouse efficacy experiment #3 and mouse liver toxicity study

In the third experiment, when the average tumor volume reached 85 mm³, mice with a moderate tumor volume were picked, and grouped and administrated according to the schemes in Table 33.

24 hours after the last administration, the serum of mice in each group (36 samples in total) was collected for blood biochemical indicator test. The levels of ALT and AST (alanine aminotransferase and aspartate aminotransferase) in mouse serum were detected. At the same time, liver tissues of 36 mice were collected and embedded in paraffin. Each liver tissue was serially sliced 6 times, and the sections were subjected to H&E staining. Finally, three stained sections were selected for scanning and microscopic analysis.

**Table 33. Experimental protocol design and tumor inhibition rate of each administration group**

| **Group** | **Number of mice (N)** | **Drug, dosage** | **Administration frequency** | **Tumor inhibition rate (%)** |
|---|---|---|---|---|
| Group 1 | 6 | hIgG1, 3.6 mg/kg | BIW, 6 times | |
| Group 2 | 6 | PR007619, 1.8 mg/kg | BIW, 6 times | 36.86 |
| Group 3 | 6 | PR007619, 3.6 mg/kg | BIW, 6 times | 55.55 |
| Group 4 | 6 | PR007619, 6.0 mg/kg | BIW, 6 times | 62.00 |
| Group 5 | 6 | PR007619, 10.8 mg/kg | BIW, 6 times | 70.94 |
| Group 6 | 6 | Selicrelumab, 5 mg/kg | BIW, 6 times | 81.79 |

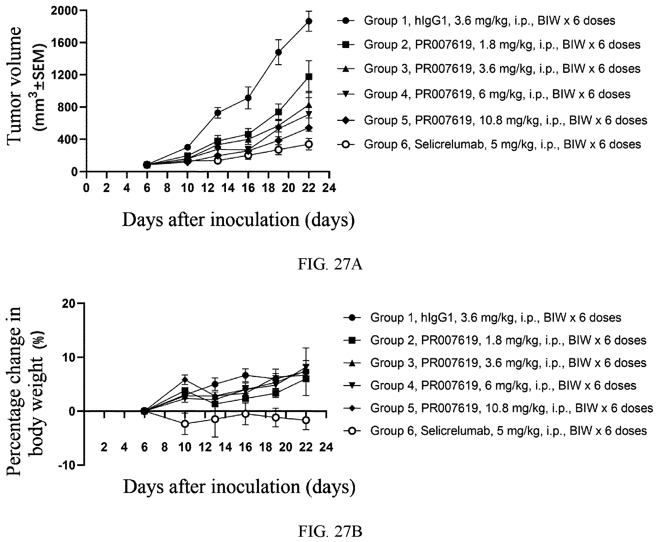

The anti-tumor efficacy results are shown in FIG. 27A and FIG. 27B. On day 22 after starting administration, the tumor volume of the control group Group 1 (hIgG1, 3.6 mg/kg) was 1865.24 ± 125.47 mm³. The tumor volumes of Group 2 (PR007619, 1.8 mg/kg), Group 3 (PR007619, 3.6 mg/kg), Group 4 (PR007619, 6 mg/kg), and Group 5 (PR007619, 10.8 mg/kg) were 1177.74 ± 198.47 mm³, 829.01 ± 164.87 mm³, 708.84 ± 208.76 mm³ and 541.99 ± 41.2 mm³, respectively; and the corresponding tumor inhibition rates were 36.86%, 55.55%, 62% and 70.94%, respectively. The tumor volume of Group 6 (Selicrelumab, 5 mg/kg) was 339.68 ± 72.9 mm³ and the corresponding tumor inhibition rate was 81.79%. During the whole experiment, the body weights of the mice in the hIgG1 control group and the PR007619 administration group were normal, and the drug had no significant effect on the mice condition. However, the mice in the Selicrelumab administration group experienced a certain degree of weight loss, suggesting that Selicrelumab had certain side effects and toxicity.

The above results indicated that the PD-L1×CD40 bispecific antibody PR007619 had dose-dependent tumor inhibitory activity, and the inhibitory effect was significant. Moreover, PR007619 had a better safety than the control molecule Selicrelumab.

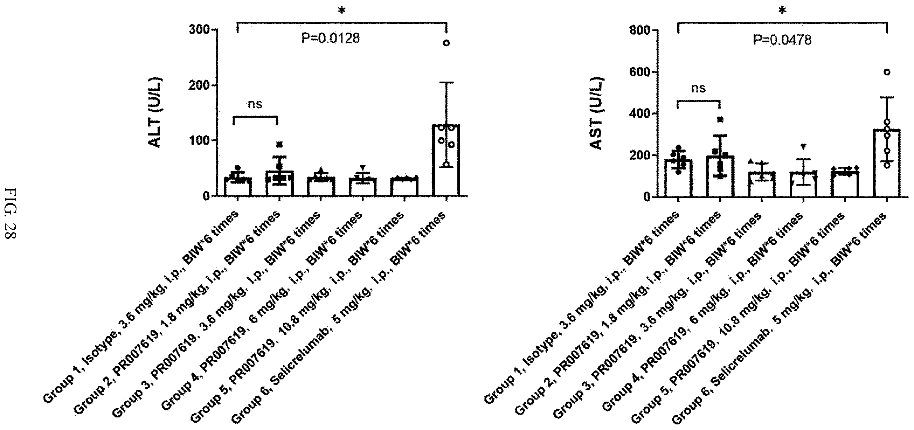

The results of blood biochemical indicator test are shown in FIG. 28. The corresponding average ALT values of Group 1 - Group 6 were 34 ± 8.83 U/L, 46 ± 24.64 U/L, 34.5 ± 7.04 U/L, 32.5 ± 9.38 U/L, 31.5 ± 1.64 U/L and 128.7 ± 76.2 U/L, respectively. In particular, the value of Group 6 (Selicrelumab, 5 mg/kg) had a significant increase compared with that of Group 1 (control group) (P=0.0128); There was no significant difference between the values of Group 2 - Group 5 (administration groups with different doses of PR007619) and Group 1 (control group). Similarly, the corresponding average AST values of Group 1 - Group 6 were 180 ± 40.3 U/L, 197.5 ± 95.9 U/L, 120.5 ± 41.2 U/L, 120 ± 61.3 U/L, 123.5 ± 16.69 U/L and 326.17±153.6 U/L, respectively. In particular, the value of Group 6 (Selicrelumab, 5 mg/kg) had a significant increase compared with that of Group 1 (control group) (P = 0.0478); There was no significant difference between the values of Group 2 - Group 5 (administration groups with different doses of PR007619) and Group 1 (control group).

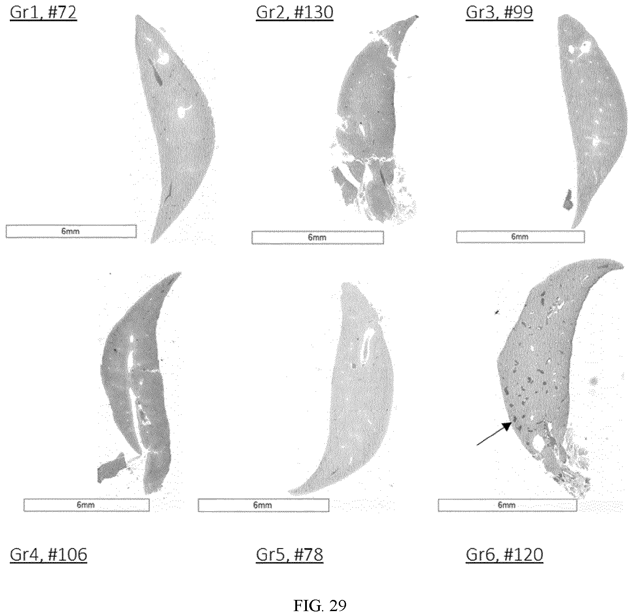

The results of tissue sections are shown in FIG. 29. The liver tissue of mice in Group 6 (Selicrelumab, 5 mg/kg) showed obvious damages, specifically such as moderate multifocal infiltration of mononuclear cells and lymphocytes in the portal vein; moderate multifocal Kupffer cell proliferation in sinusoid; significant mononuclear cell proliferation and dispersion in sinusoid; and significant multifocal hepatocellular degeneration and necrosis. However, no obvious pathological changes were observed in the liver tissues of mice in Group 1 (control group) and Group 2 - Group 5 (administration groups with different doses of PR007619).

The above blood biochemical indicators and tissue section results indicated that the CD40 monoclonal antibody Selicrelumab caused significant acute hepatotoxicity in hPD1×hCD40-C57BL/6J transgenic mice. The PD-L1×CD40 bispecific antibody PR007619 showed excellent safety.

### Example 14.4. Mouse efficacy experiment #4 and mouse liver toxicity study

The fourth experiment aimed to study the effect of PD-L1×CD40 bispecific antibody on mice, including the inhibition of tumor growth, changes in mouse body weight, changes in serum cytokine levels, and changes in blood biochemical indicators of hepatotoxicity.

In the fourth experiment, when the average tumor volume reached 82 mm³, mice with a moderate tumor volume were picked, and grouped and administrated according to the schemes in Table 34. 10 mice in each group (5 female mice and 5 male mice) were administered from the day of grouping, twice a week (BIW), for a total of 4 times (days 0, 3, 7, and 10), by intraperitoneal injection. Tumor volumes were measured during the period (days 0, 2, 5, 9, and 11). 24 hours after the first administration, serum was collected for testing cytokine (IL-6, TNF-α, IFN-γ and IL12p40) levels. 24 hours after the last administration, serum was collected for blood biochemical tests of hepatotoxicity indicators (ALT and AST). Finally, liver tissues of mice were collected (50 samples in total) and embedded in paraffin. Each liver tissue was serially sliced 6 times, and the sections were subjected to H&E staining. Finally, three stained sections were selected for scanning and microscopic analysis. In addition to the liver, the heart, spleen, lung, and kidney of the mice were collected, embedded in paraffin and subjected to section staining analysis.

**Table 34. Experimental protocol design and tumor inhibition rate of each administration group**

| **Group** | **Number of mice (N)** | **Drug, dosage** | **Administration frequency** | **Tumor inhibition rate (%)** |
|---|---|---|---|---|
| Group 1 | 10 (5F, 5M) | hIgG1, 10 mg/kg | BIW, 4 times | |
| Group 2 | 10 (5F, 5M) | PR007619, 12 mg/kg | BIW, 4 times | 66.56 |
| Group 3 | 10 (5F, 5M) | Selicrelumab, 1 mg/kg | BIW, 4 times | 67.21 |
| Group 4 | 10 (5F, 5M) | Selicrelumab, 2.5 mg/kg | BIW, 4 times | 78.80 |
| Group 5 | 10 (5F, 5M) | Selicrelumab, 5 mg/kg | BIW, 4 times | 83.51 |

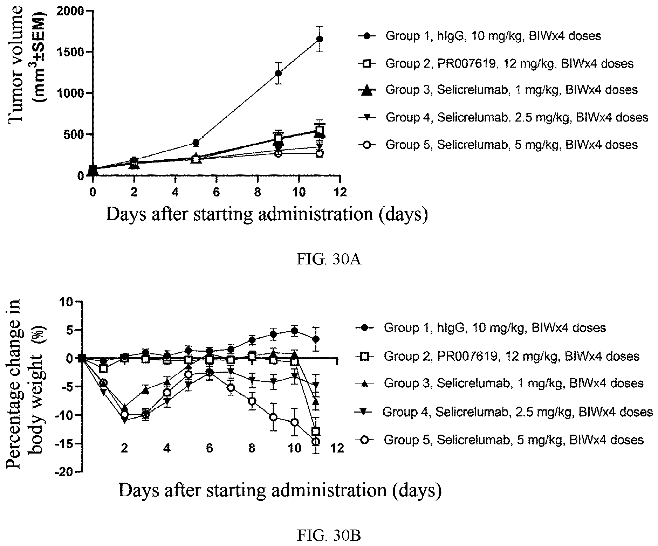

The anti-tumor efficacy results are shown in FIG. 30A and FIG. 30B. On day 11 after starting administration, the average tumor volume of the control group Group 1 (hIgG1, 10 mg/kg) was 1653.68 ± 153.10 mm³. The tumor volumes of Group 2 (PR007619, 12 mg/kg), Group 3 (Selicrelumab, 1 mg/kg), Group 4 (Selicrelumab, 2.5 mg/kg), and Group 5 (Selicrelumab, 5 mg/kg) were 552.96 ± 123.59 mm³, 542.23 ± 79.83 mm³, 350.60 ± 61.35 mm³ and 272.65 ± 45.48 mm³, respectively, and the corresponding tumor inhibition rates were 66.56%, 67.21%, 78.80% and 83.51%, respectively. There were statistical differences in each administration group compared with Group 1 (P > 0.05). On the other hand, the body weight results showed that Group 2 (PR007619, 12 mg/kg) had no significant effect on the body weight of mice. Each dose group of Selicrelumab (Groups 3-5) caused body weight loss in mice after administration, with the decrease being close to or exceeding 10%, and the larger the dose, the more difficult it was to restore body weight. In particular, the body weight has never been restored after administration in medium and high dose groups of Selicrelumab.

The above results indicated that the PD-L1×CD40 bispecific antibody had significant anti-tumor efficacy and safety, while Selicrelumab caused body weight loss in mice and exhibited significant toxic side effects.

**Table 35. Levels of blood biochemical indicators ALT and AST**

| | **Group 1** | **Group 2** | **Group 3** | **Group 4** | **Group 5** |
|---|---|---|---|---|---|
| **ALT (U/L)** | 168.3±79.8 | 107.6±54.2 | 105.1±44.8 | 310±189.3 | 507.6±150.0 |
| **AST (U/L)** | 51.6±69.1 | 19.7±7.26 | 31±19.1/L | 96.5±86.1 | 147.7±73.1 |

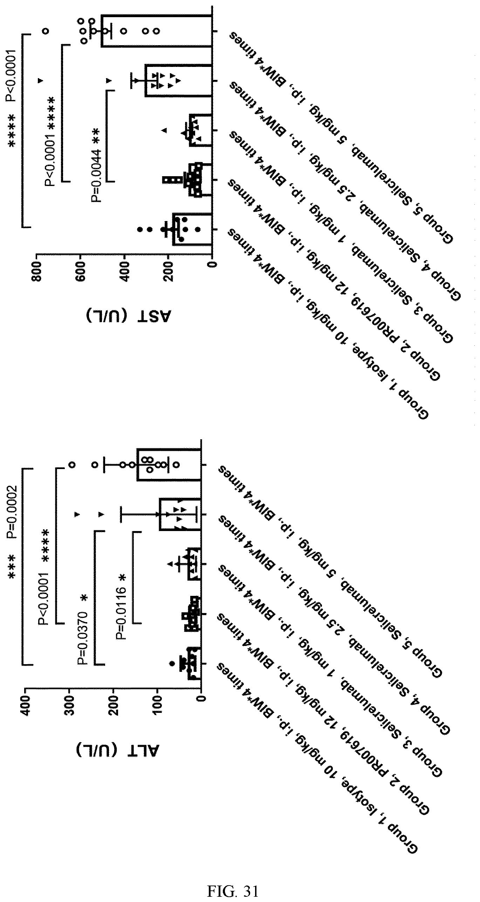

The results of blood biochemical indicator test are shown in FIG. 31 and Table 35. The corresponding ALT values of Group 1 - Group 5 were 168.3 ± 79.8 U/L, 107.6 ± 54.2 U/L, 105.1 ± 44.8 U/L, 310 ± 189.3 U/L, and 507.6 ± 150.0 U/L, respectively; the corresponding AST values of Group 1 - Group 5 were 51.6 ± 69.1 U/L, 19.7 ± 7.26 U/L, 31 ± 19.1/L, 96.5 ± 86.1 U/L and 147.7 ± 73.1 U/L, respectively. In particular, the ALT and AST of Group 5 (Selicrelumab, 5 mg/kg) had an extremely significant increase compared with that of Group 1 control group (P<0.001); and the ALT of Group 4 (Selicrelumab, 2.5 mg/kg) also had a significant difference compared with that of Group 1 control group (P = 0.037). However, there was no significant difference in ALT and AST between Group 2 (PR007619, 12 mg/kg) and Group 1 control group. This indicated that Selicrelumab caused a significant increase in the blood biochemical indicators of hepatotoxicity, ALT and AST, while the PD-L1×CD40 bispecific antibody did not cause an increase in ALT and AST levels in the serum of mice.

**Table 36. Peripheral blood cytokine level**

| | **IL-6 (pg/mL)** | **IFN-γ (pg/mL)** | **TNF-α (pg/mL)** | **IL12p40 (pg/mL)** |
|---|---|---|---|---|
| **Group 1** | 16.3 ± 8.74 | 0.54 ± 0.19 | 14.1 ± 3.79 | 1157.1 ± 125.8 |
| **Group 2** | 24.8 ± 11.7 | 1.48 ± 0.98 | 17.3 ± 8.08 | 1786.8 ± 369.5 |
| **Group 3** | 1343.8 ± 529.5 | 159.0 ± 90.9 | 1186.1 ± 255.7 | 92119.7 ± 30959.2 |
| **Group 4** | 1428.2 ± 569.5 | 138.9 ± 91.7 | 1274.7 ± 392.3 | 99263.0 ± 24254.9 |
| **Group 5** | 2038.0 ± 747.2 | 147.4 ± 79.44 | 1311.5 ± 179.9 | 110786.1 ± 21607.5 |

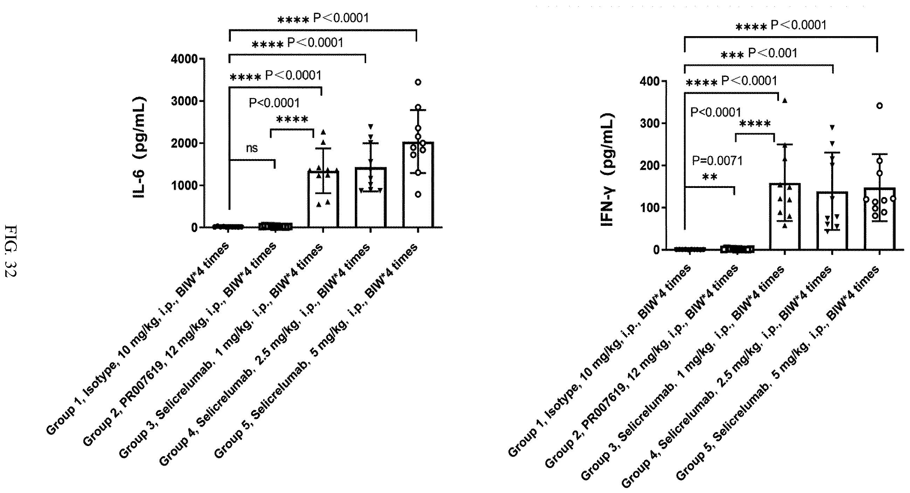

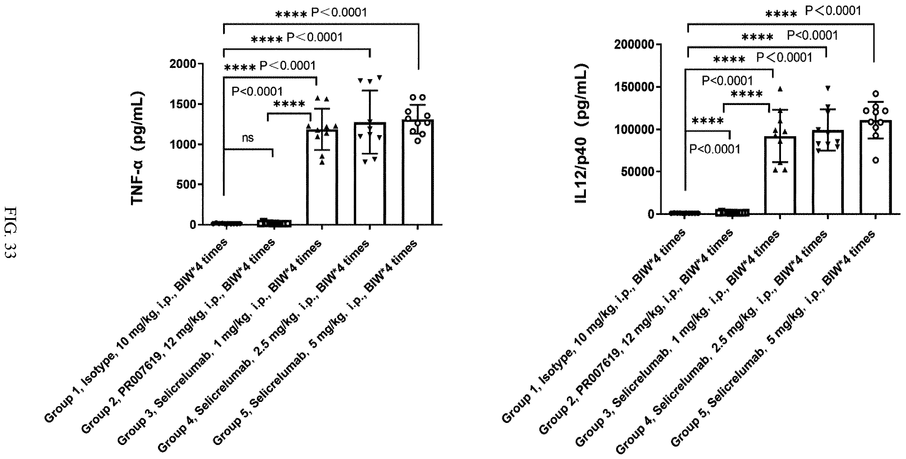

The results of peripheral blood cytokine test are shown in FIG. 32, FIG. 33 and Table 36. The corresponding cytokine IL-6 values of Group 1 - Group 5 were 16.3 ± 8.74 pg/mL, 24.8 ± 11.7 pg/mL, 1343.8 ± 529.5 pg/mL, 1428.2 ± 569.5 pg/mL, and 2038.0 ± 747.2 pg/mL, respectively. The corresponding cytokine IFN-γ values of Group 1 - Group 5 were 0.54 ± 0.19 pg/mL, 1.48 ± 0.98 pg/mL, 159.0 ± 90.9 pg/mL, 138.9 ± 91.7 pg/mL, and 147.4 ± 79.44 pg/mL, respectively. The corresponding cytokine TNF-α values of Group 1 - Group 5 were 14.1 ± 3.79 pg/mL, 17.3 ± 8.08 pg/mL, 1186.1 ± 255.7 pg/mL, 1274.7 ± 392.3 pg/mL and 1311.5 ± 179.9 pg/mL, respectively. The corresponding cytokine IL12p40 values of Group 1 - Group 5 were 1157.1 ± 125.8 pg/mL, 1786.8 ± 369.5 pg/mL, 92119.7 ± 30959.2 pg/mL, 99263.0 ± 24254.9 pg/mL, and 110786.1 ± 21607.5 pg/mL, respectively.

It can be seen that in terms of the levels of cytokines IL-6, TNF-α and IFN-γ, each dose group of Selicrelumab (Group 3 - 5) could cause a significant increase in cytokine levels, and had an extremely significant difference compared with the Group 1 control group (P < 0.0001), however, the PD-L1×CD40 bispecific antibody (Group 2) group did not cause an increase in cytokine levels, and did not have significant difference compared with the Group 1 control group (P > 0.05). In addition, in terms of the level of cytokine IL12p40, each dose group of Selicrelumab (Group 3 - 5) could cause a significant increase in IL12p40 level, and had an extremely significant difference compared with the Group 1 control group (P<0.0001), however, PD-L1×CD40 bispecific antibody (Group 2) only caused a slight increase in IL12p40 (P = 0.0004). The comprehensive results indicated that Selicrelumab caused significant peripheral blood cytokine release syndrome in mice, while PD-L1×CD40 bispecific antibody did not.

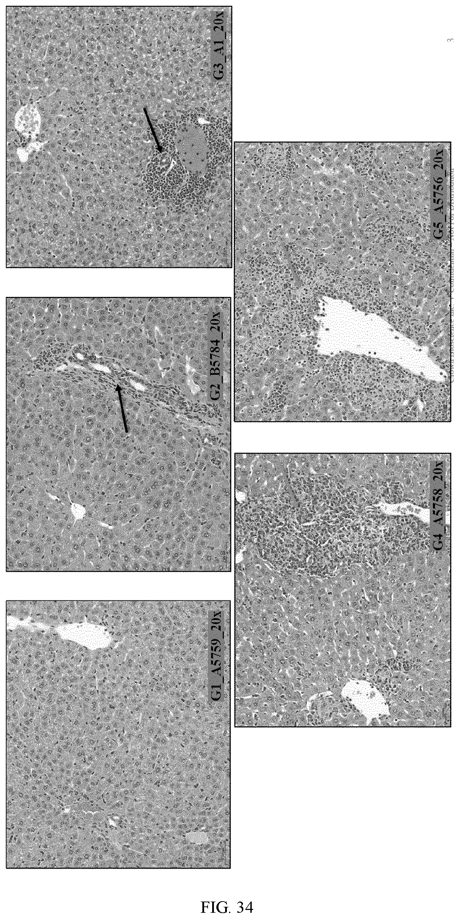

The results of liver tissue sections are shown in FIG. 34. In Group 1, the livers of all 10 animals were within the normal range. In Group 2, only one animal, B5784, had a small amount of multifocal perivascular lymphocytic infiltration, and the livers of the other 9 animals were within normal range. In Group 3, the livers of all 10 animals had minimal, mild, or mildest multifocal perivascular lymphocytic infiltration. In Group 4, the liver of animal A5757 had moderate multifocal perivascular lymphocytic infiltration, and the livers of other animals had moderate multifocal granulomatous inflammation (mononuclear cell infiltration with fibrosis, mostly lymphocytes and macrophages) around the blood vessels or in venous sinuses. In Group 5, the livers of all animals had moderate and significant multifocal granulomatous inflammation (mononuclear cell infiltration with fibrosis, mostly lymphocytes and macrophages) around the blood vessels or in venous sinuses.

In this study, the changes in the liver, kidney, lung, and spleen when administrating the PD-L1×CD40 bispecific antibody (Group 2) were minimal or mild compared with that of the control group (Group 1), and minimal lymphocyte infiltration was a predictive pharmacodynamic response. There were more adverse changes in these tissues in each dose group of Selicrelumab (Group 3, Group 4, and Group 5) compared with the control group (Group 1). These tissues had tubular degeneration, increased lymphocytic infiltration, and/or inflammation. Predictable pharmacodynamic responses were amplified, which may be harmful to animals. Therefore, the degree and incidence of lesions caused by PD-L1×CD40 bispecific antibody were smaller compared with that of Selicrelumab.

In summary, Selicrelumab caused severe cytokine release syndrome, increased serum indicators of hepatotoxicity, and damage to liver and other organ tissues in mice. The PD-L1×CD40 bispecific antibody had a strong anti-tumor efficacy and excellent safety, and did not cause cytokine release syndrome, increased serum indicators of hepatotoxicity, and damage to liver and other organ tissues in mice.

### Example 15. Pharmacokinetic evaluation of PD-L1×CD40

In this example, the pharmacokinetic properties of four bispecific binding protein molecules with similar sequences and identical structures in mice were studied.

Administration and blood collection: For each test binding protein molecule, 6 female C57BL/6J mice, weighing 18-22 grams, were selected and administered with the test binding protein molecule at a dose of 6 mg/kg by intravenous injection. Whole blood was collected from one group of 3 mice at 5 minutes, 24 hours (day 1), day 4, and day 10 after administration, and whole blood was collected from another group of 3 mice before administration and at 5 hours, day 2, day 7, and day 14 after administration. Whole blood was left to stand to clot, and then centrifuged. The separated serum samples were frozen at -80°C until analysis.

Analytical method: Two ELISA methods were used to quantify drug concentrations in mouse serum. ELISA method 1, i.e., Fc-end detection method: goat anti-human Fc polyclonal antibody (ROCKLAND, #609-101-017) coated on a 96-well plate was used to capture an antibody containing human Fc in mouse serum, and then HRP-labeled goat anti-human Fc secondary antibody (Jackson, #109-035-098) was added for detection. ELISA method 2, i.e., the functional domain detection method: the PD-L1 antigen protein (Novoprotein, #C315) coated on a 96-well plate was used to capture an antibody specifically recognizing the antigen in mouse serum, and then HRP-labeled goat anti-human Fc secondary antibody (Jackson, #109-035-098) was added for detection. Phoenix WinNonlin version 8.2 software was used to analyze the pharmacokinetic parameters using the noncompartmental analysis (NCA).

As shown in Table 37, the bispecific antibody molecule PR007556 had a serum half-life t1/2 value similar to that of conventional IgG antibody, and the PD-L1 end detection method showed that the t1/2 value thereof was 10.3 days. The bispecific antibodies PR007619, PR007281 and PR007276 had similar pharmacokinetics to conventional IgG antibody. The Fc-end detection method showed that serum half-life t_{1/2} values of PR007619, PR007281 and PR007276 in mice were about 13.6 days, 15.1 days, and 15.7 days, respectively. The PD-L1 detection method showed that serum half-life t1/2 values of PR007619, PR007281 and PR007276 in mice were about 13.5 days, 18.1 days, and 13.7 days, respectively.

**Table 37. Pharmacokinetic parameters of PD-L1 ×CD40 in mice**

| Bispecific antibody molecule | PR007619 | | PR007556 | | PR007281 | | PR007276 | |
|---|---|---|---|---|---|---|---|---|
| Animal (number) | C57BL/6J mice (n=6) | | C57BL/6J mice (n=6) | | C57BL/6J mice (n=6) | | C57BL/6J mice (n=6) | |
| Antibody dosage | 6 mg/kg, I.V. | | 6 mg/kg, I.V. | | 6 mg/kg, I.V. | | 6 mg/kg, I.V. | |
| PK parameters | Fc-end detection | PD-L1-end detection | Fc-end detection | PD-L1-end detection | Fc-end detection | PD-L1-end detection | Fc-end detection | PD-L1-end detection |
| T_{1/2} (day) | 13.6 | 13.5 | 20.3 | 10.3 | 15.1 | 18.1 | 15.7 | 13.7 |
| V_{d} (mL/kg) | 125 | 137 | 100 | 93.6 | 113 | 119 | 108 | 105 |
| AUC(µg*hour/mL ) | 11600 | 10500 | 15800 | 13700 | 12900 | 12700 | 13700 | 13600 |
| Cl (mL/hour/kg) | 0.269 | 0.298 | 0.143 | 0.258 | 0.213 | 0.186 | 0.197 | 0.223 |
| C₀ (µg/mL) | 99.9 | 97.5 | 130 | 143 | 138 | 132 | 140 | 140 |

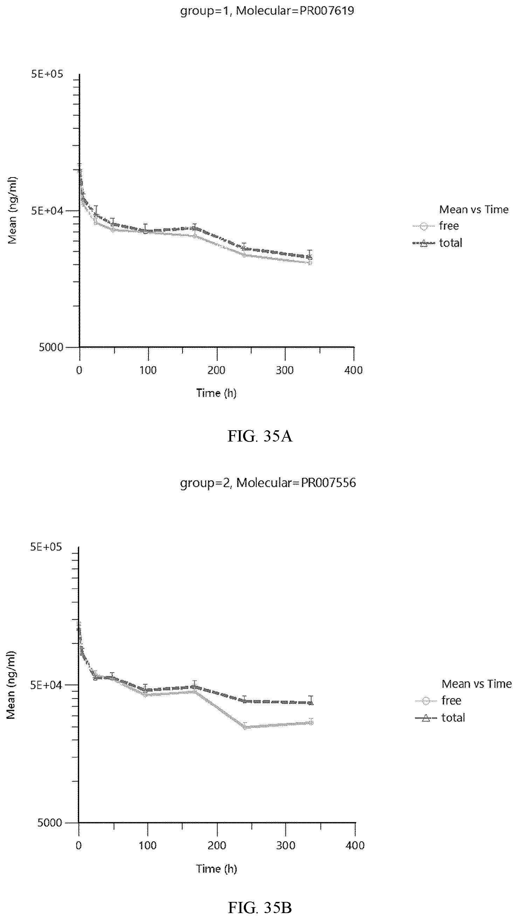

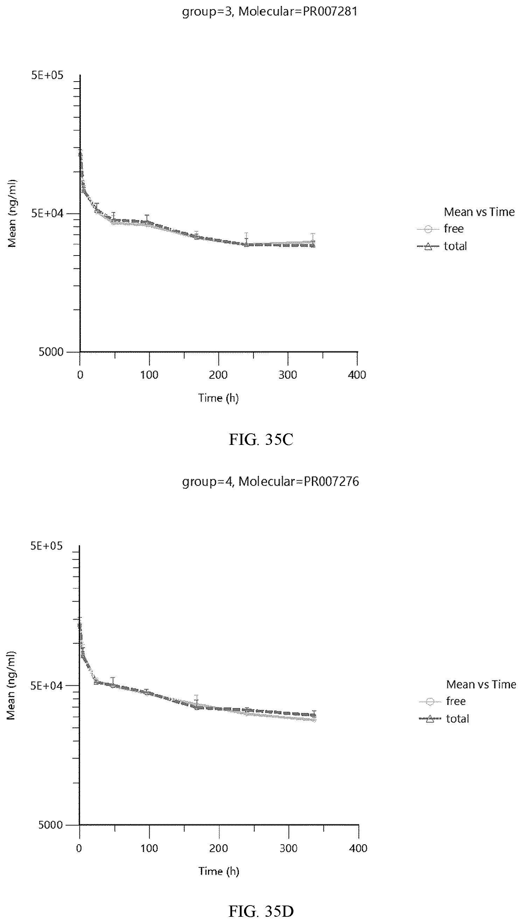

The results are shown in Table 37 and FIG. 35A-FIG. 35D. These PD-L1×CD40 bispecific antibody molecules had serum half-life t1/2 values similar to that of conventional IgG antibody. Depending on the detection method, the T_{1/2} values thereof were 10 days to 20 days.

Although the specific embodiments of the present invention have been described above, it will be understood by those skilled in the art that these are merely illustrative, and that various alterations or modifications can be made to these embodiments without departing from the principle and essence of the present invention. Therefore, the scope of protection of the present invention is defined by the appended claims.

## Claims

1. An antigen-binding protein targeting PD-L1 and CD40, comprising a first protein functional region and a second protein functional region, **characterized in that** the first protein functional region comprises an antigen-binding protein targeting CD40, and the second protein functional region comprises an antigen-binding protein targeting PD-L1, wherein
the antigen-binding protein targeting CD40 comprises a light chain variable region (VL) comprising LCDR1, LCDR2, and LCDR3 and a heavy chain variable region (VH) comprising HCDR1, HCDR2, and HCDR3, the LCDR1 comprises the amino acid sequence set forth in SEQ ID NO: 38, the LCDR2 comprises the amino acid sequence set forth in SEQ ID NO: 43, the LCDR3 comprises the amino acid sequence set forth in SEQ ID NO: 48 or a variant 3 with 3, 2 or 1 amino acid mutation in SEQ ID NO: 48, the HCDR1 comprises the amino acid sequence set forth in SEQ ID NO: 8, the HCDR2 comprises the amino acid sequence set forth in SEQ ID NO: 18, and the HCDR3 comprises the amino acid sequence set forth in SEQ ID NO: 27.

2. The antigen-binding protein targeting PD-L1 and CD40 of claim 1, **characterized in that** the variant 3 comprises an amino acid sequence having a PTM site mutation in the amino acid sequence set forth in SEQ ID NO: 48, preferably comprises an amino acid sequence having an amino acid mutation at position 4 and/or position 5 of the amino acid sequence set forth in SEQ ID NO: 48, the amino acid mutation is preferably an amino acid substitution, more preferably a conservative amino acid substitution; preferably, the variant 3 is an amino acid sequence having an N4A/F/Y/V/N and/or S5N mutation in the amino acid sequence set forth in SEQ ID NO: 48; more preferably, the variant 3 is an amino acid sequence set forth in any one of SEQ ID NOs: 49-53; further more preferably, in the antigen-binding protein targeting CD40, the LCDR1 comprises the amino acid sequence set forth in SEQ ID NO: 38, the LCDR2 comprises the amino acid sequence set forth in SEQ ID NO: 43, the LCDR3 comprises the amino acid sequence set forth in any one of SEQ ID NOs: 48-53; the HCDR1 comprises the amino acid sequence set forth in SEQ ID NO: 8, the HCDR2 comprises the amino acid sequence set forth in SEQ ID NO: 18, and the HCDR3 comprises the amino acid sequence set forth in SEQ ID NO: 27.

3. The antigen-binding protein targeting PD-L1 and CD40 of claim 1 or 2, **characterized in that** in the antigen-binding protein targeting CD40, the VL comprises the amino acid sequence set forth in SEQ ID NO: 68 or an amino acid sequence having at least 80%, 85%, 90%, 92%, 94%, 95%, 96%, 97%, 98% or 99% identity to SEQ ID NO: 68, and the VH comprises the amino acid sequence set forth in SEQ ID NO: 60 or an amino acid sequence having at least 80%, 85%, 90%, 92%, 94%, 95%, 96%, 97%, 98% or 99% identity to SEQ ID NO: 60; preferably, the VL comprises the amino acid sequence set forth in any one of SEQ ID NOs: 68-73, and the VH comprises the amino acid sequence set forth in SEQ ID NO: 60.

4. The antigen-binding protein targeting PD-L1 and CD40 of any one of claims 1-3, **characterized in that** the antigen-binding protein targeting CD40 is a full-length antibody comprising a light chain and a heavy chain, the light chain comprises a light chain constant region (CL), preferably the light chain constant region is a light chain constant region of human antibody, more preferably the light chain constant region of the human antibody is a light chain constant region of κ subtype, the heavy chain comprises a heavy chain constant region (CH), and the heavy chain constant region is preferably a heavy chain constant region of human antibody, more preferably a heavy chain constant region of hIgG1, hIgG2, hIgG3 or hIgG4 subtype, and further preferably a heavy chain constant region of hIgG1 subtype;
preferably, the antigen-binding protein targeting CD40 is a full-length antibody comprising a light chain and a heavy chain, the light chain comprises the amino acid sequence set forth in any one of SEQ ID NOs: 87-92, and the heavy chain comprises the amino acid sequence set forth in SEQ ID NO: 78 or 84, more preferably, the light chain comprises the amino acid sequence set forth in SEQ ID NO: 87, and the heavy chain comprises the amino acid sequence set forth in SEQ ID NO: 78, or, the light chain comprises the amino acid sequence set forth in any one of SEQ ID NOs: 88-92, and the heavy chain comprises the amino acid sequence set forth in SEQ ID NO: 84.

5. The antigen-binding protein targeting PD-L1 and CD40 of any one of claims 1-4, **characterized in that** the antigen-binding protein targeting PD-L1 comprises a heavy chain variable region (VH) comprising HCDR1, HCDR2, and HCDR3, the HCDR1 comprises the amino acid sequence set forth in SEQ ID NO: 5 or a variant 7 with 3, 2 or 1 amino acid mutation in SEQ ID NO: 5, the HCDR2 comprises the amino acid sequence set forth in SEQ ID NO: 16 or a variant 8 with 3, 2 or 1 amino acid mutation in SEQ ID NO: 16, and the HCDR3 comprises the amino acid sequence set forth in SEQ ID NO: 25 or a variant 9 with 3, 2 or 1 amino acid mutation in SEQ ID NO: 25; preferably, the variant 7 is an amino acid sequence having an amino acid mutation at position 3 and/or 6 of SEQ ID NO: 5, the variant 8 is an amino acid sequence having an amino acid mutation at position 1, 3 and/or 6 of SEQ ID NO: 16, and the variant 9 is an amino acid sequence having an amino acid mutation at position 4, 8 and/or 11 of SEQ ID NO: 25, preferably the amino acid mutation is an amino acid substitution, more preferably a conservative amino acid substitution; more preferably, the variant 7 is an amino acid sequence having N3T/D and/or N5S mutation in SEQ ID NO: 5, the variant 8 is an amino acid sequence having W1R, D3T and/or K5E mutation in SEQ ID NO: 16, and the variant 9 is an amino acid sequence having I4L, V8I and/or A11D mutation in SEQ ID NO: 25; further more preferably, the variant 7 is an amino acid sequence set forth in SEQ ID NO: 7 or 9, the variant 8 is an amino acid sequence set forth in SEQ ID NO: 19, and the variant 9 is an amino acid sequence set forth in SEQ ID NO: 28, 29 or 30.

6. The antigen-binding protein targeting PD-L1 and CD40 of claim 5, **characterized in that** in the antigen-binding protein targeting PD-L1, the HCDR1 comprises the amino acid sequence set forth in SEQ ID NO: 5, 7 or 9, the HCDR2 comprises the amino acid sequence set forth in SEQ ID NO: 16 or 19, and the HCDR3 comprises the amino acid sequence set forth in SEQ ID NO: 25, 28, 29 or 30; preferably, in the antigen-binding protein targeting PD-L1, the HCDR1 comprises the amino acid sequence set forth in SEQ ID NO: 5, the HCDR2 comprises the amino acid sequence set forth in SEQ ID NO: 16, and the HCDR3 comprises the amino acid sequence set forth in SEQ ID NO: 25; or, the HCDR1 comprises the amino acid sequence set forth in SEQ ID NO: 7, the HCDR2 comprises the amino acid sequence set forth in SEQ ID NO: 16, and the HCDR3 comprises the amino acid sequence set forth in SEQ ID NO: 25; or, the HCDR1 comprises the amino acid sequence set forth in SEQ ID NO: 7, the HCDR2 comprises the amino acid sequence set forth in SEQ ID NO: 19, and the HCDR3 comprises the amino acid sequence set forth in SEQ ID NO: 28; or, the HCDR1 comprises the amino acid sequence set forth in SEQ ID NO: 9, the HCDR2 comprises the amino acid sequence set forth in SEQ ID NO: 19, and the HCDR3 comprises the amino acid sequence set forth in SEQ ID NO: 25; or, the HCDR1 comprises the amino acid sequence set forth in SEQ ID NO: 9, the HCDR2 comprises the amino acid sequence set forth in SEQ ID NO: 19, and the HCDR3 comprises the amino acid sequence set forth in SEQ ID NO: 29; or, the HCDR1 comprises the amino acid sequence set forth in SEQ ID NO: 9, the HCDR2 comprises the amino acid sequence set forth in SEQ ID NO: 19, and the HCDR3 comprises the amino acid sequence set forth in SEQ ID NO: 30; or, the HCDR1 comprises the amino acid sequence set forth in SEQ ID NO: 9, the HCDR2 comprises the amino acid sequence set forth in SEQ ID NO: 19, and the HCDR3 comprises the amino acid sequence set forth in SEQ ID NO: 28.

7. The antigen-binding protein targeting PD-L1 and CD40 of claim 5 or 6, **characterized in that** in the antigen-binding protein targeting PD-L1, the VH comprises the amino acid sequence set forth in SEQ ID NO: 57 or an amino acid sequence having at least 85%, 90%, 92%, 94%, 95%, 96%, 97%, 98% or 99% identity to SEQ ID NO: 57; preferably, the VH comprises the amino acid sequence set forth in any one of SEQ ID NOs: 57-65.

8. The antigen-binding protein targeting PD-L1 and CD40 of any one of claims 1-7, **characterized in that** the first protein functional region is an immunoglobulin comprising the antigen-binding protein targeting CD40, and the second protein functional region comprises one, two or more VHs of the antigen-binding protein targeting PD-L1;
preferably, the VHs of the antigen-binding protein targeting PD-L1 are directly linked to the immunoglobulin; or, the VHs are linked to the immunoglobulin via a linker; or, when the number of the VHs is greater than 1, each of the VHs can be linked to the immunoglobulin directly or via a linker, respectively;
more preferably, when the VHs are linked to the immunoglobulin via a linker, the linker is selected from any one of the amino acid sequences set forth in GS, GGS and SEQ ID NOs: 100-106.

9. The antigen-binding protein targeting PD-L1 and CD40 of claim 8, **characterized in that** the second protein functional region comprises two VHs of the antigen-binding protein targeting PD-L1; preferably, the two VHs comprised in the second protein functional region are identical; and/or, the C-termini of the two VHs are linked to the N-termini of the two heavy chains in the immunoglobulin via a linker set forth in GGS or SEQ ID NO: 5, respectively.

10. The antigen-binding protein targeting PD-L1 and CD40 of any one of claims 1-9, **characterized in that** the first protein functional region comprises two light chains comprising the amino acid sequence set forth in any one of SEQ ID NOs: 88-92, and two heavy chains comprising the amino acid sequence set forth in SEQ ID NO: 84, the second protein functional region comprises two VHs comprising the amino acid sequence set forth in any one of SEQ ID NOs: 79-83, and the C-termini of the two VHs are linked to the N-termini of the two heavy chains in the first protein functional region via a linker set forth in GGS or SEQ ID NO: 5, respectively;
Preferably, the antigen-binding protein targeting PD-L1 and CD40 comprises a first polypeptide chain and a second polypeptide chain as shown below:
a second polypeptide chain consisting of the amino acid sequence set forth in SEQ ID NO: 89, and a first polypeptide chain consisting of the amino acid sequence set forth in SEQ ID NO: 93; or, a second polypeptide chain consisting of the amino acid sequence set forth in SEQ ID NO: 88, and a first polypeptide chain consisting of the amino acid sequence set forth in SEQ ID NO: 94; or, a second polypeptide chain consisting of the amino acid sequence set forth in SEQ ID NO: 91, and a first polypeptide chain consisting of the amino acid sequence set forth in SEQ ID NO: 94; or, a second polypeptide chain consisting of the amino acid sequence set forth in SEQ ID NO: 90, and a first polypeptide chain consisting of the amino acid sequence set forth in SEQ ID NO: 93; or, a second polypeptide chain consisting of the amino acid sequence set forth in SEQ ID NO: 92, and a first polypeptide chain consisting of the amino acid sequence set forth in SEQ ID NO: 93; or, a second polypeptide chain consisting of the amino acid sequence set forth in SEQ ID NO: 88, and a first polypeptide chain consisting of the amino acid sequence set forth in SEQ ID NO: 95; or, a second polypeptide chain consisting of the amino acid sequence set forth in SEQ ID NO: 88, and a first polypeptide chain consisting of the amino acid sequence set forth in SEQ ID NO: 96; or, a second polypeptide chain consisting of the amino acid sequence set forth in SEQ ID NO: 88, and a first polypeptide chain consisting of the amino acid sequence set forth in SEQ ID NO: 97; or, a second polypeptide chain consisting of the amino acid sequence set forth in SEQ ID NO: 91, and a first polypeptide chain consisting of the amino acid sequence set forth in SEQ ID NO: 95; or, a second polypeptide chain consisting of the amino acid sequence set forth in SEQ ID NO: 91, and a first polypeptide chain consisting of the amino acid sequence set forth in SEQ ID NO: 93; or, a second polypeptide chain consisting of the amino acid sequence set forth in SEQ ID NO: 91, and a first polypeptide chain consisting of the amino acid sequence set forth in SEQ ID NO: 96; or, a second polypeptide chain consisting of the amino acid sequence set forth in SEQ ID NO: 89, and a first polypeptide chain consisting of the amino acid sequence set forth in SEQ ID NO: 97; or, a second polypeptide chain consisting of the amino acid sequence set forth in SEQ ID NO: 90, and a first polypeptide chain consisting of the amino acid sequence set forth in SEQ ID NO: 97; or, a second polypeptide chain consisting of the amino acid sequence set forth in SEQ ID NO: 88, and a first polypeptide chain consisting of the amino acid sequence set forth in SEQ ID NO: 98; or, a second polypeptide chain consisting of the amino acid sequence set forth in SEQ ID NO: 88, and a first polypeptide chain consisting of the amino acid sequence set forth in SEQ ID NO: 99; or, a second polypeptide chain consisting of the amino acid sequence set forth in SEQ ID NO: 91, and a first polypeptide chain consisting of the amino acid sequence set forth in SEQ ID NO: 98; or, a second polypeptide chain consisting of the amino acid sequence set forth in SEQ ID NO: 91, and a first polypeptide chain consisting of the amino acid sequence set forth in SEQ ID NO: 99.

11. A chimeric antigen receptor, **characterized in that** the chimeric antigen receptor comprises the antigen-binding protein targeting PD-L1 and CD40 of any one of claims 1-10.

12. An isolated nucleic acid, **characterized in that** the isolated nucleic acid encodes the antigen-binding protein targeting PD-L1 and CD40 of any one of claims 1-10.

13. A recombinant expression vector, **characterized in that** the recombinant expression vector comprises the isolated nucleic acid of claim 12; preferably, the recombinant expression vector comprises a eukaryotic cell expression vector and/or a prokaryotic cell expression vector.

14. A transformant, **characterized in that** the transformant comprises the isolated nucleic acid of claim 12 or the recombinant expression vector of claim 13; preferably, the host cell of the transformant is a prokaryotic cell and/or a eukaryotic cell, the prokaryotic cell is preferably an *E. coli* cell such as a TG1 or a BL21, and the eukaryotic cell is preferably an HEK293 cell or a CHO cell.

15. A method for preparing the antigen-binding protein targeting PD-L1 and CD40 of any one of claims 1-10, **characterized in that** the preparation method comprises the following steps: culturing the transformant of claim 14, and obtaining the antigen-binding protein targeting PD-L1 and CD40 from the culture.

16. An antibody-drug conjugate, **characterized in that** the antibody-drug conjugate comprises the antigen-binding protein targeting PD-L1 and CD40 of any one of claims 1-10, and a cytotoxic agent or a label; preferably, the cytotoxic agent is MMAF or MMAE, and the label is a fluorescent agent.

17. A genetically modified cell, **characterized in that** the genetically modified cell expresses the chimeric antigen receptor of claim 11; preferably, the genetically modified cell is a eukaryotic cell, preferably an isolated human cell, more preferably an immune cell such as T cell or NK cell.

18. A pharmaceutical composition, **characterized in that** the pharmaceutical composition comprises one or more of the antigen-binding protein targeting PD-L1 and CD40 of any one of claims 1-10, the chimeric antigen receptor of claim 11, the isolated nucleic acid of claim 12, the recombinant expression vector of claim 13, the genetically modified cell of claim 17, and the antibody-drug conjugate of claim 16, and/or a pharmaceutically acceptable carrier; preferably, the pharmaceutical composition further comprises other anti-tumor drugs as active ingredients, and the anti-tumor drugs include, but are not limited to: a chemotherapeutic drug, a nucleotide drug, a small molecule targeted drug, a monoclonal antibody drug, a bi/multi-specific antibody drug, a recombinant protein drug, an immunomodulatory drug, a cell therapy drug and a gene therapy drug.

19. A detection reagent, **characterized in that** the detection reagent comprises the antigen-binding protein targeting PD-L1 and CD40 of any one of claims 1-10 and/or the antibody-drug conjugate of claim 16; preferably, the detection reagent is in a liquid dosage form, a gaseous dosage form, a solid dosage form and a semi-solid dosage form; more preferably, the detection reagent further comprises a secondary antibody, CD40 or a derivative thereof, the secondary antibody is, for example, an anti-human IgG antibody conjugated to horseradish peroxidase and an anti-human IgG antibody conjugated to biotin.

20. A kit of parts, **characterized in that** the kit of parts comprises kit A containing one or more of the antigen-binding protein targeting PD-L1 and CD40 of any one of claims 1-10, the chimeric antigen receptor of claim 11, the pharmaceutical composition of claim 18, the detection reagent of claim 19, the genetically modified cell of claim 17, and the antibody-drug conjugate of claim 16;
preferably, the kit of parts further comprises kit B containing other anti-tumor antibodies or a pharmaceutical composition comprising the other anti-tumor antibodies, and/or one or more selected from the group consisting of a hormone preparation, a targeting small molecule preparation, a proteasome inhibitor, an imaging agent, a diagnostic agent, a chemotherapeutic agent, an oncolytic drug, a cytotoxic agent, a cytokine, an activator of a costimulatory molecule, an inhibitor of an inhibitory molecule, and a vaccine.

21. Use of one or more of the antigen-binding protein targeting PD-L1 and CD40 of any one of claims 1-10, the chimeric antigen receptor of claim 11, the isolated nucleic acid of claim 12, the recombinant expression vector of claim 13, the pharmaceutical composition of claim 18, the detection reagent of claim 19, the kit of parts of claim 20, the genetically modified cell of claim 17, and the antibody-drug conjugate of claim 16 in the preparation of a drug for diagnosing, preventing and/or treating PD-L1 and/or CD40 related diseases; preferably, the PD-L1 and/or CD40 related diseases are PD-L1 related tumors, CD40 related tumors or PD-L1×CD40 related tumors, the CD40 related tumors include solid tumors and hematologic malignancies, and the PD-L1 related tumors include solid tumors and hematologic malignancies; more preferably, the CD40 related tumors include B-cell NHL, chronic lymphocytic leukemia (CLL), hairy cell leukemia (HCL), Hodgkin's disease, multiple myeloma, bladder cancer, kidney cancer, ovarian cancer, cervical cancer, breast cancer, lung cancer, nasopharyngeal cancer, malignant melanoma, pancreatic cancer and colon cancer, the PD-L1 related tumors include squamous cell carcinoma, myeloma, small cell lung cancer, non-small cell lung cancer (NSCLC), head and neck squamous cell carcinoma (HNSCC), glioma, Hodgkin's lymphoma, non-Hodgkin's lymphoma, diffuse large B-cell lymphoma (DLBCL), follicular lymphoma, acute lymphoblastic leukemia (ALL), acute myeloid leukemia (AML), chronic lymphocytic leukemia (CLL), chronic myeloid leukemia (CML), primary mediastinal large B-cell lymphoma, mantle cell lymphoma (MCL), small lymphocytic lymphoma (SLL), T-cell/histiocyte-rich large B-cell lymphoma, multiple myeloma, myeloid cell leukemia-1 protein (Mcl-1), myelodysplastic syndrome (MDS), gastrointestinal cancer, kidney cancer, ovarian cancer, liver cancer, lymphoblastic leukemia, lymphocytic leukemia, colorectal cancer, endometrial cancer, kidney cancer, prostate cancer, thyroid cancer, melanoma, chondrosarcoma, neuroblastoma, pancreatic cancer, glioblastoma multiforme, gastric cancer, bone cancer, Ewing's sarcoma, cervical cancer, brain cancer, stomach cancer, bladder cancer, hepatoma, breast cancer, colon cancer, hepatocellular carcinoma (HCC), clear cell renal cell carcinoma (RCC), head and neck cancer, throat cancer, hepatobiliary cancer, central nervous system cancer, esophageal cancer, malignant pleural mesothelioma, systemic light chain amyloidosis, lymphoplasmacytic lymphoma, myelodysplastic syndrome, myeloproliferative neoplasm, neuroendocrine tumor, Merkel cell carcinoma, testicular cancer and skin cancer, preferably colon cancer with high expression of PD-L1.

22. A method for detecting PD-L1 and/or CD40 in a sample, **characterized in that** the method comprises the step of contacting the sample with one or more of the antigen-binding protein targeting PD-L1 and CD40 of any one of claims 1-10, the detection reagent of claim 19, and the antibody-drug conjugate of claim 16, the sample comprises, for example, a blood sample (e.g., a whole blood sample and a serum sample) and a reagent comprising PD-L1 and/or CD40, and preferably, the method is for a non-therapeutic/diagnostic purpose.

23. A method for diagnosing, treating and/or preventing PD-L1 and/or CD40 related diseases, **characterized in that** the method comprises administering to a patient in need thereof a therapeutically effective amount of one or more of the antigen-binding protein targeting PD-L1 and CD40 of any one of claims 1-10, the chimeric antigen receptor of claim 11, the isolated nucleic acid of claim 12, the recombinant expression vector of claim 13, the pharmaceutical composition of claim 18, the detection reagent of claim 19, the kit of parts of claim 20, the genetically modified cell of claim 17, and the antibody-drug conjugate of claim 16; preferably, the PD-L1 and/or CD40 related diseases are PD-L1 related tumors, CD40 related tumors or PD-L1×CD40 related tumors.
